# EUROPEAN PATENT SPECIFICATION

(11) **EP 1 599 477 B1**
(45) Date of publication and mention of the grant of the patent: **30.01.2008**
(21) Application number: 04710346.0
(22) Date of filing: 12.02.2004
(51) Int. Cl.: C07D 473/04, A61K 31/52, A61P 3/10

(54) **SULFONAMIDE SUBSTITUTED XANTHINE DERIVATIVES FOR USE AS PEPCK INHIBITORS**
SULFONAMIDSUBSTITUIERTE XANTHINDERIVATE ZUR VERWENDUNG ALS PEPCK-INHIBITOREN
DERIVES DE XANTHINE SUBSTITUES PAR DES SULFONAMIDES ET UTILES COMME INHIBITEURS DE PEPCK

(30) Priority: 19.02.2003 US 448562 P
(43) Date of publication of application: 30.11.2005
(73) Proprietor: F. HOFFMANN-LA ROCHE AG, 4070 Basel (CH)
(72) Inventor: FOLEY, Louise, Helen, Fort Myers, FL (US); HUBY, Nicholas, John, Silvester, Scotch Plains, NJ 07076 (US); PIETRANICO-COLE, Sherrie, Lynn, Montclair, NJ 07043 (US); YUN, Weiya, Warren, NJ 07059 (US); DUNTEN, Pete, William, Mountain View, CA 94040 (US)
(74) Representative: Klostermeyer, Dörte
(86) International application number: PCT/EP2004/001289
(87) International publication number: WO 2004/074288

(56) References cited:
- WO-A-01/77110
- WO-A-03/106459

## Description

The present invention is directed to sulfonamide substituted xanthine derivatives of formula I

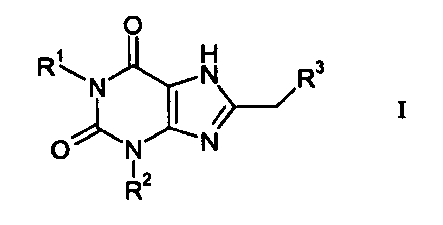

Compounds of formula I and pharmaceutically acceptable salts thereof are modulators of gluconeogenesis and are useful in the treatment of type 2 diabetes.

The control of glucose production is one of the key aspects of anti-diabetic therapy. Type 2 diabetics have elevated levels of postprandial and fasting blood glucose (Consoli, A., Nurjhan, N., Capani, F. and Gerich, J. Diabetes 38, 550-7,1989; Shulman, GI Am.J. Card. 84 (Suppl.1A):3J-10J, 1999). Excessive hepatic glucose production (HGP) contributes to the fasting hyperglycemia observed in patients with Type 2 diabetes (T2D) (Gastadelli, A., Baldi S., Pettiti M., Toschi, E., Camastra, S., Natali, A., Landau, B.R. & Ferranini, E., Diabetes 49:1367-1373, 2000). Gluconeogenesis is believed to be the major pathway for this increased glucose production (Defronzo, R.A., Bonadonna, R.C. and Ferrannini, E., Diabetes Care 15:318-367, 1992).

Phosphoenolpyruvate carboxykinase (PEPCK) is a key regulatory enzyme in the gluconeogenic pathway. PEPCK is believed to be the flux controlling, rate limiting enzyme for this pathway (Cimbala, A.N., Lamers, W.H., Nelson, J.E., Monahan, J.E., Yoo-Warren, H., and Hanson R.W., J.Biol.Chem. 257:7629-7636, 1982), hence inhibition of this enzyme represents a novel way to improve glucose homeostasis. Previous attempts to control hepatic glucose production through inhibition of gluconeogeneis were limited to biguanides such as metformin, which inhibits HGP (Defronzo, R.A., Diabetes Reviews 6:89-131, 1998). Metformin has side effects such as gastrointestinal (GI) disturbances and lactic acidosis. Inhibition of PEPCK provides superior efficacy and, coupled with reduced side effects, represents a novel treatment for type 2 diabetes.

Structurally related Xanthine derivatives which are taught to act as PDE 5 inhibitors are described in the PCT Publication WO 01/77110.

The present invention is directed to a compound of formula 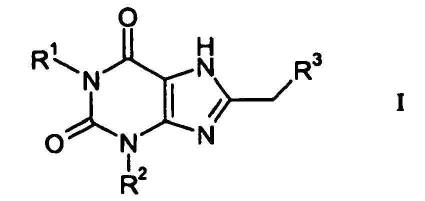 wherein
- R¹: is selected from the group consisting of
C₁₋₇-alkyl,
C₁₋₇-alkyl substituted by phenyl and
C₁₋₇-alkyl substituted by halogen substituted phenyl;
- R²: is selected from the group consisting of
C₁₋₇-alkyl and
C₁₋₇-alkyl substituted by C₃₋₇- cycloalkyl;
- R³: is selected from the group consisting of

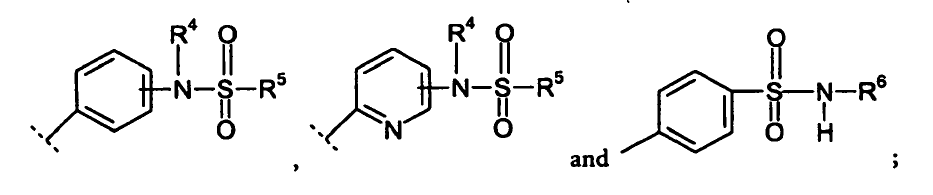

- R⁴: is selected from the group consisting of H and C₁₋₇- alkyl;
- R⁵: is selected from the group consisting of
C₁₋₇- alkyl,
amino C₁₋₇- alkyl,
C₁₋₇- alkyl substituted by phenyl,
C₂₋₇- alkenyl substituted by phenyl,
phenyl,
phenyl substituted by at least one substituent selected from the group consisting of C₁₋₇- alkyl, C₁₋₇- alkyl substituted by halogen, halogen, C₁₋₇- alkoxy, C₁₋₇- alkoxy substituted by halogen, nitro and acetamido,
a 5-membered heteroaromatic ring having one heteroatom independently selected from the group consisting of N, O and S,
the 5-membered heteroaromatic ring being unsubstituted or substituted by at least one substituent selected from the group consisting of C₁₋₇- alkyl, halogen, carboxy C₁₋₇- alkyl, amino, alkyl amino and acetamido,
a 5-membered heteroaromatic ring having two heteroatoms wherein a first heteroatom is N and a second heteroatom is independently selected from the group consisting of N, O and S,
the 5-membered heteroaromatic ring being unsubstituted or substituted by at least one substituent selected from the group consisting of C₁₋₇- alkyl, halogen, carboxy C₁₋₇- alkyl, amino, alkyl amino and acetamido,
a 6-membered heteroaromatic ring having one N, the 6-membered heteroaromatic ring being unsubstituted or substituted by at least one substituent selected from the group consisting of C₁₋₇- alkyl, halogen, carboxy C₁₋₇- alkyl, amino, alkyl amino and acetamido, 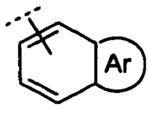 wherein Ar is selected from the group consisting of a 5-membered heteroaromatic ring fused to the 6-membered ring, having one, two, or three heteroatoms, and wherein a first heteroatom is N, a second heteroatom is N and a third heteroatom is selected from the group consisting of O and S,
a 6-membered aromatic ring fused to the 6-membered ring, having no or one N heteroatoms, the fused 6-membered aromatic ring being unsubstituted or substituted by at least one substituent selected from the group consisting of C₁₋₇-alkyl, halogen, carboxy C₁₋₇- alkyl, amino, alkyl amino and acetamido; and
- R⁶: is selected from the group consisting of
H,
a 5-membered aromatic heterocyclic ring with one or two heteroatoms wherein a first heteroatom is N and a second heteroatom is selected from the group consisting of N and S, the 5-membered heterocyclic ring being unsubstituted or substituted by
at least one substituent selected from the group consisting of C₁₋₇- alkyl, halogen, carboxy C₁₋₇- alkyl, amino, alkyl amino and acetamido, and
a 6-membered aromatic heterocyclic ring with one or two N heteroatoms, the 6-membered heterocyclic ring being unsubstituted or substituted by at least one substituent selected from the group consisting of C₁₋₇- alkyl, halogen, carboxy C₁₋₇-alkyl, amino, alkyl amino and acetamido,
or a pharmaceutically acceptable salt thereof.

Phosphoenolpyruvate carboxykinase (PEPCK) is a key regulatory enzyme in the gluconeogenic pathway. As stated above, PEPCK is believed to be the flux controlling, rate limiting enzyme for this pathway, hence inhibition of this enzyme represents a novel way to improve glucose homeostasis. Previous attempts to control hepatic glucose production (HGP) through inhibition of gluconeogenesis were limited to biguanides such as metformin which inhibits HGP, but by an unknown mechanism. Inhibition of HGP by specifically targeting an enzyme, PEPCK, known to be in the gluconeogenic pathway, by administration of a therapeutically effective amount of a compound of formula I or a pharmaceutically effective salt thereof is an alternative therapy. In addition, inhibition of PEPCK by administration of a therapeutically effective amount of a compound of formula I provides superior efficacy and, coupled with reduced side effects, represents a novel treatment for type 2 (non-insulin dependent) diabetes.

The present invention is also directed to pharmaceutical compositions comprising a therapeutically effective amount of one or more compounds of formula I, or a pharmaceutically acceptable salt thereof, and a pharmaceutically acceptable carrier or excipient.

The present invention is further directed to a method of treatment of type 2 diabetes comprising administering a therapeutically effective amount of a compound of formula I or a pharmaceutically acceptable salt thereof to a patient in need of such treatment.

The following definitions of the general terms used in the present description apply irrespective of whether the terms in question appear alone or in combination.

"Lower alkyl" refers to both straight chain and branched chain hydrocarbon groups having from one to seven carbon atoms, such as methyl, ethyl, propyl, butyl, isopropyl, isobutyl and the like. Preferred alkyl groups are methyl, ethyl, butyl and isopropyl. n-Butyl is particularly preferred.

The term "lower alkoxy" denotes a group wherein the alkyl residues are as defined above and which is attached via an oxygen atom.

"Lower alkenyl" refers to a hydrocarbon chain as defined for lower alkyl having at least one olefinic double bond, e.g., vinyl, allyl, butenyl and the like.

"Lower cycloalkyl" refers to cyclic saturated hydrocarbons having between three and seven carbon atoms, such as cyclopropyl, cyclobutyl, cyclopentyl and the like. Cyclopropyl, and cyclobutyl are preferred, with cyclopropyl being more preferred. These cycloalkyl groups may be unsubstituted or substituted with one or more substituents. Preferably, the cycloalkyl groups are unsubstituted.

"Carboxy lower alkyl" refers to both straight chain and branched chain hydrocarbon groups having one to seven carbon atoms with at least one carbon possessing a carboxy group.

"Amino lower alkyl" refers to both straight chain and branched chain hydrocarbon groups having one to seven carbon atoms with at least one carbon substituted with an amino group.

The term "unsubstituted" denotes that there are no other atoms attached to a chain or ring other than hydrogen. The term "substituted" as in substituted alkyl, substituted phenyl or substituted aromatic heterocycle, means that the substitution can occur at one or more positions and, unless otherwise indicated, that the substituent at each substitution site are independently selected from the specified options. The term "at least one" substituted means one, two, three, four or five substituents.

As used herein, the terms "halogen" means fluorine, chlorine, bromine and iodine. Preferred halogens are fluorine, chlorine and bromine.

Other chemical and structural terms used in the description are to be interpreted with their normal meaning in the art of organic chemistry. The terms "amino" and formula "-NH₂" may be used interchangably.

"Alkyl amino" means an amino group substituted with one or two lower alkyl groups such as for example methylamino, dimethylamino, ethylamino, n-propylamino or isopropylamino. "Acetamido" means an acetylamino group.

The term "five-membered heteroaromatic ring" means a 5-membered aromatic ring with one heteroatom independently selected from the group consisting of nitrogen, sulfur and oxygen. Exemplary of these five-membered heteroaromatic ring moieties are furane, pyrrole and thiophene.

The term "6-membered heteroaromatic having one or two N heteroatoms", means pyridine with the ring attachment at the 2, 3, or 4 position or pyridazine, pyrimidine or pyrazine with the attachment point being adjacent to one or two N atoms.

The term "a 5-membered heteroaromatic ring having two heteroatoms wherein a first heteroatom is N and a second heteroatom is independently selected from the group consisting of N, O and S" means an aromatic ring with two heteroatoms, one of which is always N. Exemplary of these 5-membered heteroaromatic rings are oxazole, imidazole, thiazole, isoxazole, pyrazole, isothiazole and the like.

The term "5-membered heteroaromatic ring fused to the 6-membered ring, having one, two or three heteroatoms wherein a first heteroatom is N, a second heteroatom is N and a third heteroatom is selected from the group consisting of O and S" means a group such as benzothiadiazole, benzoxadiazole, benzimidazole, benzopyrazole and the like.

The term "a 6-membered aromatic ring fused to the 6-membered ring, having no or one N heteroatoms" encompasses the moieties napthalene, quinoline and isoquinoline.

"Pharmaceutically acceptable salt" refers to conventional acid-addition salts or base-addition salts that retain the biological effectiveness and properties of the compounds of formula I and are formed from suitable non-toxic organic or inorganic acids or organic or inorganic bases. Exemplary acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, trifluoroacetic acid, acetic acid, methanesulfonic acid, oxalic acid, succinic acid, citric acid, malic acid, lactic acid, fumaric acid, and the like. Exemplary base-addition salts include those derived from ammonium, potassium, sodium and, quaternary ammonium hydroxides, such as for example, tetramethylammonium hydroxide. The chemical modification of a pharmaceutical compound (i.e., drug) into a salt is a technique well known to pharmaceutical chemists to obtain improved physical and chemical stability, hygroscopicity, flowability and solubility of compounds. See, e.g., H. Ansel et. al., Pharmaceutical Dosage Forms and Drug Delivery Systems (6th Ed. 1995) at pp. 196 and 1456-1457.

"Therapeutically effective amount" means an amount that is effective to prevent, alleviate or ameliorate symptoms of disease or prolong the survival of the subject being treated. Determination of a therapeutically effective amount is within the skill in the art.

In one embodiment, the present invention is directed to a compound of formula 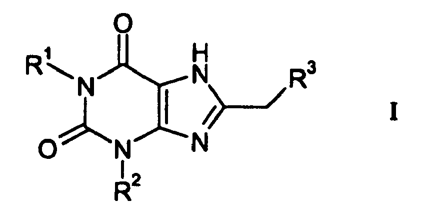 wherein,
- R¹: is selected from the group consisting of
C₁₋₇- alkyl,
C₁₋₇- alkyl substituted by phenyl and
C₁₋₇- alkyl substituted by halogen substituted phenyl;
- R²: is selected from the group consisting of
C₁₋₇- alkyl and
C₁₋₇- alkyl substituted by C₃₋₇- cycloalkyl;
- R³: is selected from the group consisting of 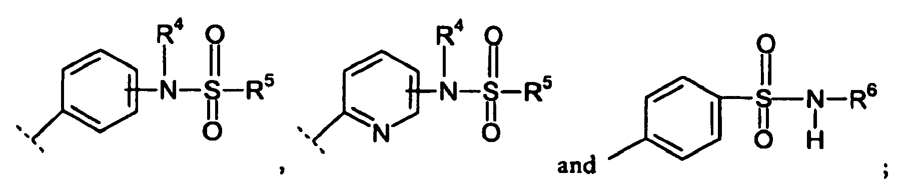
- R⁴: is selected from the group consisting of H and C₁₋₇- alkyl;
- R⁵: is selected from the group consisting of
C₁₋₇- alkyl,
amino C₁₋₇- alkyl,
C₁₋₇- alkyl substituted by phenyl,
C₂₋₇- alkenyl substituted by phenyl,
phenyl,
phenyl substituted by at least one substituent selected from the group consisting of C₁₋₇- alkyl, C₁₋₇- alkyl substituted by halogen, halogen, C₁₋₇- alkoxy, C₁₋₇-alkoxy substituted by halogen, nitro and acetamido,
a 5-membered heteroaromatic ring having one heteroatom independently selected from the group consisting of N, O and S,
the 5-membered heteroaromatic ring being unsubstituted or substituted by at least one substituent selected from the group consisting of C₁₋₇- alkyl, halogen, carboxy C₁₋₇-alkyl, amino, alkyl amino and acetamido,
a 5-membered heteroaromatic ring having two heteroatoms wherein a first heteroatom is N and a second heteroatom is independently selected from the group consisting of N, O and S,
the 5-membered heteroaromatic ring being unsubstituted or substituted by at least one substituent selected from the group consisting of C₁₋₇-alkyl, halogen, carboxy C₁₋₇-alkyl, amino, alkyl amino and acetamido,
a 6-membered heteroaromatic ring having one N, the 6-membered heteroaromatic ring being unsubstituted or substituted by at least one substituent selected from the group consisting of C₁₋₇-alkyl, halogen, carboxy C₁₋₇-alkyl, amino, alkyl amino and acetamido, 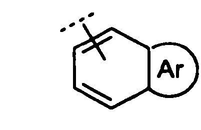 wherein Ar is selected from the group consisting of a 5-membered heteroaromatic ring fused to the 6-membered ring, having one, two, or three heteroatoms, and wherein a first heteroatom is N, a second heteroatom is N and a third heteroatom is selected from the group consisting of O and S, and
a 6-membered aromatic ring fused to the 6-membered ring, having no or one N heteroatoms, the fused 6-membered aromatic ring being unsubstituted or substituted by at least one substituent selected from the group consisting of C₁₋₇-alkyl, halogen, carboxy C₁₋₇-alkyl, amino, alkyl amino andacetamido; and
- R⁶: is selected from the group consisting of H,
a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇-alkyl, halogen, carboxy C₁₋₇-alkyl, amino, alkyl amino and acetamido, and
a 6-membered aromatic heterocyclic ring with one or two N heteroatoms, the 6-membered heterocyclic ring being unsubstituted or substituted by at least one substituent selected from the group consisting of C₁₋₇-alkyl, halogen, carboxy C₁₋₇-alkyl, amino, alkyl amino and acetamido,
or a pharmaceutically acceptable salt thereof.

In one preferred embodiment of a compound of formula I of the present invention, R¹ is C₁₋₇-alkyl.

Especially preferred are compounds of formula I wherein R¹ is C₁₋₇-alkyl and wherein R² is C₁₋₇-alkyl. Examples of such compounds are: 1,3-dimethyl-1H-pyrazole-4-sulfonic acid [4-(1,3-dimethyl-2,6-dioxo-2,3,6,7-tetrahydro-1H-purin-8-ylmethyl)-phenyl]-amide; and 5-chloro-1,3-dimethyl-1H-pyrazole-4-sulfonic acid {4-[1,3-dimethyl-2,6-dioxo-2,3,6,7-tetrahydro-1H-purin-8-ylmethyl]-phenyl}-amide.

In one preferred embodiment of a compound of formula I of the present invention, R¹ is C₁₋₇-alkyl substituted by phenyl. Especially preferred are compounds of formula I wherein R¹ is C₁₋₇-alkyl substituted by phenyl and R² is C₁₋₇-alkyl.

An exemplary compound of this preferred compound of formula I is selected from the group consisting of
4-(1-benzyl-3-butyl-2,6-dioxo-2,3,6,7-tetrahydro-1H-purin-8-ylmethyl)-benzenesulfonamide;
*N*-[4-(1-benzyl-3-butyl-2,6-dioxo-2,3,6,7-tetrahydro-1H-purin-8-ylmethyl)-phenyl]-4-methyl-benzenesulfonamide; and
*N*-[4-(1-benzyl-3-butyl-2,6-dioxo-2,3,6,7-tetrahydro-1H-purin-8-ylmethyl)-phenyl]-methanesulfonamide.

Another preferred compound of formula I has the structure

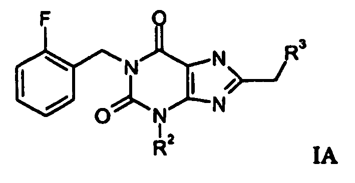

A preferred compound of formula IA is where R² is C₁₋₇-alkyl.

A more preferred compound of formula IA is where R² is n-butyl.

An even more preferred compound of formula IA is where R² is C₁₋₇-alkyl, i.e. n-butyl, and R³ is

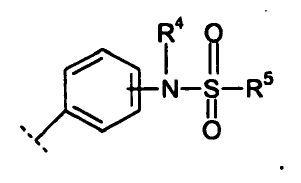

In this embodiment, R⁴ preferably is H.

Especially preferred are compounds within this embodiment where R⁵ is a 5-membered heteroaromatic ring having one heteroatom independently selected from the group consisting of N, O and S, with the 5-membered heteroaromatic ring being unsubstituted or substituted by at least one substituent selected from the group consisting of C₁₋₇-alkyl, halogen, carboxy C₁₋₇-alkyl, amino, alkyl amino and acetamido.

An exemplary compound of these preferred compounds of formula IA is selected from the group consisting of
thiophene-2-sulfonic acid {4-[3-butyl-1-(2-fluoro-benzyl)-2,6-dioxo-2,3,6,7-tetrahydro-1H-purin-8-ylmethyl]-phenyl}-amide;
5-chlorothiophene-2-sulfonic acid {4-[3-butyl-1-(2-fluoro-benzyl)-2,6-dioxo-2,3,6,7-tetrahydro-1H-purin-8-ylmethyl]-phenyl}-amide;
3-{4-[3-butyl-1-(2-fluoro-benzyl)-2,6-dioxo-2,3,6,7-tetrahydro-1H-purin-8-ylmethyl]-phenylsulfamoyl}-thiophene-2-carboxylic acid methyl ester;
4,5-dibromothiophene-2-sulfonic acid {4-[3-butyl-1-(2-fluoro-benzyl)-2,6-dioxo-2,3,6,7-tetrahydro-1H-purin-8-ylmethyl]-phenyl}-amide; and
5-bromo-thiophene-2-sulfonic acid {4-[3-butyl-1-(2-fluoro-benzyl)-2,6-dioxo-2,3,6,7-tetrahydro-1H-purin-8-ylmethyl]-phenyl}-amide.

Furthermore, preferred compounds within this embodiment are wherein R⁵ is a 5-membered heteroaromatic ring having two heteroatoms wherein a first heteroatom is N and a second heteroatom is independently selected from the group consisting of N, O and S, the 5-membered heteroaromatic ring being unsubstituted or substituted by at least one substituent selected from the group consisting of C₁₋₇-alkyl, halogen, carboxy C₁₋₇-alkyl, amino, alkyl amino and acetamido.

An exemplary compound of these preferred compounds of formula IA is selected from the group consisting of
methyl-1H-imidazole-4-sulfonic acid {4-[3-butyl-1-(2-fluoro-benzyl)-2,6-dioxo-2,3,6,7-tetrahydro-1H-purin-8-ylmethyl]-phenyl}-amide trifluoroacetic acid salt; and 5-chloro-1,3-dimethyl-1H-pyrazole-4-sulfonic acid {4-[3-butyl-1-(2-fluoro-benzyl)-2,6-dioxo-2,3,6,7-tetrahydro-1H-purin-8-ylmethyl]-phenyl}-amide.

Another preferred compound of formula IA is where R² is n-butyl and R⁵ is a 6-membered heteroaromatic ring having one N, with the 6-membered heteroaromatic ring being unsubstituted or substituted by at least one substituent selected from the group consisting of C₁₋₇-alkyl, halogen, carboxy C₁₋₇-alkyl, amino, alkyl amino and acetamido.

Exemplary of these compounds of formula IA is 6-chloro-pyridine-3-sulfonic acid {4-[3-butyl-1-(2-fluoro-benzyl)-2,6-dioxo-2,3,6,7-tetrahydro-1H-purin-8-ylmethyl] - phenyl}-amide.

Another preferred compound of formula IA wherein R² is n-butyl is where R⁵ is 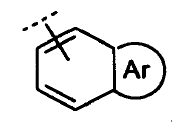 and Ar is selected from the group consisting of a 5-membered heteroaromatic ring fused to the 6-membered ring, having one, two, or three heteroatoms, and wherein a first heteroatom is N, a second heteroatom is N and a third heteroatom is selected from the group consisting of O and S; and
a 6-membered aromatic ring fused to the 6-membered ring, having no or one N heteroatoms, the fused 6-membered aromatic ring being unsubstituted or substituted by at least one substituent selected from the group consisting of C₁₋₇-alkyl, halogen, carboxy C₁₋₇-alkyl, amino, alkyl amino and acetamido.

A compound representative of these preferred compounds is selected from the group consisting of
*N*-{4-[3-butyl-1-(2-fluoro-benzyl)-2,6-dioxo-2,3,6,7-tetrahydro-1H-purin-8-ylmethyl]-phenyl}-1-naphthalenesulfonamide;
5-dimethylamino-naphthalene-1-sulfonic acid {4-[3-butyl-1-(2-fluoro-benzyl)-2,6-dioxo-2,3,6,7-tetrahydro-1H-purin-8-ylmethyl]-phenyl}-amide;
benzo[1,2,5]thiadiazole-4-sulfonamid {4-[3-butyl-1-(2-fluoro-benzyl)-2,6-dioxo-2.3,6,7-tetrahydro-1H-purin-8-ylmethyl]-phenyl}-amide; and
benzo[1,2,5]oxadiazole-4-sulfonic acid {4- [3-butyl-1-(2-fluoro-benzyl)-2,6-dioxo-2,3,6,7-tetrahydro-1H-purin-8-ylmethyl]-phenyl}-amide.

Another preferred compound of formula IA is where R² is n-butyl and R⁵ is C₁₋₇-alkyl or C₁₋₇-alkyl substituted by phenyl.

A compound representative of these preferred compounds is selected from the group consisting of
propane-2-sulfonic acid {4-[3-butyl-1-(2-fluoro-benzyl)-2,6-dioxo-2,3,6,7-tetrahydro-1H-purin-8-ylmethyl]-phenyl}-amide;
*N*-{4-[3-butyl-1-(2-fluoro-benzyl)-2,6-dioxo-2,3,6,7-tetrahydro-1H-purin-8-ylmethyl]-phenyl}-C-phenyl-methanesulfonamide; and
ethanesulfonic acid {4-[3-butyl-1-(2-fluoro-benzyl)-2,6-dioxo-2,3,6,7-tetrahydro-1H-purin-8-ylmethyl]-phenyl}-amide.

Yet another preferred compound of formula IA is where R² is n-butyl and R⁵ is C₂₋₇- alkenyl substituted by phenyl.

A compound representative of this preferred embodiment is 2-phenyl-ethenesulfonic acid {4-[3-butyl-1-(2-fluoro-benzyl)-2,6-dioxo-2,3,6,7-tetrahydro-1H-purin-8-ylmethyl]-phenyl}-amide.

Another preferred compound of formula IA is where R² is n-butyl and R⁵ is phenyl. Representative of this compound is *N*-{4-[3-butyl-1-(2-fluoro-benzyl)-2,6-dioxo-2,3,6,7-tetrahydro-1H-purin-8-ylmethyl]-phenyl}-benzenesulfonamide.

A further preferred compound of formula IA wherein R² is n-butyl is where R⁵ is phenyl substituted by at least one substituent selected from the group consisting of C₁₋₇-alkyl, C₁₋₇- alkyl substituted by halogen, halogen, C₁₋₇-alkoxy, C₁₋₇-alkoxy substituted by halogen, nitro and acetamido.

A preferred compound of formula IA is where R² is n-butyl and R⁵ is phenyl substituted by one substituent on the phenyl selected from the group consisting of C₁₋₇-alkyl, C₁₋₇-alkyl substituted by halogen, halogen, C₁₋₇-alkoxy, C₁₋₇-alkoxy substituted by halogen, nitro and acetamido.

A more preferred compound of this embodiment is where the one substituent is halogen.

A compound representative of the preferred compound wherein the phenyl has one halogen substituent is selected from the group consisting of
*N*-{4-[3-butyl-1-(2-fluoro-benzyl)-2,6-dioxo-2,3,6,7-tetrahydro-1H-purin-8-ylmethyl]-phenyl}-4-chloro-benzenesulfonamide;
*N*-{4-[3-butyl-1-(2-fluoro-benzyl)-2,6-dioxo-2,3,6,7-tetrahydro-1H-purin-8-ylmethyl]-phenyl}-4-iodo-benzenesulfonamide;
*N*-{4-[3-butyl-1-(2-fluoro-benzyl)-2,6-dioxo-2,3,6,7-tetrahydro-1H-purin-8-ylmethyl]-phenyl}-2-chloro-benzenesulfonamide;
*N*-{4-[3-butyl-1-(2-fluoro-benzyl)-2,6-dioxo-2,3,6,7-tetrahydro-1H-purin-8-ylmethyl]-phenyl}-3-chloro-benzenesulfonamide;
*N*-{4-[3-butyl-1-(2-fluoro-benzyl)-2,6-dioxo-2,3,6,7-tetrahydro-1H-purin-8-ylmethyl]-phenyl}-4-fluoro-benzenesulfonamide;
*N*-{4-[3-butyl-1-(2-fluoro-benzyl)-2,6-dioxo-2,3,6,7-tetrahydro-1H-purin-8-ylmethyl]-phenyl}-2-fluoro-benzenesulfonamide; and
*N*-{4-[3-butyl-1-(2-fluoro-benzyl)-2,6-dioxo-2,3,6,7-tetrahydro-1H-purin-8-ylmethyl]-phenyl}-4-bromo-benzenesulfonamide.

Another preferred embodiment of formula IA is where R² is n-butyl and where R⁵ is phenyl substituted by one substituent selected from the group C₁₋₇-alkyl and C₁₋₇-alkyl substituted by halogen.

Representative of this preferred embodiment of formula IA is a compound selected from the group
*N*-{4-[3-butyl-1-(2-fluoro-benzyl)-2,6-dioxo-2,3,6,7-tetrahydro-1H-purin-8-ylmethyl]-phenyl}-3-trifluoromethyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-4-isopropyl-benzenesulfonamide;
*N*-{4- [3-butyl-1-(2-fluoro-benzyl)-2,6-dioxo-2,3,6,7-tetrahydro-1H-purin-8-ylmethyl]-phenyl}-3-methyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-2-trifluoromethyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-4-ethyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-2-methyl-benzenesulfonamide; and
*N*-{4-[3-butyl-1-(2-fluoro-benzyl)-2,6-dioxo-2,3,6,7-tetrahydro-1H-purin-8-ylmethyl]-phenyl}-4-trifluoromethyl-benzenesulfonamide.

Another preferred embodiment of formula IA is where R² is n-butyl and where R⁵ is phenyl substituted by one substituent selected from the group nitro, alkoxy and alkoxy substituted by halogen.

Representative of this preferred embodiment of formula IA is a compound selected from the group
*N*-{4-[3-butyl-1-(2-fluoro-benzyl)-2,6-dioxo-2,3,6,7-tetrahydro-1H-purin-8-ylmethyl]-phenyl}-4-trifluoromethyl-benzenesulfonamide,
*N*-{4-[3-butyl-1-(2-fluoro-benzyl)-2,6-dioxo-2,3,6,7-tetrahydro-1H-purin-8-ylmethyl]-phenyl}-3-nitro-benzenesulfonamide,
*N*-{4-[3-butyl-1-(2-fluoro-benzyl)-2,6-dioxo-2,3,6,7-tetrahydro-1H-purin-8-ylmethyl]-phenyl}-4-methoxy-benzenesulfonamide, and
*N*-{4-[3-butyl-1-(2-fluoro-benzyl)-2,6-dioxo-2,3,6,7-tetrahydro-1H-purin-8-ylmethyl]-phenyl}-4-trifluoromethoxy-benzenesulfonamide.

Another preferred compound of formula IA is where R² is n-butyl and R⁵ is phenyl substituted by two substituents selected from the group consisting of C₁₋₇-alkyl, C₁₋₇-alkyl substituted by halogen, halogen, C₁₋₇-alkoxy, C₁₋₇-alkoxy substituted by halogen, nitro and acetamido.

In one more preferred embodiment, these two substituents are halogen. A further more preferred embodiment is when the two halogen substituents are chloro.

Representative of the preferred compound when the two halogen substituents are chloro is a compound selected from the group consisting of
*N*-{4- [3-butyl-1-(2-fluoro-benzyl)-2,6-dioxo-2,3,6,7-tetrahydro-1H-purin-8-ylmethyl]-phenyl}-2,4-dichloro-benzenesulfonamide;
*N*-{4- [3-butyl-1-(2-fluoro-benzyl)-2,6-dioxo-2,3,6,7-tetrahydro-1H-purin-8-ylmethyl]-phenyl}-2,5-dichloro-benzenesulfonamide;
*N*-{4-[3-butyl-1-(2-fluoro-benzyl)-2,6-dioxo-2,3,6,7-tetrahydro-1H-purin-8-ylmethyl]-phenyl}-2,6-dichloro-benzenesulfonamide;
*N*-{4-[3-butyl-1-(2-fluoro-benzyl)-2,6-dioxo-2,3,6,7-tetrahydro-1H-purin-8-ylmethyl]-phenyl}-2,3-dichloro-benzenesulfonamide;
*N*-{4- [3-butyl-1-(2-fluoro-benzyl)-2,6-dioxo-2,3,6,7-tetrahydro-1H-purin-8-ylmethyl]-phenyl}-3,5-dichloro-benzenesulfonamide; and
*N*-{4-[3-butyl-1-(2-fluoro-benzyl)-2,6-dioxo-2,3,6,7-tetrahydro-1H-purin-8-ylmethyl]-phenyl}-3,4-dichloro-benzenesulfonamide.

Another preferred embodiment of the compound of formula IA is where R² is n-butyl and R⁵ is phenyl substituted by two halogen substituents, and particularly when on of the halogen substituents is chloro and the other of the halogen substituents is fluoro.

A compound exemplary of this preferred embodiment is selected from the group consisting of
*N*-{4-[3-butyl-1-(2-fluoro-benzyl)-2,6-dioxo-2,3,6,7-tetrahydro-1H-purin-8-ylmethyl]-phenyl}-2-chloro-4-fluoro-benzenesulfonamide; and
*N*-{4-[3-butyl-1-(2-fluoro-benzyl)-2,6-dioxo-2,3,6,7-tetrahydro-1H-purin-8-ylmethyl]-phenyl}-3-chloro-4-fluoro-benzenesulfonamide.

Another preferred compound of formula IA is where R² is n-butyl and R⁵ is phenyl substituted by two halogen substituents, particularly when both of the halogen substituents are fluoro.

A compound exemplary of this preferred compound is selected from the group consisting of
*N*-{4-[3-butyl-1-(2-fluoro-benzyl)-2,6-dioxo-2,3,6,7-tetrahydro-1H-purin-8-ylmethyl]-phenyl}-2,4-difluoro-benzenesulfonamide;
*N*-{4-[3-butyl- 1-(2-fluoro-benzyl)-2,6-dioxo-2,3,6,7-tetrahydro-1H-purin-8-ylmethyl]-phenyl}-2,6-difluoro-benzenesulfonamide; and
*N*-{4-[3-butyl-1-(2-fluoro-benzyl)-2,6-dioxo-2,3,6,7-tetrahydro-1H-purin-8-ylmethyl]-phenyl}-3,4-difluoro-benzenesulfonamide.

Yet another preferred compound of formula IA is where R² is n-butyl and R⁵ is phenyl substituted by two substituents selected from the group consisting of C₁₋₇-alkyl, C₁₋₇-alkyl substituted by halogen, halogen, C₁₋₇-alkoxy, C₁₋₇-alkoxy substituted by halogen, nitro and acetamido, particularly where one of the two phenyl substituents is halogen and the other phenyl substituent is C₁₋₇-alkyl.

A compound exemplary of this preferred compound is selected from the group consisting of
*N*-{4-[3-butyl-1-(2-fluoro-benzyl)-2,6-dioxo-2,3,6,7-tetrahydro-1H-purin-8-ylmethyl]-phenyl}-3-chloro-4-methyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-3-chloro-2-methyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-2-chloro-6-methyl-benzenesulfonamide; and
*N*-{4-[3-butyl-1-(2-fluoro-benzyl)-2,6-dioxo-2,3,6,7-tetrahydro-1H-purin-8-ylmethyl]-phenyl}-5-fluoro-2-methyl-benzenesulfonamide.

Yet another preferred compound of formula IA is where R² is n-butyl and R⁵ is phenyl substituted by two substituents selected from the group consisting of C₁₋₇-alkyl, C₁₋₇-alkyl substituted by halogen, halogen, C₁₋₇-alkoxy, C₁₋₇-alkoxy substituted by halogen, nitro and acetamido, particularly wherein the two phenyl substituents are selected from the group consisting of C₁₋₇-alkyl, C₁₋₇-alkoxyl and nitro.

A compound exemplary of this preferred embodiment is selected from the group consisting of
*N*-{4-[3-butyl-1-(2-fluoro-benzyl)-2,6-dioxo-2,3,6,7-tetrahydro-1H-purin-8-ylmethyl]-phenyl}-2,5-dimethoxy-benzenesulfonamide;
*N*-{4- [3-butyl-1-(2-fluoro-benzyl)-2,6-dioxo-2,3,6,7-tetrahydro-1H-purin-8-ylmethyl]-phenyl}-2-methoxy-5-methyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-3,4-dimethoxy-benzenesulfonamide;
*N*-{4-[3-butyl-1-(2-fluoro-benzyl)-2,6-dioxo-2,3,6,7-tetrahydro-1H-purin-8-ylmethyl]-phenyl}-2,5-dimethyl-benzenesulfonamide; and
*N*-{4-[3-butyl-1-(2-fluoro-benzyl)-2,6-dioxo-2,3,6,7-tetrahydro-1H-purin-8-ylmethyl]-phenyl}-2-methyl-5-nitro-benzenesulfonamide.

A further preferred compound of formula IA is where R² is n-butyl and R⁵ is phenyl substituted by three substituents selected from the group consisting of C₁₋₇-alkyl, C₁₋₇-alkyl substituted by halogen, halogen, C₁₋₇-alkoxy, C₁₋₇-alkoxy substituted by halogen, nitro and acetamido. A more preferred compound with three substituents on the phenyl is where the three substituents are selected from C₁₋₇-alkyl and halogen.

A compound representative of this preferred compound is selected from the group consisting of
*N*-{4-[3-butyl-1-(2-fluoro-benzyl)-2,6-dioxo-2,3,6,7-tetrahydro-1H-purin-8-ylmethyl]-phenyl}-2,4,6-trimethyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-4-chloro-2,5-dimethyl-benzenesulfonamide;
*N*-{4-[3-butyl-1-(2-fluoro-benzyl)-2,6-dioxo-2,3,6,7-tetrahydro-1H-purin-8-ylmethyl]-phenyl}-2,4-dichloro-6-methyl-benzenesulfonamide; and
*N*-{4-[3-butyl-1-(2-fluoro-benzyl)-2,6-dioxo-2,3,6,7-tetrahydro-1H-purin-8-ylmethyl]-phenyl}-C-phenyl-methanesulfonamide.

A further preferred compound of formula IA is where R² is n-butyl and R⁵ is phenyl substituted by four substituents selected from the group consisting of C₁₋₇-alkyl, C₁₋₇-alkyl substituted by halogen, halogen, C₁₋₇-alkoxy, C₁₋₇-alkoxy substituted by halogen, nitro and acetamido. A more preferred compound with four substituents on the phenyl is where the four substituents are selected from C₁₋₇-alkyl and halogen.

A compound exemplary of this preferred compound is selected from the group consisting of
*N*-{4-[3-butyl-1-(2-fluoro-benzyl)-2,6-dioxo-2,3,6,7-tetrahydro-1H-purin-8-ylmethyl]-phenyl}-2,3,5,6-tetramethyl-benzenesulfonamide; and
*N*-{4-[3-butyl-1-(2-fluoro-benzyl)-2,6-dioxo-2,3,6,7-tetrahydro-1H-purin-8-ylmethyl]-phenyl}-4-methoxy-2,3,6-trimethyl-benzenesulfonamide.

Another preferred compound of formula 1A is where R² is n-butyl, R³ is 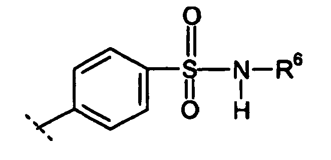 and R⁶ is a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇-alkyl, halogen, carboxy C₁₋₇-alkyl, amino, alkyl amino and acetamido.

A compound representative of this preferred compound is selected from the group consisting of
4- [3-butyl-1-(2-fluoro-benzyl)-2,6-dioxo-2,3,6,7-tetrahydro-1H-purin-8-ylmethyl]*-N-*(1,3,5-trimethyl-1H-pyrazol-4-yl)-benzenesulfonamide; and
4-[3-butyl-1-(2-fluoro-benzyl)-2,6-dioxo-2,3,6,7-tetrahydro-1H-purin-8-ylmethyl]-*N-*thiazol-2-yl-benzenesulfonamide.

Another preferred compound of formula 1A is where R² is n-butyl, R³ is 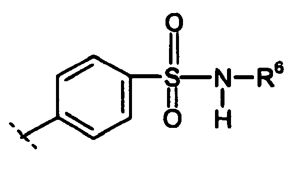 and R⁶ is a 6-membered aromatic heterocyclic ring with one or two N heteroatoms, the 6-membered heterocyclic ring being unsubstituted or substituted by at least one substituent selected from the group consisting of C₁₋₇-alkyl, halogen, carboxy C₁₋₇-alkyl, amino, alkyl amino and acetamido.

A compound exemplary of this preferred compound is selected from the group consisting of
4-[3-butyl-1-(2-fluoro-benzyl)-2,6-dioxo-2,3,6,7-tetrahydro-1H-purin-8-ylmethyl]-*N-*pyridin-2-yl-benzenesulfonamide;
4-[3-butyl-1-(2-fluoro-benzyl)-2,6-dioxo-2,3,6,7-tetrahydro-1H-purin-8-ylmethyl]-*N-*pyridin-3-yl-benzenesulfonamide;
4-[3-butyl-1-(2-fluoro-benzyl)-2,6-dioxo-2,3,6,7-tetrahydro-1H-purin-8-ylmethyl]-*N-*pyridin-4-yl-benzenesulfonamide;
4-[3-butyl-1-(2-fluoro-benzyl)-2,6-dioxo-2,3,6,7-tetrahydro-1H-purin-8-ylmethyl]-*N-*pyrimidin-2-yl-benzenesulfonamide; and
4-[3-butyl-1-(2-fluoro-benzyl)-2,6-dioxo-2,3,6,7-tetrahydro-1H-purin-8-ylmethyl]-*N-*(1,3,5-trimethyl-1H-pyrazol-4-yl)-benzenesulfonamide.

Another preferred compound of formula 1A is where R² is n-butyl, R³ is 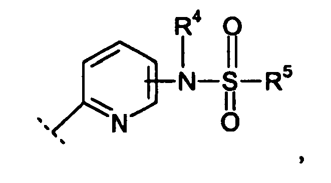 R⁴ is H and R⁵ is a 5-membered aromatic heterocyclic ring with one or two heteroatoms where a first heteroatom is N and a second heteroatom is selected from the group consisting of N and S, the 5-membered heterocyclic ring being unsubstituted or substituted by at least one substituent selected from the group consisting of C₁₋₇-alkyl, halogen, carboxy C₁₋₇-alkyl, amino, alkyl amino and acetamido.

Representative of this preferred compound is 5-chloro-1,3-dimethyl-1H-pyrazole-4-sulfonic acid {6-[3-butyl-1-(2-fluoro-benzyl)-2,6-dioxo-2,3,6,7-tetrahydro-1H-purin-8-ylmethyl]-pyridin-3-yl}-amide.

Another preferred compound of formula 1A is where R² is C₁₋₇-alkyl substituted by cyclobutyl, R³ is 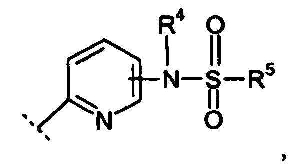 R⁴ is H and R⁵ is a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇-alkyl, halogen, carboxy C₁₋₇-alkyl, amino, alkyl amino and acetamido.

Representative of this preferred compound is 5-chloro-1,3-dimethyl-1H-pyrazole-4-sulfonic acid {6-[3-Cyclobutylmethyl-1-(2-fluoro-benzyl)-2,6-dioxo-2,3,6,7-tetrahydro-1H-purin-8-ylmethyl]-pyridin-3-yl}-amide.

Another preferred compound of formula 1 has the formula

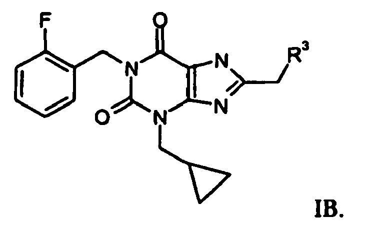

A preferred compound of formula IB is wherein R³ is 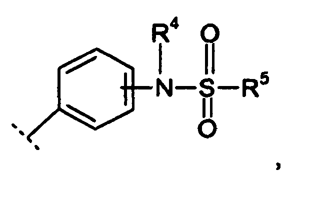 R⁴ is H and R⁵ is a 5-membered heteroaromatic ring having two heteroatoms wherein a first heteroatom is N, and a second heteroatom is independently selected from the group consisting of N, O and S, the 5-membered heteroaromatic ring being unsubstituted or substituted by at least one substituent selected from the group consisting of C₁₋₇-alkyl, halogen, carboxy C₁₋₇-alkyl, amino, alkyl amino and acetamido.

A further preferred compound of formula IB is where the 5-membered heteroaromatic ring with two heteroatoms has two C₁₋₇-alkyl substituents.

Exemplary of this preferred compound is a compound selected from the group consisting of
1,2-dimethyl-1H-imidazole-4-sulfonic acid {4-[3-cyclopropylmethyl-1-(2-fluorobenzyl)-2,6-dioxo-2,3,6,7-tetrahydro-1H-purin-8-ylmethyl]-phenyl}-amide;
3,5-dimethyl-isoxazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide; and
1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide.

An additional preferred compound of formula IB is where the 5-membered heteroaromatic ring with two heteroatoms has one C₁₋₇-alkyl substituent.

Representative of this preferred compound is a compound selected from the group consisting of
methyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide, and
methyl-1H-imidazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide.

A further preferred compound of formula IB is where the 5-membered heteroaromatic ring with two heteroatoms has one C₁₋₇-alkyl substituent and one amino substituent.

Exemplary of this preferred compound is the compound 2-amino-4-methylthiazole-5-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt.

A further preferred compound of formula IB is where the 5-membered heteroaromatic ring with two heteroatoms has one C₁₋₇-alkyl substituent and one acetamido substituent.

Exemplary of this preferred compound is *N*-(5-{4-[3-cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide.

Another preferred compound of formula IB is where the 5-membered heteroaromatic ring with two heteroatoms has one C₁₋₇-alkyl substituent and one halogen substituent.

Exemplary of this preferred compound is 5-chloro-1,3-dimethyl-1H-pyrazole-4-sulfonic acid {4-[3-ryclopropylmethyl-1-(2-fluoro-benzyl)-2,6-dioxo-2,3,6,7-tetrahydro-1 H-purin-8-ylmethyl]-phenyl}-amide.

Another preferred compound of formula IB is where the 5-membered heteroaromatic ring with two heteroatoms has three substituents selected from the group C₁₋₇-alkyl, halogen, carboxy C₁₋₇-alkyl, amino, alkyl amino and acetamido.

Exemplary of this preferred compound is 5-chloro-1,3-dimethyl-1H-pyrazole-4-sulfonic acid {3-[3-cyclopropylmethyl-1-(2-fluoro-benzyl)-2,6-dioxo-2,3,6,7-tetrahydro-1H-purin-8-ylmethyl]-phenyl}-amide.

Another preferred compound of formula IB is where R³ is 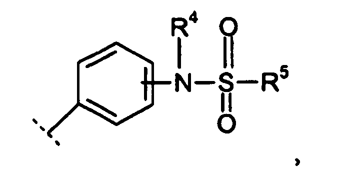 R⁴ is H and R⁵ is phenyl substituted by at least one substituent selected from the group consisting of C₁₋₇-alkyl, C₁₋₇-alkyl substituted by halogen, halogen, C₁₋₇-alkoxy, C₁₋₇-alkoxy substituted by halogen, nitro and acetamido.

A further preferred compound of formula IB is where R⁴ is H and R⁵ is phenyl substituted by one halogen.

Exemplary of this preferred compound is *N*-{4-[3-cyclopropylmethyl-1-(2-fluorobenzyl)-2,6-dioxo-2,3,6,7-tetrahydro-1H-purin-8-ylmethyl]-phenyl}-3-fluoro-benzenesulfonamide.

A further preferred compound of formula IB is where R⁴ is H and R⁵ is phenyl substituted by one alkoxy. Exemplary of this preferred compound is N-{4-[3-cyclopropylmethyl-1-(2-fluoro-benzyl)-2,6-dioxo-2,3,6,7-tetrahydro-1H-purin-8-ylmethyl]-phenyl}-3-methoxy-benzenesulfonamide.

A further preferred compound of formula IB is where R⁴ is H and R⁵ is phenyl substituted by one acetamido. Exemplary of this preferred compound is *N*-(4-{4-[3-cyclopropylmethyl-1-(2-fluoro-benzyl)-2,6-dioxo-2,3,6,7-tetrahydro-1H-purin-8-ylmethyl]-phenylsulfamoyl}-phenyl)-acetamide.

Another preferred compound of formula IB is where R⁴ is H and R⁵ is a 6-membered heteroaromatic ring having one N, the 6-membered heteroaromatic ring being unsubstituted or substituted by at least one substituent selected from the group consisting of C₁₋₇-alkyl, halogen, carboxy C₁₋₇-alkyl, amino, alkyl amino and acetamido.

A preferred compound of formula IB is where R⁴ is H and R⁵ is an unsubstituted 6-membered heteroaromatic ring having one N. Exemplary of this preferred compound is pyridine-3-sulfonic acid {4- [3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt.

Another preferred compound of IB is where R⁴ is H and R⁵ is a 6-membered heteroaromatic ring having one N is substituted by two halogen substituents.

Exemplary of this preferred compound is 5-bromo-6-chloro-pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide.

Another preferred compound of formula IB is where R³ is 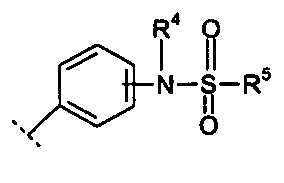 and
R⁴ is H is wherein R⁵ is C₁₋₇-alkyl. Exemplary of this preferred compound is *N*-{4-[3-cyclopropylmethyl-1-(2-fluoro-benzyl)-2,6-dioxo-2,3,6,7-tetrahydro-1H-purin-8-ylmethyl]-phenyl}-methanesulfonamide.

Another preferred compound of formula IB is where R³ is 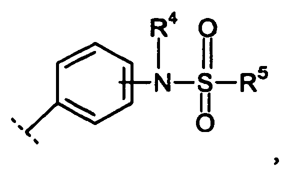 R⁴ is H, and where R⁵ is C₁₋₇-alkyl amino. Exemplary of this preferred compound is N-{4-[3-cyclopropylmethyl-1-(2-fluoro-benzyl)-2,6-dioxo-2,3,6,7-tetrahydro-1H-purin-8-ylmethyl]-phenyl}-*N,N*-dimethylsulfamide.

Another preferred compound of formula IB is where R³ is 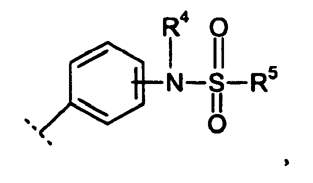 R⁴ is H, and R⁵ is 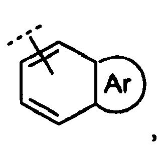 wherein Ar is selected from the group consisting of a 5-membered heteroaromatic ring fused to the 6-membered ring, having one, two, or three heteroatoms, and wherein a first heteroatom is N, a second heteroatom is N and a third heteroatom is selected from the group consisting of O and S, the 5-membered heteroaromatic ring being unsubstituted or substituted by at least one substituent selected from the group consisting of C₁₋₇-alkyl, halogen, carboxy C₁₋₇-alkyl, amino, alkyl amino and acetamido.

Exemplary of this preferred compound is quinoline-8-sulfonic acid {4-[3-cyclopropylmethyl-1-(2-fluoro-benzyl)-2,6-dioxo-2,3,6,7-tetrahydro-1H-purin-8-ylmethyl]-phenyl}-amide.

Another preferred compound of formula 1B is where R³ is 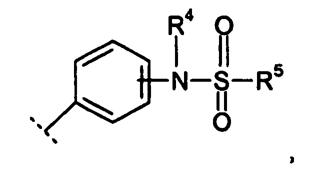 R⁴ is C₁₋₇-alkyl and R⁵ is a 5-membered heteroaromatic ring having two heteroatoms wherein a first heteroatom is N and a second heteroatom is independently selected from the group consisting of N, O and S, the 5-membered heteroaromatic ring being unsubstituted or substituted by at least one substituent selected from the group consisting of C₁₋₇-alkyl, halogen, carboxy C₁₋₇-alkyl, amino, alkyl amino and acetamido.

Exemplary of this preferred compound is a compound selected from the group consisting of
1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide; and 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methylamide.

Especially preferred is a compound of formula I selected from the group consisting of
5-chloro-1,3-dimethyl-1H-pyrazole-4-sulfonic acid {4-[3-cyclopropylmethyl-1-(2-fluoro-benzyl)-2,6-dioxo-2,3,6,7 - tetrahydro-1H-purin-8-ylmethyl]-phenyl}-amide, 1-methyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide
*N*-{4-[3-cyclopropylmethyl-1-(2-fluoro-benzyl)-2,6-dioxo-2,3,6,7-tetrahydro-1H-purin-8-ylmethyl]-phenyl}-3-methoxy-benzenesulfonamide,
*N*-{4-[3-cyclopropylmethyl-1-(2-fluoro-benzyl)-2,6-dioxo-2,3,6,7-tetrahydro-1H-purin-8-ylmethyl]-phenyl}-3-fluoro-benzenesulfonamide,
5-bromo-6-chloro-pyridine-3-sulfonic acid {4-[3-cydopropylmethyl-1-(2-fluorobenzyl)-2,6-dioxo-2,3,6,7-tetrahydro-1H-purin-8-ylmethyl]-phenyl}-amide, 5-chloro-1,3-dimethyl-1H-pyrazole-4-sulfonic acid {3- [3-cyclopropylmethyl-1-(2-fluoro-benzyl)-2,6-dioxo-2,3,6,7-tetrahydro-1H-purin-8-ylmethyl]-phenyl}-amide, 1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
*N*-(5-{4-[3-cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide,
2-amino-4-methyl-thiazole-5-sulfonic acid {4-[3-cyclopropylmethyl-1-(2-fluorobenzyl)-2,6-dioxo-2,3,6,7-tetrahydro-1H-purin-8-ylmethyl]-phenyl}-amide trifluoro-acetic acid salt,
3,5-dimethyl-isoxazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
1,2-dimethyl-1H-imidazole-4-sulfonic acid {4-[3-cyclopropylmethyl-1-(2-fluorobenzyl)-2,6-dioxo-2,3,6,7-tetrahydro-1H-purin-8-ylmethyl]-phenyl}-amide,
1-methyl-1H-imidazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
*N*-{4-[3-cyclopropylmethyl-1-(2-fluoro-benzyl)-2,6-dioxo-2,3,6,7-tetrahydro-1H-purin-8-ylmethyl]-phenyl}-*N*,*N*-dimethylsulfamide,
5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methylamide,
quinoline-8-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
*N*-(4-{4-[3-cyclopropylmethyl-1-(2-auoro-benzyl)-2,6-dioxo-2,3,6,7-tetrahydro-1H-purin-8-ylmethyl] -phenylsulfamoyl}-phenyl)-acetamide,
*N-* [4-(1-benzyl-3-butyl-2,6-dioxo-2,3,6,7-tetrahydro-1H-purin-8-ylmethyl)-phenyl]-4-methyl-benzenesulfonamide,
pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt,
*N*-{4-[3-cyclopropylmethyl-1-(2-fluoro-benzyl)-2,6-dioxo-2,3,6,7-tetrahydro-1H-purin-8-ylmethyl] -phenyl}-methanesulfonamide,
*N-* [4-(1-benzyl-3-butyl-2,6-dioxo-2,3,6,7-tetrahydro-1H-purin-8-ylmethyl)-phenyl]-methanesulfonamide,
5-chloro-1,3-dimethyl-1H-pyrazole-4-sulfonic acid {6-[3-butyl-1-(2-fluoro-benzyl)-2,6-dioxo-2,3,6,7-tetrahydro-1H-purin-8-ylmethyl]-pyridin-3-yl}-amide,
5-chloro-1,3-dimethyl-1H-pyrazole-4-sulfonic acid {6-[3-cyclobutylmethyl-1-(2-fluorobenzyl)-2,6-dioxo-2,3,6,7-tetrahydro-1H-purin-8-ylmethyl]-pyridin-3-yl}-amide,
1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide,
4-[3-butyl-1-(2-fluoro-benzyl)-2,6-dioxo-2,3,6,7-tetrahydro-1H-purin-8-ylmethyl]-*N-*pyridin-2-yl-benzenesulfonamide,
4-[3-butyl-1-(2-fluoro-benzyl)-2,6-dioxo-2,3,6,7-tetrahydro-1H-purin-8-ylmethyl]-*N-*pyridin-4-yl-benzenesulfonamide,
4- [3-butyl-1-(2-fluoro-benzyl)-2,6-dioxo-2,3,6,7-tetrahydro- 1H-purin-8-ylmethyl]-*N-*pyridin-3-yl-benzenesulfonamide,
4-[3-butyl-1-(2-fluoro-benzyl)-2,6-dioxo-2,3,6,7-tetrahydro-1H-purin-8-ylmethyl]-*N-*pyrimidin-2-yl-benzenesulfonamide, and
4-[3-butyl-1-(2-fluoro-benzyl)-2,6-dioxo-2,3,6,7-tetrahydro-1H-purin-8-ylmethyl]-*N-*(1,3,5-trimethyl-1H-pyrazol-4-yl)-benzenesulfonamide.

In a further embodiment, the present invention is concerned with a process for the preparation of a compound of formula I of the present invention, which process comprises cyclisation of a compound of formula II 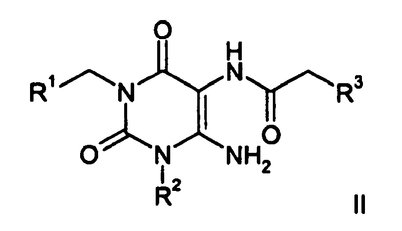 wherein R¹, R² and R³ are as defined in any one of claims 1 to 36, to yield the compound of formula I.

In a further embodiment, the invention relates to pharmaceutical compositions comprising a compound of formula I or a pharmaceutically acceptable salt thereof, and a pharmaceutically acceptable excipient.

In a further embodiment, the invention relates to the use of compounds of formula I as therapeutic active substances, especially to the use of compounds of formula for the preparation of medicaments for the treatment and/or prophylaxis of diseases which are associated with phosphoenolpyruvate carboxykinase, and more specifically to the use of compounds of formula I for the treatment of type 2 diabetes.

### Biological Testing:

### Purification of GST-PEPCK

E. coli cells expressing GST-PEPCK were suspended in 5 volumes of lysis buffer (50 mM Tris-hydroxymethyl aminomethyl (TRIS), 150 mM sodium chloride (NaCl), 10 mM ethylene-diametetraacetic acid (EDTA), 5 mM dithiothreitol (DTT), 1% Nonidet P-40, pH 7.4) in the presence of protease inhibitors, incubated with lysozyme at 200 micrograms/ml for 30 min. at room temperature, and sonicated 4x30 seconds at 4 °C. After centrifugation at 12,000x g for 20 min. to remove insoluble material, the supernatant was loaded onto glutathione Sepharose (Pharmacia), washed with lysis buffer followed by washing with lysis buffer in the absence of NP-40. GST-PEPCK was eluted with the same buffer containing 20 mM glutathione. The eluted protein was concentrated and dialyzed against 25 mM Hepes (*N*-[2-hydroxyethyl]piperazine-N'[2-ethanesulfonic acid), 150 mM NaCl, 2.5 mM EDTA, 5 mM DTT, 30% glycerol, pH 7.4, and stored at -20 °C.

### PEPCK Enzyme assay

The inhibitory effect of the compounds of the present invention on PEPCK enzymatic activity was determined using recombinant human cytosolic PEPCK, expressed and purified from E.coli as a GST-fusion. Guanosine triphosphate (GTP) and manganese dependent PEPCK enzyme activity catalyzed the decarboxylation of oxalacetate leading to the formation of guanosine diphosphate (GDP) and phosphoenol pyruvate (PEP). This reaction is coupled to pyruvate kinase and lactate dehydrogenase catalyzed reactions and the overall reaction rate determined by measuring the change in absorbance at 340nM (Chang, H.C. and Lane M.D., J. Biol.Chem. 241:2413-2420, 1966). The following modifications were made to the protocol: 2.5µg of recombinant, human cytosolic glutathione-S-transferase (GST)-PEPCK was added to a reaction mixture at room temperature which contained 0.3mM GTP, 0.3mM oxaloacetate (OAA), 3mM magnesium chloride (MgCl₂), 0.075mM manganese chloride (MnCl₂), 30mM potassium phosphates (K₂PO₄), pH 7.6,1mM dithiothreitol (DTT), 0.2mM adenosine diphosphate (ADP), 1mM nicotinamide adenine dinucleotide, reduced form (NADH), 0.9Units/ml each of pyruvate kinase and lactate dehydrogenase and 1mg/ml bovine serum albumin (BSA). Test compounds were added such that final concentration of DMSO was 10%. Reactions were run for twenty minutes.

Kₘ values for GTP and OAA were determined according to Michelis-Menten conditions as described in Cornish-Bowden (Fundamentals of Enzyme Kinetics, 1995) in essentially the coupled assay conditions described above. To determine the concentrations at which test compounds inhibited the enzyme 50% (IC₅₀), reaction mixtures containing 3 fold and 10 fold the calculated Kₘ values for GTP and OAA, respectively, were employed. Test compounds were added to reactions over a range of concentrations and IC₅₀'s were calculated from plots of inhibitor concentration versus enzyme rate. This method of determination of IC₅₀ values is equally applicable to calculations based on PEPCK cellular assay.

### General Description of Synthetic Pathways

### PEPCK Sulfonamide Derivatives -

Compounds with the general formula provided herein may be prepared by application of the appropriate transformations as outlined in schemes 1 through 7 inclusive. The typical strategy employed was to prepare an aniline bearing derivative of a 1,3,8-trisubstituted xanthine which were then reacted with a sulfonyl chloride to form the desired sulfonamide derivative. If the resulting sulfonamide derivatives also contain reactive functional groups, further derivatization was possible by application of standard chemical techniques. In cases where it was not possible to assemble the desired sulfonamide derivatives by reaction of an 1,3,8-trisubstituted xanthine containing an appropriately substituted aniline with a sulfonyl chloride, the desired derivatives were prepared by pre-assembling the sulfonamide component and incorporating this component into the synthetic scheme in place of the protected aniline or aniline precursor.

One method used for preparing some of the 1,3,8-trisubstituted xanthine derivatives bearing an aniline substituent which were subsequently used for preparation of sulfonamide derivatives is shown in scheme 1. Starting from commercially available 6-amino-1H-pyrimidine-2,4-dione 2, alkylation of the nitrogen at position 3 to give compounds of general structure 3 was achieved using the procedure of Müller as described in Tetrahedron Lett. 1991, 32(45), 6539. Compounds of general formula 2 were converted to their per-silylated derivatives by heating in commercially available 1,1,1,3,3,3-hexamethyldisilazane (HMDS) in the presence of a catalytic amount of commercially available ammonium sulfate. The intermediate per-silylated derivatives of compounds of general formula 2 were isolated by concentration under high vacuum and reacted immediately with the desired commercially available alkylating agent and a catalytic amount of commercially available elemental iodine at reflux. The reaction was judged complete when a good level of conversion to the 3-substituted derivatives of general formula 3 had been achieved (as judged by thin layer chromatography) and prior to the formation of significant amounts of undesired by-products.

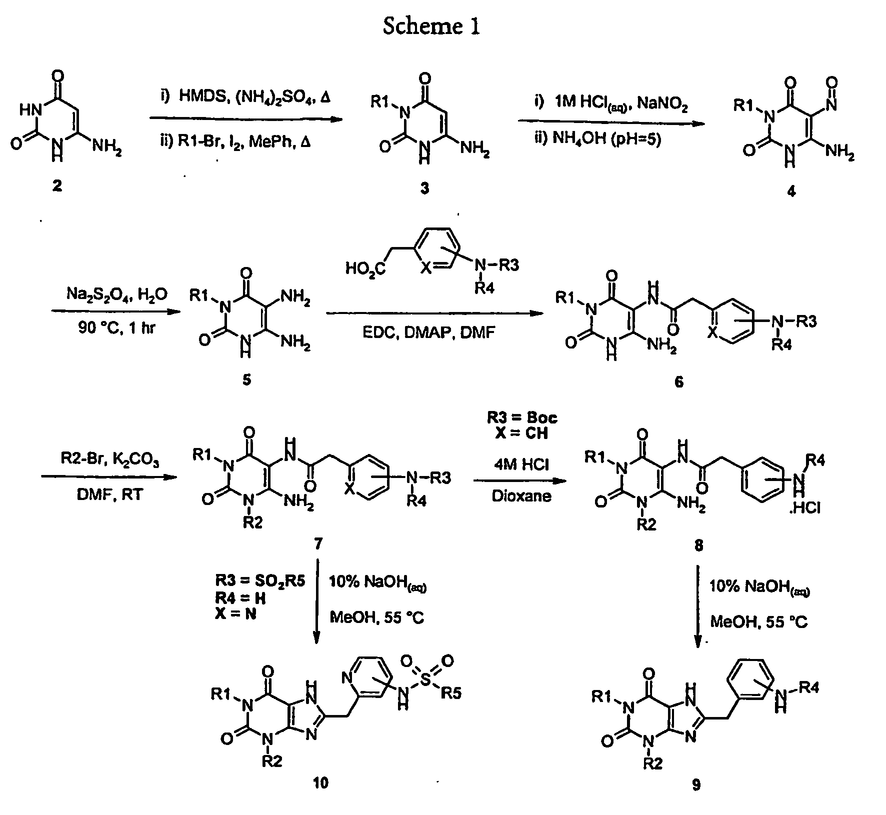

Nitrosylation of 3-substituted-6-amino-1H-pyrimidine-2,4-diones of general formula 3 was performed by use of a procedure similar to that of Müller et al. as described in J. Med. Chem. 1993, 36, 3341. Compounds of general formula 3 were heated in aqueous 1 Molar hydrochloric acid and treated with commercially available sodium nitrite to form the orange to red colored 6-amino-5-nitroso-1H-pyrimidine-2,4-diones of general formula 4 which were isolated as solids after treating the reaction mixture with 1 Molar aqueous ammonium hydroxide until the reaction mixture was at pH = 5. The crude products were precipitated, isolated by filtration and used without further purification. The 6-amino-5-nitroso-1H-pyrimidine-2,4-diones of general formula 4 were reduced to the 5,6-diamino-1H-pyrimidine-2,4-diones of general formula 5 by use of a procedure similar to that of Müller et al. as described in J. Med. Chem. 1993, 36, 3341.

Commercially available sodium dihydrosulfite was added portionwise to a suspension of the nitroso derivatives of general formula 4 in water at approximately 90 °C. The reaction was judged complete when the color of the nitroso compounds had been fully discharged. The 5,6-diamino-1H-pyrimidine-2,4-diones of general formula 5 were relatively unstable and were used immediately in the next step in the synthetic pathway without additional purification.

Acylation of the 5,6-diamino-1H-pyrimidine-2,4-diones of general formula 5 was performed according to the procedure of Jacobson et al. as described in J. Med. Chem. 1993, 36(10),1333. Treatment of a mixture of a diamine of general formula 5 with an appropriately substituted aryl or hetero-aryl acetic acid derivative and commercially available 1-(3-dimethylaminopropyl)-3-ethylcarbodiimide hydrochloride (EDC) resulted in amide formation selectively at the 5-position of the 1H-pyrimidine-2,4-dione ring to give amide derivatives of general formula 6. The aryl and hetero-aryl acetic acids used in this step were either commercially available or were prepared by the routes outlined in schemes 6 and 7.

5-Acetamido-6-amino uracil derivatives of general formula 6 were selectively alkylated at the 1 position of the 1H-pyrimidine-2,4-dione to give compounds of general structure 7 by use of the appropriate alkylating agent with anhydrous potassium carbonate in DMF by analogy with the procedure of Müller et al. as described in Synthesis 1995,1295. In the case where R³ in compounds of general formula 7 was a Boc-protecting group and the ring atom X was carbon with an attached hydrogen, the Boc-protecting group was removed by treatment with commercially available 4 Molar hydrogen chloride in p-dioxane solution. The crude hydrochloride salts of general formula 8 precipitated from the reaction mixture and were isolated by filtration and used without further purification. 5-Acetamido-6-amino derivatives of general formula 8 were cyclized to form the 1,3,8-trisubstituted xanthines of general formula 9 by heating to approximately 50 °C in methanol containing 10% aqueous sodium hydroxide solution in a manner similar to that described by Müller et al. in Synthesis 1995, 1295. The reactions were monitored by TLC until all of the starting 5-acetamido-6-amino derivative of general formula 8 had been consumed. The aniline bearing 1,3,8-trisubstituted xanthine derivative of general formula 9 were isolated from the reaction mixture with sufficient purity for subsequent chemical modification by extraction and concentration.

In the case where in compounds of general formula 7, R³ was a sulfonyl group and the ring atom X was nitrogen, cyclization to compounds of general formula 10 was also be achieved by heating to approximately 50 °C in methanol containing 10% aqueous sodium hydroxide solution as described previously.

Another route which was used for the preparation of 1,3,8-trisubstituted xanthine derivatives which either bear an aniline substituent suitable for subsequent derivatization into a sulfonamide group or a pre-formed sulfonamide group is shown in scheme 2. Commercially available ethyl cyanoacetate 12 and an appropriately substituted commercially available mono-substituted urea 11 were condensed in the presence of sodium ethoxide in refluxing ethanol according to the procedure of Papesch and Schroeder as described in J. Org. Chem. 1951,16,1879 to give 1-substituted-6-amino-1H-pyrimidine-2,4-diones of general formula 13.

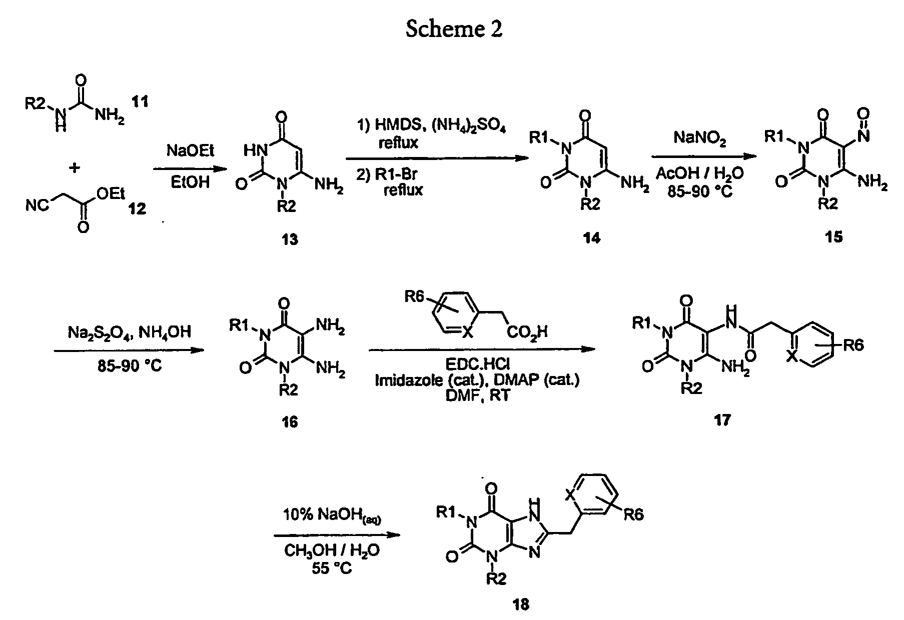

Substituted-6-amino-1H-pyrimidine-2,4-diones of general formula 13 were then alkylated at the 3-position by application of the method of Müller et al. as described in J. Med. Chem. 1993, 36, 3341. This method is similar to that described above for scheme 1 wherein compounds of general formula 2 are converted into compounds of general formula 3 and involved the mono-alkylation of the per-silylated derivatives of compounds of general formula 13 with an appropriate alkylating agent to give the 1,3-dialkylated compounds of general formula 14.

Nitrosylation of 1,3-disubstituted-6-amino-1H-pyrimidine-2,4-diones of general formula 14 was performed according to the procedure of Müller et al. as described in J. Med. Chem. 1993, 36, 3341. Compounds of general formula 14 were heated in aqueous acetic acid and treated with commercially available sodium nitrite to form the orange to red colored 6-amino-5-nitroso-1H-pyrimidine-2,4-diones of general formula 15 which were isolated by filtration as solids after cooling to 0 °C.

The 6-amino-5-nitroso-1H-pyrimidine-2,4-diones 15 were reduced to the 5,6-diamino-1H-pyrimidine-2,4-diones of general formula 16 with commercially available sodium dihydrosulfite in 10% aqueous ammonium hydroxide at approximately 90 °C. This method is based on the method described by Müller et al. in J. Med. Chem. 1993, 36, 3341. The reaction was judged complete when the color of the nitroso compounds had been fully discharged. The 5,6-diamino-1H-pyrimidine-2,4-diones of general formula 16 were relatively unstable and were used immediately in the next step in the synthetic pathway without additional purification.

Acylation of the 5,6-diamino-1H-pyrimidine-2,4-diones of general formula 16 was performed according to the procedure of Jacobson et al. as described in J. Med. Chem. 1993, 36(10), 1333. Treatment of a mixture of a diamine of general formula 16 and the appropriately substituted phenyl or heterocyclic acetic acid derivative with commercially available 1-(3-dimethylaminopropyl)-3-ethylcarbodiimide hydrochloride (EDC) resulted in amide formation selectively at the 5-position of the 1H-pyrimidine-2,4-dione ring to give compounds of general formula 17. Amide derivatives of general formula 17 were stable products which were typically not purified following isolation of the crude reaction product. In the case where R⁶ = -NR³R⁴, compounds of general formula 17 were identical with compounds of general formula 7 and could be derivatized in a similar fashion (as shown in scheme 1). In the case where R⁶ = -N(Boc)R⁴, the protected aniline was carried through the xanthine cyclization step to form compounds of general formula 18 and removed subsequently (see scheme 4). R⁶ could also be a sulfonamide group which was attached to the aryl or hetero-aryl ring via nitrogen. In addition, R⁶ could be a sulfonamide group which was attached to the phenyl ring (X = CH) through sulfur. The requisite sulfonamide substituted aryl acetic acids were prepared as outlined in scheme 6. 5-Acetamido-6-amino derivatives of general formula 17 were cyclized to form the 1,3,8-trisubstituted xanthines of general formula 18 by heating to approximately 50 °C in methanol containing 10% aqueous sodium hydroxide solution in a manner similar to that described by Müller et al. in Synthesis 1995,1295. The reactions were monitored by TLC until all of the starting 5-acetamido-6-amino derivatives of general formula 17 had been consumed. Pure products were obtained using standard chemical purification techniques such as chromatography, crystallization or trituration.

A third route which was used to prepare some of the 1,3,8-trisubstituted xanthine derivatives claimed in this patent application is shown in scheme 3. Starting from commercially available 6-chloro-1H-pyrimidine-2,4-dione 19, selective alkylation of the nitrogen at the 1-position of the pyrimidine ring was achieved with the appropriate alkylating agent to give compounds of general formula 20 by use of a method similar to that of Ishikawa et al. as described in Heterocycles 1990, 31 (9), 1641. Using the same reaction conditions and a second alkylating agent (or a second equivalent of the first alkylating agent) a substituent was then introduced at the 3-position of the pyrimidine ring to furnish compounds of general formula 21.

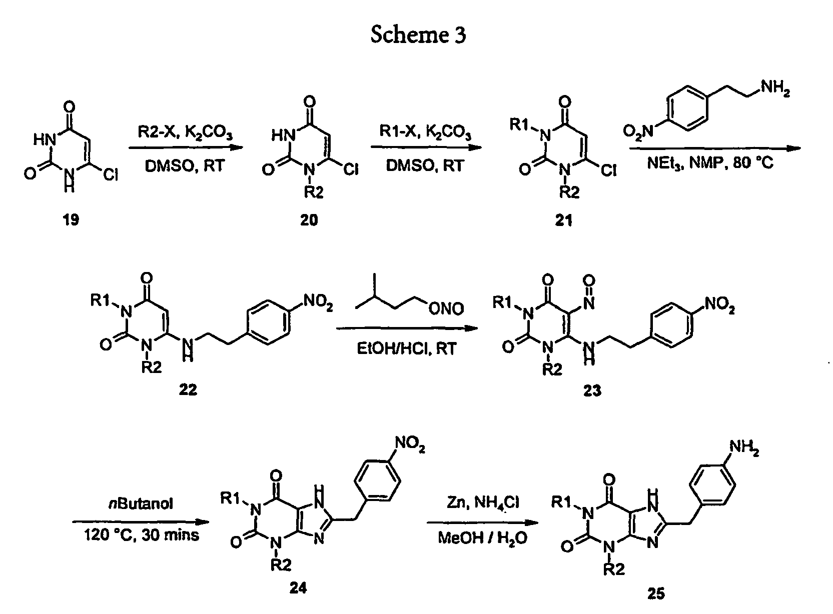

Displacement of chloride from compounds of general formula 21 with 2-(4-nitrophenyl)-ethylamine hydrochloride salt and triethylamine in *N*-methylpyrrolidinone (NMP) at 75 °C results in formation of compounds of general formula 22. These procedures were similar in nature to those previously described by Müller et al. in J. Med. Chem. 1993, 36, 3341 and by Shamin et al. in J. Med. Chem. 1989, 32(6), 1231 wherein similar transformations are performed on N¹-unsubstituted uracils.

Treatment of compounds of general formula 22 with commercially available isoamyl nitrite resulted in introduction of a nitroso group at the 5-position of the uracil ring as in compounds of general formula 23.

Cyclization of the 5-nitroso-6-amino substituted uracil derivatives of general formula 23 to the trisubstituted xanthines of general formula 24 was effected in refluxing n-butanol as shown in scheme 3. Compounds of general formula 24 were then reduced to the corresponding anilines of general formula 25 with zinc powder and ammonium chloride in aqueous methanol.

In the case where the final product from scheme 2, compounds of general formula 18, was a Boc-protected aniline, the protecting group had to be removed prior to further functionalization of the aniline nitrogen. As shown in scheme 4, when the protecting group was Boc as.in compounds of general formula 26, the aniline was liberated by treatment with commercially available 4M hydrogen chloride in p-dioxane solution to give anilines of general formula 27 (which were isolated as the hydrochloride salts). Anilines of general formula 27 were equivalent with anilines of general formula 9 and 25 prepared as shown in schemes 1 and 3 respectively. Reaction of anilines prepared by either of these 3 routes with a sulfonyl chloride resulted in formation of sulfonamide derivatives of general formula 28. In the case where R⁵ contained chemically reactive groups further modification of this residue was possible by application of appropriate standard chemical techniques.

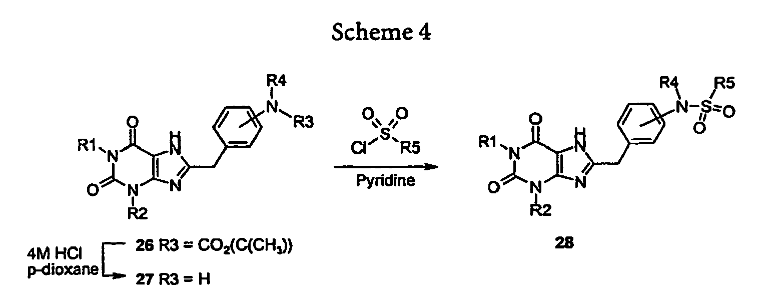

It was also possible to form a sulfonamide with anilines of general formula 8 prior to cyclization to the xanthine as shown in scheme 5. The sulfonamide containing 5-acetamido-6-amino uracil derivatives of general formula 29 were cyclized to the sulfonamide containing xanthine derivatives of general formula 30 using the standard conditions described previously.

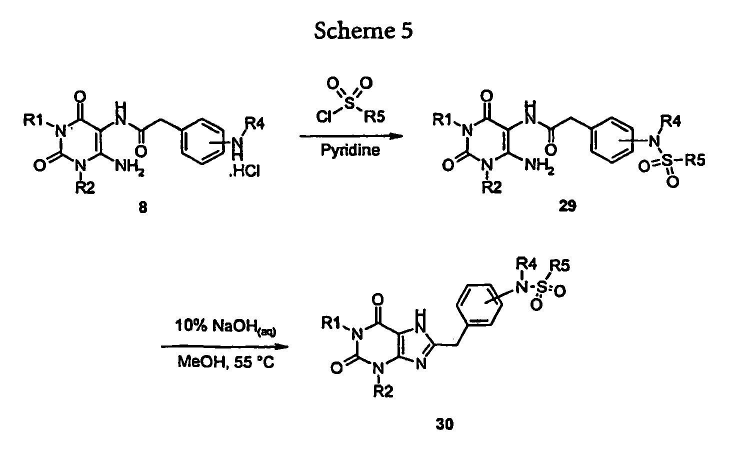

In scheme 2, when the aryl acetic acid component used to acylate diamines of general formula 16 contained a sulfonamide group which was attached to the aryl ring through sulfur, the aryl acetic acid components were prepared as shown in scheme 6. (4-Chlorosulfonyl-phenyl)-acetic acid ethyl ester (compound 32) was prepared from commercially available phenylacetic acid ethyl ester with commercially available chlorosulfonic acid according to the procedure of Kawashima et al. as described in Chem. Pharm. Bull. 1995, 43(7), 1132. Sulfonyl chlorides of general formula 32 were condensed with amine derivatives to give sulfonamides of general formula 33 in pyridine either between 0 °C and room temperature or by warming to 50 °C. The ethyl ester was removed by heating with potassium hydroxide in ethanol followed by treatment with aqueous hydrochloric acid to form carboxylic acids of general formula 34.

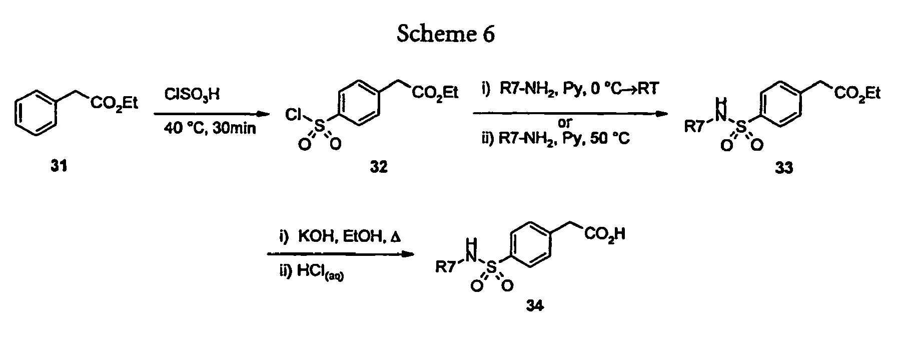

In schemes 1 and 2, when the acetic acid component used to acylate diamines of general formula 5 and 16 respectively was attached to a sulfonamide substituted pyridine ring, the heteroaryl acetic acid component was prepared by application of the procedures outlined in scheme 7. The potassium salt of dibenzyl malonate was used to displace chloride from commercially available 2-chloro-5-nitro-pyridine in dimethylsulfoxide solution at 95 °C. Reduction of the nitro group in compounds of general formula 36 was achieved using zinc powder and ammonium chloride in aqueous methanol to give the 5-amino-pyridines of general formula 37. 5-Amino-pyridines of general formula 37 were then condensed with sulfonyl chloride derivatives under standard conditions to give sulfonamides of general formula 38. When subjected to hydrogenolysis using 1 atmosphere pressure of hydrogen and 10% palladium on carbon catalyst the dibenzyl malonates of general formula 38 are converted into sulfonamide substituted 2-pyridylacetic acid derivatives of general formula 39.

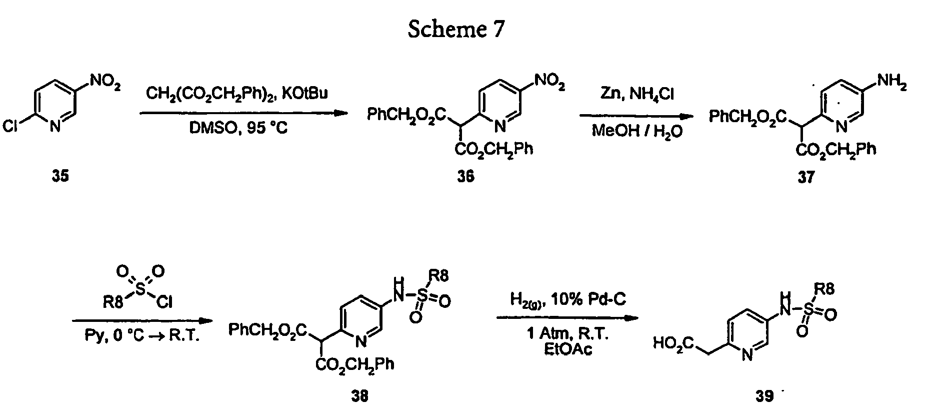

### Examples

### Example 1

### 5-Chloro-1,3-dimethyl-1H-pyrazole-4-sulfonic acid {4-[3-rydopropylmethyl-1-(2-fluoro-benzyl)-2,6-dioxo-2,3,6,7-tetrahydro-1H-purin-8-ylmethyl]-phenyl}-amide

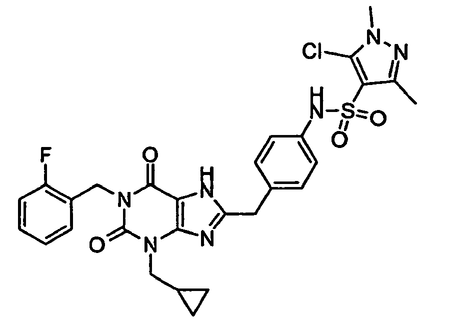

This compound was prepared by the routes outlined in schemes 1 and 4.

### Step 1: Preparation of 6-amino-3-(2-fluoro-benzyl)-1H-pyrimidine-2,4-dione.

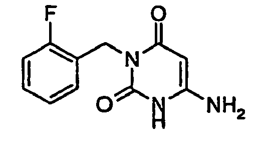

A mixture of 4-amino-2,6-dihydroxypyrimidine (10 g, 79.0 mmol), ammonium sulfate (570 mg, 3.95 mmol), and hexamethyldisilizane (60 mL, 292.3 mmol) was heated to reflux for 3.25 h. At this time, the reaction was cooled to 25°C and concentrated in vacuo to afford a white solid. The solid was mixed with toluene (12 mL) under argon and then treated with 2-fluorobenzyl bromide (12.6 mL, 102.7 mmol) and iodine (470 mg, 1.58 mmol). This mixture was heated to reflux for 2 h. A brown suspension was formed. The reaction was stirred at 25 °C overnight. At this time, the reaction mixture was cooled to 0°C and then treated with a solution of sodium thiosulfate (2.47 g in 40 mL of water). A very thick suspension formed which needed to be stirred by hand. The solids were broken up with a spatula. The mixture was then treated with a saturated aqueous sodium bicarbonate solution (300 mL) and was stirred at 0 °C for 30 min. The resulting solid was collected by filtration and washed well with water, toluene, and then ether. The solid was then dried in vacuo to afford 6-amino-3-(2-fluoro-benzyl)-1H-pyrimidine-2,4-dione (20.80 g, quant.) as a tan solid; LRMS for C₁₁H₁₀FN₃O₂S(M+H)⁺ at m/z = 236.

### Step 2: Preparation of 6-amino-3-(2-fluoro-benzyl)-5-nitroso-1H-pyrimidine-2,4-dione.

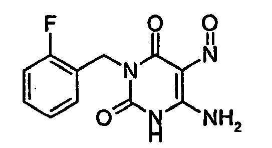

A mixture of 6-amino-3-(2-fluoro-benzyl)-1H-pyrimidine-2,4-dione (2.4 g, 10.6 mmol) in a 1.0 N aqueous hydrochloric acid solution at 25 °C was treated with a solution of sodium nitrite (0.95 g, 13.78 mmol) in water (10 mL). The mixture was stirred at 25 °C for 3 h. At this time, the reaction was brought to pH = 5 through treatment with a 1.0 N aqueous ammonium hydroxide solution. The resulting solid was collected by filtration and washed with cold water to afford 6-amino-3-(2-fluoro-benzyl)-5-nitroso-1H-pyrimidine-2,4-dione (2.58g, 92%); LRMS for C₁₁H₉FN₄O₃ (M+H)⁺ at m/z = 265.

### Step 3: Preparation of 5,6-diamino-3-(2-fluoro-benzyl)-1H-pyrimidine-2,4-dione.

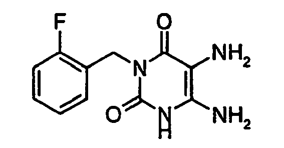

A mixture of 6-amino-3-(2-fluoro-benzyl)-5-nitroso-1H-pyrimidine-2,4-dione in water at 80 °C was treated with sodium hydrosulfite (5.94 g, 34.16 mmol). The reaction was stirred at 80 °C for 30 min. At this time, the reaction mixture was cooled to 0 °C for 10 min. The resulting solids were collected by filtration, rinsed with cold water and cold ether, and dried in vacuo to afford 5,6-diamino-3-(2-fluoro-benzyl)-1H-pyrimidine-2,4-dione (2.34g, 96%); LRMS for C₁₁H₁₁FN₄O₂ (M+H)⁺ at m/z = 251.

### Step 4: Preparation of (4-{[6-amino-3-(2-fluoro-benzyl)-2,4-dioxo-1,2,3,4-tetrahydro-pyrimidin-5-ylcarbamoyl]-methyl}-phenyl)-carbamic acid tert-butyl ester.

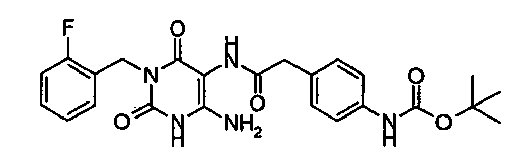

A solution of (4-tert-butoxycarbonylamino-phenyl)-acetic acid in *N,N-*dimethylformamide under argon at 25 °C was treated with 1-(3-dimethylaminopropyl)-3-ethylcarbodiimide hydrochloride. The reaction was stirred at 25 °C for 5 min. At this time, the reaction was treated with 5,6-diamino-3-(2-fluoro-benzyl)-1H-pyrimidine-2,4-dione and 4-dimethylaminopyridine. The reaction was stirred at 25 °C for 18 h. At this time, the reaction was concentrated in vacuo. The residue was diluted with water (75 mL) and then acidified to pH=5 with a 1.0 N aqueous hydrochloric acid solution. The resulting solid was collected by filtration, rinsed well with water and cold ether, and dried in vacuo to afford (4-{[6-amino-3-(2-fluoro-benzyl)-2,4-dioxo-1,2,3,4-tetrahydro-pyrimidin-5-ylcarbamoyl]-methyl}-phenyl)-carbamic acid tert-butyl ester (4.10 g, 91%) as a yellow solid; LRMS for C₂₈H₃₂FN₅O₅ (M+H)⁺ at m/z = 538.

### Step 5: Preparation of (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-carbamic acid tert-butyl ester.

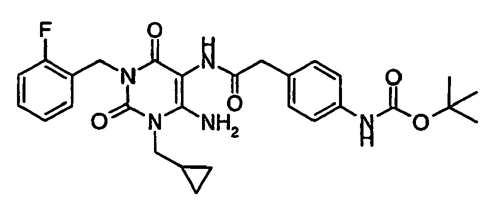

A solution of (4-{[6-amino-3-(2-fluoro-benzyl)-2,4-dioxo-1,2,3,4-tetrahydro-pyrimidin-5-ylcarbamoyl]-methyl}-phenyl)-carbamic acid tert-butyl ester in *N,N-*dimethylformamide (2.5 g, 5.17 mmol) at 25 °C under argon was treated with potassium carbonate (6.43 g, 46.53 mmol) followed by cyclopropyl methyl bromide (752 mg, 7.76 mmol). The suspension was stirred at 25 °C overnight. At this time, the reaction was concentrated in vacuo. The residue was diluted with a solution of water (100 mL) and a 1.0 N aqueous hydrochloric acid solution (31 mL). The resulting suspension was diluted with chloroform and neutralized with a 1.0 N aqueous hydrochloric acid solution. The layers were separated. The aqueous layer was extracted with chloroform. The organic layers were washed with a saturated aqueous sodium chloride solution, dried over magnesium sulfate, filtered, and concentrated in vacuo. Flash chromatography (Merck Silica gel 60, 230-400 mesh, 97/3 methylene chloride/methanol) afforded (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-carbamic acid tert-butyl ester (1.45 g, 52%) as an orange foam; EI-HRMS m/e calculated for C₂₈H₃₂FN₅O₅ (M⁺) 537.2387, found 537.2387.

### Step 6: Preparation of N-[6-amino-1-cyclopropylmethyl-3-(2-fluoro-benzyl)-2,4-dioxo-1,2,3,4-tetrahydro-pyrimidin-5-yl]-2-(4-amino-phenyl)-acetamide, hydrochloride salt.

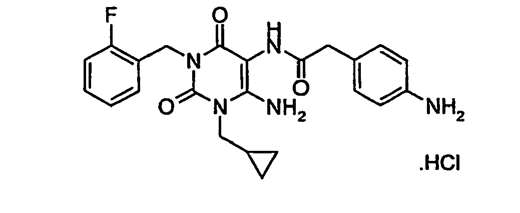

A solution of (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-carbamic acid tert-butyl ester (2.20 g, 4.10 mmol) in a solution of a 4.0 N aqueous hydrochloric acid solution in dioxane (37.0 mL) was stirred at 25 °C for 1 h. A suspension formed during this time. The reaction mixture was cooled to 0 °C. The resulting solid was collected by filtration, washed with dioxane and cold diethyl ether, and dried in vacuo to afford N- [6-amino-1-cyclopropylmethyl-3-(2-fluoro-benzyl)-2,4-dioxo-1,2,3,4-tetrahydro-pyrimidin-5-yl]-2-(4-amino-phenyl)-acetamide, hydrochloride salt (1.73g, 89%); LRMS for C₂₃H₂₃FN₅O₃ (M+H)⁺ at m/z = 438.

### Step 7: Preparation of 8-(4-amino-benzyl)-3-ryclopropylmethyl-1-(2-fluoro-benzyl)-3,4,5,7-tetrahydro-purine-2,6-dione.

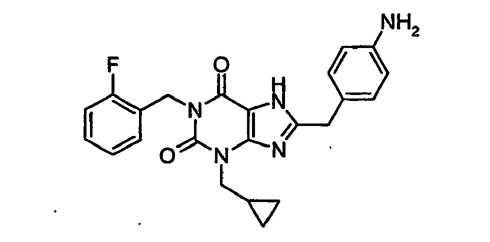

A solution of *N*-[6-amino-1-cyclopropylmethyl-3-(2-fluoro-benzyl)-2,4-dioxo-1,2,3,4-tetrahydro-pyrimidin-5-yl]-2-(4-amino-phenyl)-acetamide (850 mg, 1.79 mmol) in methanol (20 mL) at 25 °C was treated with a 10% aqueous sodium hydroxide solution (10.7 mL). The reaction mixture was heated to 55 °C for 4.5 h. At this time, reaction was diluted with chloroform (100 mL) and a saturated aqueous sodium chloride solution (15 mL). The layers were separated. The aqueous layer was extracted with chloroform, neutralized with a 1.0 N aqueous hydrochloric acid solution, and then reextracted with chloroform. The organic layers were combined and washed with a saturated aqueous sodium chloride solution, dried over magnesium sulfate, filtered, and concentrated in vacuo to afford 8-(4-amino-benzyl)-3-cyclopropylmethyl-1-(2-fluorobenzyl)-3,4,5,7-tetrahydro-purine-2,6-dione (607 mg, 81%) as a pale yellow solid; LRMS for C₂₈H₂₉FN₇O₅S (M+H)⁺ at m/z = 630.

### Step 8: Preparation of 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide.

A solution of 8-(4-amino-benzyl)-3-cyclopropylmethyl-1-(2-fluoro-benzyl)-3,7-dihydro-purine-2,6-dione (2.0 g, 4.77 mmol) in pyridine (30 mL) under argon at 25 °C was treated with 5-chloro-1,3-dimethyl-pyrazole-4-sulfonyl chloride (1.31 g, 5.72 mmol). The reaction mixture was stirred at 25 °C for 24 h. The reaction mixture was then diluted with methylene chloride (500 mL) and washed with a 1.0 N aqueous hydrochloric acid solution (3 x 100 mL). The combined water layers were extracted with methylene chloride (1 x 250 mL). The combined organics were washed with a saturated aqueous sodium chloride solution (1 x 100 mL), dried over magnesium sulfate, filtered, and concentrated in vacuo. The resulting solid was stirred in acetonitrile (25 mL) for 20 min and then placed in the freezer for 1 h. The solids were collected by filtration, washed with cold acetonitrile, and dried in vacuo to afford 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (2.53 g, 87%) as a yellow solid; FAB-HRMS m/e calculated for C₂₈H₂₇ClFN₇O₄S (M+H)⁺ 612.1596, found 612.1585.

The compounds cited in examples 2 to 6 were obtained in an anologous manner to that described in Example 1.

### Example 2

### Methyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide

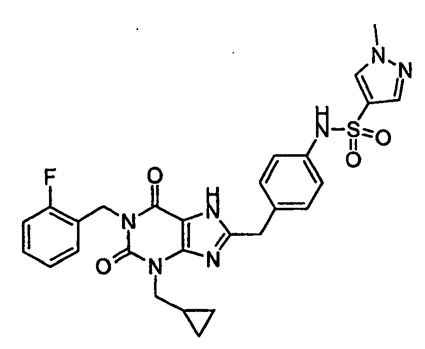

Prepared from 8-(4-amino-benzyl)-3-cyclopropylmethyl-1-(2-fluoro-benzyl)-3,7-dihydro-purine-2,6-dione and 1-methyl-1H-pyrazole-4-sulfonyl chloride. Off-white solid; EI-HRMS m/e calculated for C₂₇H₂₆FN₇O₄S (M⁺) 563.1751, found 563.1752.

### Example 3

### N-{4-[3-Cyclopropylmethyl-1-(2-fluoro-benzyl)-2,6-dioxo-2,3,6,7-tetrahydro-1H-purin-8-ylmethyl]-phenyl}-3-methoxy-benzenesulfonamide

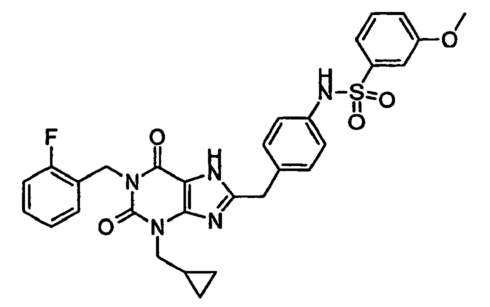

Prepared from 8-(4-amino-benzyl)-3-cyclopropylmethyl-1-(2-fluoro-benzyl)-3,7-dihydro-purine-2,6-dione and 3-methoxy-benzenesulfonyl chloride. White solid; EI-HRMS m/e calculated for C₃₀H₂₈FN₅O₅S (M⁺) 589.1795, found 589.1801.

### Example 4

### N-{4-[3-Cyclopropylmethyl-1-(2-fluoro-benzyl)-2,6-dioxo-2,3,6,7-tetrahydro-1H-purin-8-ylinethyl] -phenyl}-3-fluoro-benzenesulfonamide

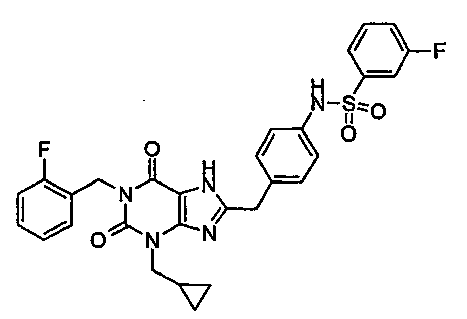

Prepared from 8-(4-amino-benzyl)-3-cyclopropylmethyl-1-(2-fluoro-benzyl)-3,7-dihydro-purine-2,6-dione and 3-fluoro-benzenesulfonyl chloride. Tan solid; EI-HRMS m/e calculated for C₂₉H₂₅F₂N₅O₄S (M+) 577.1595, found 577.1599.

### Example 5

### 5-Bromo-6-chloro-pyridine-3-sulfonic acid {4-[3-cyclopropylmethyl-1-(2-fluorobenzyl)-2,6-dioxo-2,3,6,7-tetrahydro-1H-purin-8-ylmethyl]-phenyl}-amide

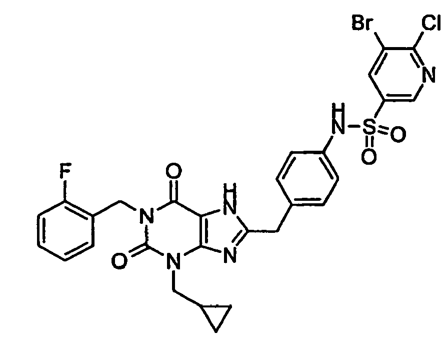

Prepared from 8-(4-amino-benzyl)-3-cyclopropylmethyl-1-(2-fluoro-benzyl)-3,7-dihydro-purine-2,6-dione and 3-fluoro-benzenesulfonyl chloride. Pink solid; EI-HRMS m/e calculated for C₂₈H₂₃BrClFN₆O₄S (M+H)⁺ 673.0430, found 673.0439.

### Example 6

### 5-Chloro-1,3-dimethyl-1H-pyrazole-4-sulfonic acid {3-[3-cyclopropylmethyl-1-(2-fluoro-benzyl)-2,6-dioxo-2,3,6,7-tetrahydro-1H-purin-8-ylmethyl]-phenyl}-amide

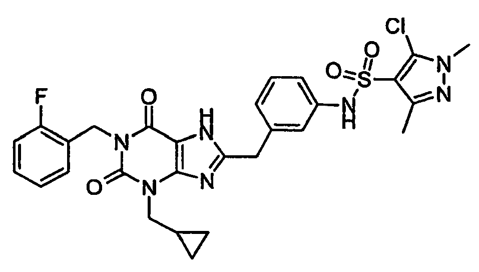

Prepared from 8-(3-amino-benzyl)-3-cyclopropylmethyl-1-(2-fluoro-benzyl)-3,7-dihydro-purine-2,6-dione and 5-chloro-1,3-dimethyl-1H-pyrazole-4-sulfonyl chloride. Light yellow solid; (ES)⁺-HRMS m/e calculated for C₂₈H₂₇ClFN₇O₄S (M+Na)⁺ 634.1411, found 634.1410.

### Example 7

### 1,3-Dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide

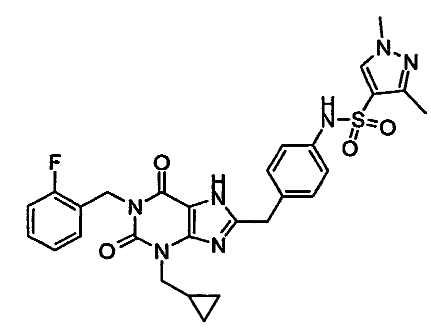

A mixture of 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (300 mg, 0.49 mmol) in methylene chloride (125 mL) and methanol (125 mL) at 25 °C was treated with 10% Pd/C (1.0 g) and sodium acetate (134 mg, 1.22 mmol). The resulting mixture was hydrogenated in a Parr bomb at 50 psi for 24 h. At this time, the reaction was filtered through a pad of celite and was washed with an 8/2 methylene chloride/methanol solution (200 mL). The filtrate was concentrated in vacuo to afford a white solid. This solid was dissolved in a 5/95 methanol/methylene chloride solution. The organics were washed with water (1 x 25 mL). The aqueous layer was then back-extracted with chloroform (1 x 50 mL). The combined organics were dried over magnesium sulfate, filtered, and concentrated in vacuo to afford 1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (184 mg 65%) as a white solid; (ES)⁺-HRMS m/e calculated for C₂₈H₂₈FN₇O₄S (M+H)⁺ 578.1980, found 578.1987.

### Example 8

### 5-Chloro-1,3-dimethyl-1H-pyrazole-4-sulfonic acid {4-[1,3-dimethyl-2,6-dioxo-2,3,6,7-tetrahydro-1H-purin-8-ylmethyl]-phenyl}-amide

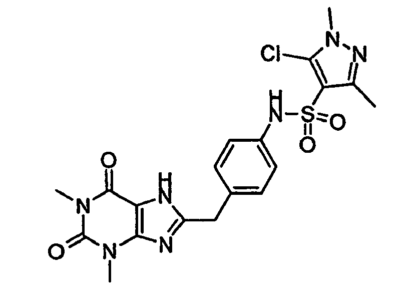

This compound was prepared by a combination of the routes outlined in schemes 1, 2 and 4.

### Step 1: Preparation of 6-amino-1,3-dimethyl-5-nitroso-1H-pyrimidine-2,4-dione.

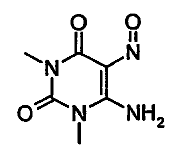

Commercially available 6-amino-1,3-dimethyl-1H-pyrimidine-2,4-dione (10 mmol) was dissolved in 1:1 water / glacial acetic acid (50 mL total volume) at 80 °C and sodium nitrite (20 mmol) added in portions. After the addition was complete the reaction mixture was stirred at 80 °C for 1 hr. Cooled in ice for 30 mins then filtered and washed with water. The precipitate was dried in vacuo to give crude 6-amino-1,3-dimethyl-5-nitroso-1H-pyrimidine-2,4-dione as ared-violet colored solid which was of sufficient purity for subsequent use without additional purification (89%).

### Step 2: Preparation of 5,6-diamino-1,3-dimethyl-1H-pyrimidine-2,4-dione.

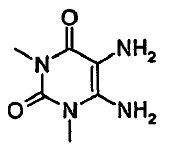

Crude 6-amino-1,3-dimethyl-5-nitroso-1H-pyrimidine-2,4-dione (5 mmol) was suspended in water (7 mL) at 40 °C and treated with sodium dithionite (17.5 mmol). After 20 minutes at 40 °C the reaction mixture was warmed to 80°C for 30 mins and then cooled in ice for 30 mins. The precipitate was isolated by filtration, washed with cold water and dried in vacuo to give crude 5,6-diamino-1,3-dimethyl-1H-pyrimidine-2,4-dione as a light beige solid which was of sufficient purity for subsequent use without additional purification (80%).

### Step 3: Preparation of {4-[(6-amino-1,3-dimethyl-2,4-dioxo-1,2,3,4-tetrahydro-pyrimidin-5-ylcarbamoyl)-methyl]-phenyl}-carbamic acid tert-butyl ester.

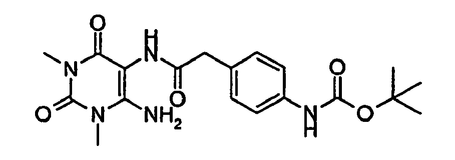

To a solution of (4-tert-butoxycarbonylamino-phenyl)-acetic acid (0.825 mmol) and crude 5,6-diamino-1,3-dimethyl-1H-pyrimidine-2,4-dione (0.75 mmol) in *N,N-*dimethylformamide (3 mL) under argon at 25 °C was added 1-(3-dimethylaminopropyl)-3-ethylcarbodiimide hydrochloride(0.825 mmol) and 4-dimethylaminopyridine (0.15 mmol). The reaction was stirred at 25 °C for 18 h. At this time, the reaction was concentrated in vacuo. The residue was triturated with water and the resulting solid collected by filtration, washed well with water and dried in vacuo to afford crude {4-[(6-amino-1,3-dimethyl-2,4-dioxo-1,2,3,4-tetrahydro-pyrimidin-5-ylcarbamoyl)-methyl]-phenyl}-carbamic acid tert-butyl ester as a light beige solid which was of sufficient purity for subsequent use without additional purification (87%).

### Step 4: Preparation of N-(6-amino-1,3-dimethyl-2,4-dioxo-1,2,3,4-tetrahydro-pyrimidin-5-yl)-2-(4-amino-phenyl)-acetamide hydrochloride salt.

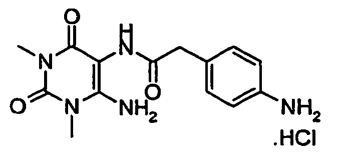

Crude {4-[(6-amino-1,3-dimethyl-2,4-dioxo-1,2,3,4-tetrahydro-pyrimidin-5-ylcarbamoyl) -methyl]-phenyl}-carbamic acid tert-butyl ester (0.65 mmol) was added to a 4M solution of hydrogen chloride in p-dioxane (10 mL) and the mixture stirred at ambient temperature for 2 hrs. The precipitate was isolated by filtration, washed with p-dioxane and ether then dried in vacuo to give crude *N*-(6-amino-1,3-dimethyl-2,4-dioxo-1,2,3,4-tetrahydro-pyrimidin-5-yl)-2-(4-amino-phenyl)-acetamide hydrochloride salt as a light beige solid which was of sufficient purity for subsequent use without additional purification (quantitative).

### Step 5: Preparation of 8-(4-amino-benzyl)-1,3-dimethyl-3,7-dihydro-purine-2,6-dione.

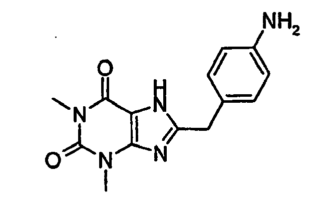

To a solution of crude *N*-(6-amino-1,3-dimethyl-2,4-dioxo-1,2,3,4-tetrahydro-pyrimidin-5-yl)-2-(4-amino-phenyl)-acetamide hydrochloride salt (0.68 mmol) in methanol (35 mL) was added 10% w/v aqueous sodium hydroxide (3.4 mL) and the mixture heated to 50°C for 7 hrs. The reaction mixture was concentrated in vacuo to dryness and the residue triturated with warm tetrahydrofuran and filtered to remove insoluble inorganic material. The filtrate was concentrated in vacuo to give crude 8-(4-amino-benzyl)-1,3-dimethyl-3,7-dihydro-purine-2,6-dione which was of sufficient purity for subsequent use without additional purification (58%).

### Step 6: Preparation of 5-chloro-1,3-dimethyl-1H-pyrazole-4-sulfonic acid {4-[1,3-dimethyl-2,6-dioxo-2,3,6,7-tetrahydro-1H-purin-8-ylmethyl]-phenyl}-amide.

To a solution of crude 8-(4-amino-benzyl)-1,3-dimethyl-3,7-dihydro-purine-2,6-dione (0.2 mmol) in pyridine (2 mL) at 0 °C was added 5-chloro-1,3-dimethyl-1H-pyrazole-4-sulfonyl chloride (0.2 mmol) and the mixture left to stir and warm slowly to ambient temperature overnight The reaction mixture was poured into ethyl acetate, washed with 1M aqueous hydrochloric acid until all pyridine was removed from the organic phase and the combined aqueous washings back extracted with ethyl acetate. The combined organic layers were dried and concentrated in vacuo to give an orange solid. Purified by trituration with warm acetonitrile to give 5-chloro-1,3-dimethyl-1H-pyrazole-4-sulfonic acid {4-[1,3-dimethyl-2,6-dioxo-2,3,6,7-tetrahydro-1H-purin-8-ylmethyl]-phenyl}-amide as a pale orange solid (51%); EI-HRMS m/e calculated for C₁₉H₂₀ClN₇O₄S (M⁺) 477.0986, found 477.0994.

### Example 9

### 1,3-Dimethyl-1H-pyrazole-4-sulfonic acid [4-(1,3-dimethyl-2,6-dioxo-2,3,6,7-tetrahydro-1H-purin-8-ylmethyl)-phenyl]-amide

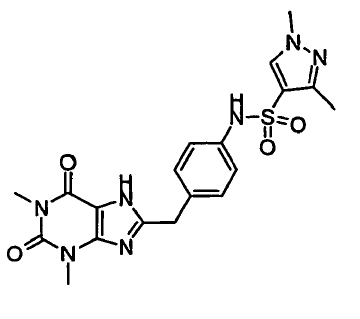

5-Chloro-1,3-dimethyl-1H-pyrazole-4-sulfonic acid {4-[1,3-dimethyl-2,6-dioxo-2,3,6,7-tetrahydro-1H-purin-8-ylmethyl]-phenyl}-amide (0.082 mmol) was dissolved in methanol (70 mL) and dichloromethane (10 mL) and the reaction vessel flushed with argon before adding 10% palladium on carbon (100 mg). The reaction vessel was charged with hydrogen gas at 50 psi pressure and shaken for 24 hrs. Additional 10% palladium on carbon (50 mg) was added and hydrogenolysis continued at 50 psi pressure of hydrogen gas for an additional 24 hrs. At this time no starting maerial remained. The reaction mixture was filtered through celite and concentrated in vacuo. The crude product was purified by chromatography using silica eluted with 10% v/v methanol in chloroform to give after concentration in vacuo 1,3-dimethyl-1H-pyrazole-4-sulfonic acid [4-(1,3-dimethyl-2,6-dioxo-2,3,6,7-tetrahydro-1H-purin-8-ylmethyl)-phenyl]-amide as a colorless solid (38%); EI-HRMS m/e calculated for C₁₉H₂₁N₇O₄S (M+) 443.1376, found 443.1364.

### Example 10

### N-(5-{4-[3-Cyclopropylmethyl-1-(2-fluoro-benzyl)-2,6-dioxo-2,3,6,7-tetrahydro-1H-purin-8-ylmethyl] -phenylsulfamoyl}-4-methyl-thiazol-2-yl)-acetamide

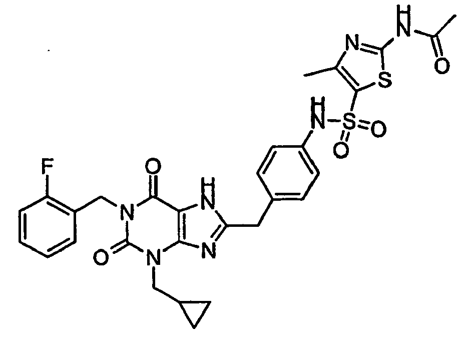

### Step 1: Preparation of 2-[4-(2-acetylamino-4-inethyl-thiazole-5-sulfonylamino)-phenyl]-N-[6-amino-1-cydopropylmethyl-3-(2-fluoro-benzyl)-2,4-dioxo-1,2,3,4-tetrahydro-pyrimidin-5-yl] -acetamide.

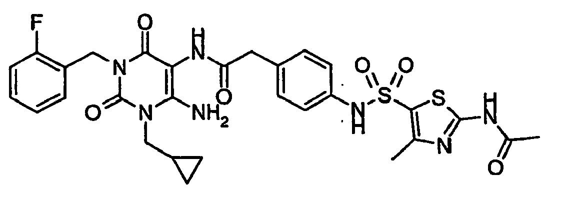

A solution of *N*-[6-amino-1-cyclopropylmethyl-3-(2-fluoro-benzyl)-2,4-dioxo-1,2,3,4-tetrahydro-pyrimidin-5-yl]-2-(4-amino-phenyl)-acetamide (prepared as in Example 1, 75 mg, 0.16 mmol) in pyridine (1.6 mL) was stirred at 25 °C for 5 min. This solution was then treated with 2-acetylamino-4-methyl-thiazole-5-sulfonyl chloride (44 mg, 0.17 mmol). The reaction mixture was stirred at 25 °C for 24 h. The reaction mixture was then poured into a mixture of methylene chloride (50 mL) and a 1.0N aqueous hydrochloric acid solution (10 mL). This mixture was extracted with a 1/9 methanol/methylene chloride solution (1 x 50 mL). The organics were dried over magnesium sulfate, filtered, and concentrated in vacuo. Flash chromatography (Merck Silica gel 60, 230-400 mesh, 7/93 methanol/methylene chloride) afforded 2-[4-(2-acetylamino-4-methyl-thiazole-5-sulfonylamino)-phenyl]-*N*-[6-amino-1-cyclopropylmethyl-3-(2-fluoro-benzyl)-2,4-dioxo-1,2,3,4-tetrahydro-pyrimidin-5-yl]-acetamide (56.4 mg, 54%) as an off-white solid; FAB-HRMS m/e calculated for C₂₉H₃₀FN₇O₆S₂ (M⁺) 655.1683, found 655.1679

### Step 2: Preparation of N-(5-{4-[3-cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide.

A solution of 2-[4-(2-acetylamino-4-methyl-thiazole-5-sulfonylamino)-phenyl]-*N-*[6-amino-1-cyclopropylmethyl-3-(2-fluoro-benzyl)-2,4-dioxo-1,2,3,4-tetrahydro-pyrimidin-5-yl]-acetamide (56 mg, 0.085 mmol) in methanol warmed to 50 °C was treated with a 10% aqueous sodium hydroxide solution (0.34 mL, 0.085 mmol). The reaction was stirred at 50 °C for 6 h. At this time, the reaction was concentrated in vacuo, diluted with water, and acidified to pH = 2 with a 1.0 N aqueous hydrochloric acid solution (0.9 mL). This mixture was cooled in an ice-bath. The resulting solids were collected by filtration, washed with water, and dried in vacuo. HPLC (20-80% acetonitrile/water) afforded *N*-(5-{4-[3-cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide (16.1 mg, 29.7%) as a white solid; FAB-HRMS m/e calculated for C₂₉H₂₈FN₇O₅S₂ (M+H)⁺ 638.1656, found 638.1641; and 2-amino-4-methyl-thiazole-5-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt (5.4 mg, 10%) as a white solid; FAB-HRMS m/e calculated for C₂₇H₂₆FN₇O₄S₂ (M+H)+ 596.1550, found 596.1547.

The compounds cited in examples 11 to 18 were obtained in an anologous manner to that described in example 10.

### Example 11

### Amino-4-methyl-thiazole-5-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt

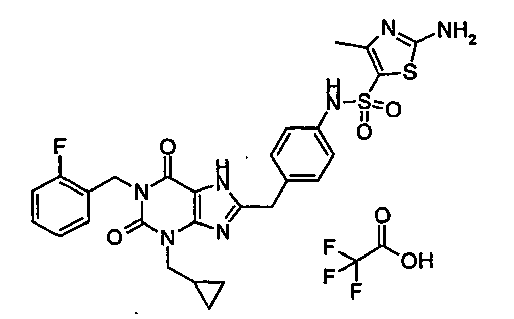

Prepared from 2-[4-(2-acetylamino-4-methyl-thiazole-5-sulfonylamino)-phenyl]-*N*-[6-amino-1-cyclopropylmethyl-3-(2-fluoro-benzyl)-2,4-dioxo-1,2,3,4-tetrahydro-pyrimidin-5-yl]-acetamide. White solid; FAB-HRMS m/e calculated for C₂₇H₂₆FN₇O₄S₂ (M+H)⁺ 596.1550, found 596.1547.

### Example 12

### 3,5-Dimethyl-isoxazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide

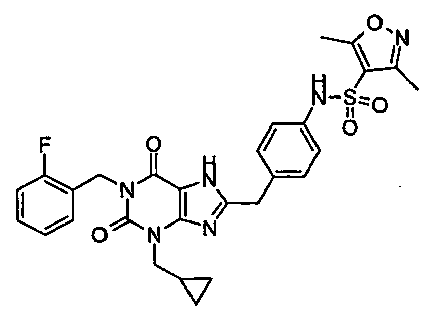

Prepared from *N*-[6-Amino-1-cyclopropylmethyl-3-(2-fluoro-benzyl)-2,4-dioxo-1,2,3,4-tetrahydro-pyrimidin-5-yl]-2-[4-(3,5-dimethyl-isoxazole-4-sulfonylamino)-phenyl]-acetamide. White solid; FAB-HRMS m/e calculated for C₂₈H₂₇FN₆O₅S (M+H)⁺ 579.1826, found 579.1822.

### Example 13

### 1,2-Dimethyl-1H-imidazole-4-sulfonic acid {4-[3-cyclopropylmethyl-1-(2-fluorobenzyl)-2,6-dioxo-2,3,6,7-tetrahydro-1H-purin-8-ylmethyl]-phenyl}-amide

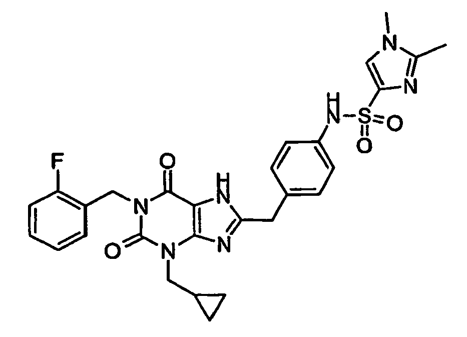

Prepared from *N*-[6-Amino-1-cyclopropylmethyl-3-(2-fluoro-benzyl)-2,4-dioxo-1,2,3,4-tetrahydro-pyrimidin-5-yl]-2-[4-(1,2-dimethyl-1H-imidazole-4-sulfonylamino)-phenyl]-acetamide. White solid; FAB-HRMS m/e calculated for C₂₈H₂₈FN₇O₄S (M+H)⁺ 578.1986, found 578.1976.

### Example 14

### Methyl-1H-imidazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide

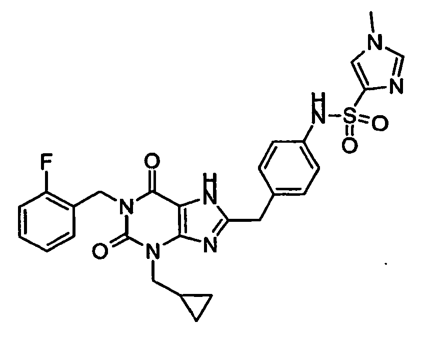

Prepared from *N*-[6-Amino-1-cyclopropylmethyl-3-(2-fluoro-benzyl)-2,4-dioxo-1,2,3,4-tetrahydro-pyrimidin-5-yl]-2-[4-(1-methyl-1H-imidazole-4-sulfonylamino)-phenyl]-acetamide. White solid; LRMS for C₂₇H₂₆FN₇O₄S (M+H)⁺ at m/z = 564.

### Example 15

### Methyl-1H-imidazole-4-sulfonic acid {4-[3-butyl-1-(2-fluoro-benzyl)-2,6-dioxo-2,3,6,7-tetrahydro-1H-purin-8-ylmethyl]-phenyl}-amide hydrochloride salt

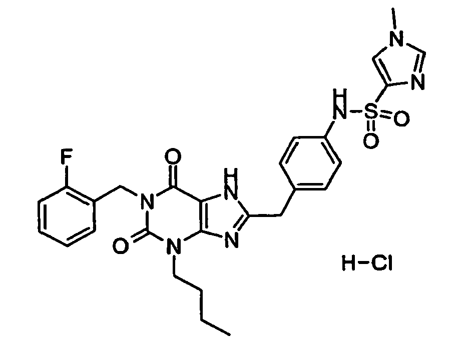

Prepared from *N*-[6-Amino-1-butyl-3-(2-fluoro-benzyl)-2,4-dioxo-1,2,3,4-tetrahydro-pyrimidin-5-yl] -2-[4-(1-methyl-1H-imidazole-4-sulfonylamino)-phenyl]-acetamide. White solid; FAB-HRMS m/e calculated for C₂₇H₂₈FN₇O₄S (M+H)+ 566.1986, found 566.1971.

### Example 16

### Methyl-1H-imidazole-4-sulfonic acid {4-[3-butyl-1-(2-fluoro-benzyl)-2,6-dioxo-2,3,6,7-tetrahydro-1H-purin-8-ylmethyl]-phenyl}-amide trifluoroacetic acid salt

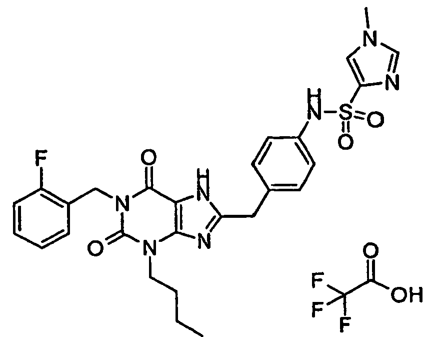

Prepared from *N*-[6-Amino-1-butyl-3-(2-fluoro-benzyl)-2,4-dioxo-1,2,3,4-tetrahydro-pyrimidin-5-yl]-2-[4-(1-methyl-1H-imidazole-4-sulfonylamino)-phenyl]-acetamide. White solid; LRMS for C₂₇H₂₈FN₇O₄S (M+H)+ at m/z = 566.

### Example 17

### N-{4-[3-Cyclopropylmethyl-1-(2-fluoro-benzyl)-2,6-dioxo-2,3,6,7-tetrahydro-1H-purin-8-ylmethyl]-phenyl}-N,N-dimethylsulfamide

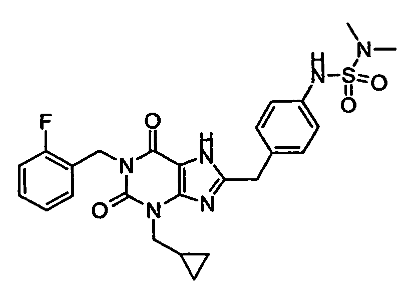

Prepared from *N*-[6-Amino-1-cyclopropylmethyl-3-(2-fluoro-benzyl)-2,4-dioxo-1,2,3,4-tetrahydro-pyrimidin-5-yl]-2-[4-(3,5-dimethyl-isoxazole-4-sulfonylamino)-phenyl]-acetamide. Off-white solid; FAB-HRMS m/e calculated for C₂₅H₂₇FN₆O₄S (M+H)⁺ 527.1877, found 527.1858.

### Example 18

### 5-Chloro-1,3-dimethyl-1H-pyrazole-4-sulfonic acid {4- [3-cyclopropylmethyl-1- (2-fluoro-benzyl)-2,6-dioxo-2,3,6,7-tetrahydro-1H-purin-8-ylmethyl] -phenyl}-methylamide

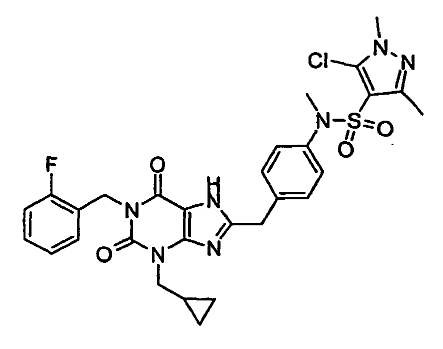

### Step 1: Preparation of [4-(tert-butoxycarbonyl-methyl-amino)-phenyl]-acetic acid.

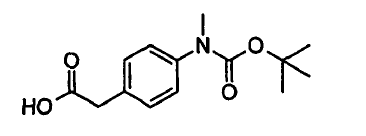

To a solution of [4-(tert-butoxycarbonyl-methyl-amino)-phenyl]-acetic acid methyl ester (prepared according to the procedure of Hay et al. as described in J. Chem. Soc. Perkin Trans. 11999, 19, 2759) (2.68 mmol) in methanol (10 mL) was added 1.0M aqueous lithium hydroxide solution (13.7 mL) and the mixture heated to 50°C for 35 mins. The reaction mixture was cooled in an ice bath then washed with ether (25 mL), acidified with 3.0 M aqueous hydrochloric acid to pH = 4 while stirring and cooling in an ice bath. The precipitate was isolated by filtration, washed with water and dried in vacuo to give [4-(tert-butoxycarbonyl-methyl-amino)-phenyl]-acetic acid as a colorless solid (70%).

### Step 2: Preparation of (4-{[6-amino-3-(2-fluoro-benzyl)-2,4-dioxo-1,2,3,4-tetrahydro-pyrimidin-5-ylcarbamoyl]-methyl}-phenyl)-methyl-carbamic acid tert-butyl ester.

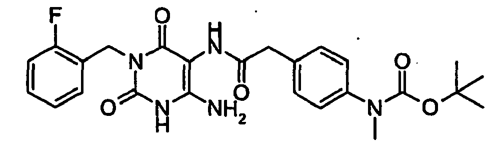

Prepared by the same procedure as described for the preparation of (4-{ [6-amino-3-(2-fluoro-benzyl)-2,4-dioxo-1,2,3,4-tetrahydro-pyrimidin-5-ylcarbamoyl] -methyl}-phenyl)-carbamic acid tert-butyl ester (example 1, step 4) except that [4-(tert-butoxycarbonyl-methyl-amino)-phenyl]-acetic acid was used in place of (4-tert-butoxycarbonylamino-phenyl)-acetic acid. Crude (4-{[6-amino-3-(2-fluoro-benzyl)-2,4-dioxo-1,2,3,4-tetrahydro-pyrimidin-5-ylcarbamoyl] -methyl}-phenyl)-methyl-carbamic acid tert-butyl ester was obtained as a yellow solid which was of sufficient purity for subsequent used without additional purification.

### Step 3: Preparation of (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-methyl-carbamic acid tert-butyl ester.

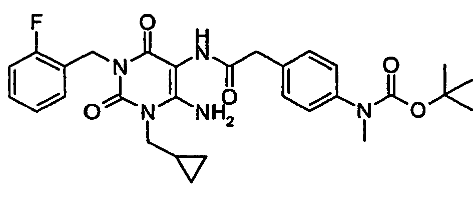

Prepared by the same procedure as described for the preparation of (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-carbamic acid tert-butyl ester (example 1, step 5) except that (4-{[6-amino-3-(2-fluoro-benzyl)-2,4-dioxo-1,2,3,4-tetrahydro-pyrimidin-5-ylcarbamoyl]-methyl}-phenyl)-methyl-carbamic acid tert-butyl ester was used in place of (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-carbamic acid tert-butyl ester. Purified by chromatography using silica eluted with 3% v/v methanol in chloroform to give (4-{[6-amino-1-cyclopropylmethyl-3-(2-fluoro-benzyl)-2,4-dioxo-1,2, 3,4-tetrahydro-pyrimidin-5-ylcarbamoyl]-methyl}-phenyl)-methyl-carbamic acid tert-butyl ester as a colorless solid foam (63% yield from [4-(tert-butoxycarbonyl-methyl-amino)-phenyl]-acetic acid).

### Step 4: Preparation of N-[6-amino-1-cyclopropylmethyl-3-(2-fluoro-benzyl)-2,4-dioxo-1,2,3,4-tetrahydro-pyrimidin-5-yl] -2-(4-methylamino-phenyl)-acetamide, hydrochloride.

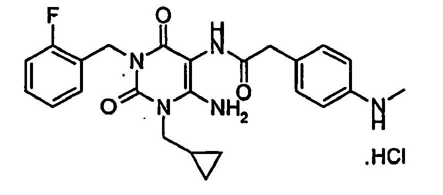

Prepared by the same method as described for the preparation of *N*-[6-amino-1-cyclopropylmethyl-3-(2-fluoro-benzyl)-2,4-dioxo-1,2,3,4-tetrahydro-pyrimidin-5-yl]-2-(4-amino-phenyl)-acetamide, hydrochloride salt (example 1, step 6) except that (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-methyl-carbamic acid tert-butyl ester was used in place of (4-{[6-amino-1-cyclopropylmethyl-3-(2-fluoro-benzyl)-2,4-dioxo-1,2,3,4-tetrahydro-pyrimidin-5-ylcarbamoyl]-methyl}-phenyl)-carbamic acid tert-butyl ester. *N*-[6-Amino-1-cyclopropylmethyl-3-(2-fluoro-benzyl)-2,4-dioxo-1,2,3,4-tetrahydro-pyrimidin-5-yl]-2-(4-methylamino-phenyl)-acetamide; hydrochloride was obtained as a green solid which was of sufficient purity for subsequent used without additional purification.

### Step 5: Preparation of N-[6-amino-1-cyclopropylmethyl-3-(2-fluoro-benzyl)-2,4-dioxo-1,2,3,4-tetrahydro-pyrimidin-5-yl]-2-{4-[(5-chloro-1,3-dimethyl-1H-pyrazole-4-sulfonyl)-methyl-amino] -phenyl}-acetamide.

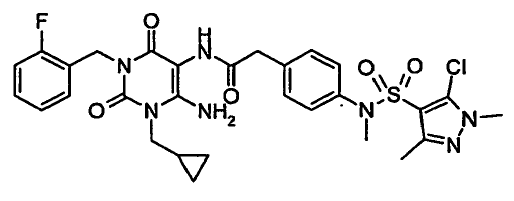

To a solution of *N*-[6-amino-1-cyclopropylmethyl-3-(2-fluoro-benzyl)-2,4-dioxo-1,2,3,4-tetrahydro-pyrimidin-5-yl]-2-(4-methylamino-phenyl)-acetamide; hydrochloride (0.246 mmol) in pyridine (2.5 mL) was added 5-chloro-1,3-dimethyl-pyrazole-4-sulfonyl chloride (0.295 mmol) and the mixture stirred at ambient temperature for 48 hrs. The reaction mixture was poured into dichloromethane and washed twice with 1.0M aqueous hydrochloric acid, washed with brine, dried with magnesium sulfate, filtered and concentrated in vacuo. Purified by chromatography using silica eluted with 3% v/v methanol in dichloromethane to give *N*-[6-amino-1-cyclopropylmethyl-3-(2-fluorobenzyl)-2,4-dioxo-1,2,3,4-tetrahydro-pyrimidin-5-yl]-2-{4-[(5-chloro-1,3-dimethyl-1H-pyrazole-4-sulfonyl)-methyl-amino]-phenyl}-acetamide as an off white solid (53%).

### Step 6: Preparation of 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl] -phenyl}-methyl-amide.

To a solution of *N*-[6-amino-1-cyclopropylinethyl-3-(2-fluoro-benzyl)-2,4-dioxo-1,2,3,4-tetrahydro-pyrimidin-5-yl]-2-{4- [(5-chloro-1,3-dimethyl-1H-pyrazole-4-sulfonyl)-methyl-amino]-phenyl}-acetamide (0.119 mmol) in methanol (2 mL) was added 10% w/v aqueous sodium hydroxide solution (1 mL) and the mixture heated to 50 °C for 1.5 hrs. The methanol was removed in vacuo and the aqueous residue acidified to pH = 4 with 1.0M aqueous hydrochloric acid. The aqueous suspension was extracted with chloroform, the combined chloroform extracts washed with brine, dried with magnesium sulfate, filtered and concentrated in vacuo. the residue was triturated with acetonitrile to give 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide as a colorless solid (50%); (ES)+-HRMS m/e calculated for C₂₉H₂₉ClFN₇O₄S (M+Na)⁺ 648.1566, found 648.1566.

### Example 19

### Quinoline-8-sulfonic acid {4-[3-cyclopropylmethyl-1-(2-fluoro-benzyl)-2,6-dioxo-2,3,6,7-tetrahydro-1H-purin-8-ylmethyl]-phenyl}-amide

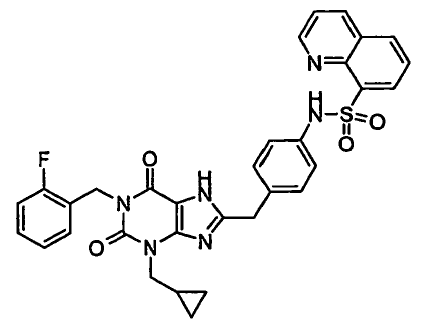

This compound was prepared by the routes shown in schemes 2 and 4.

8-(4-Amino-benzyl)-3-cyclopropylmethyl-1-(2-fluoro-benzyl)-3,7-dihydro-purine-2,6-dione was condensed with 8-quinolinesulfonyl chloride under standard conditions to give the stated sulfonamide. Recrystallization from acetonitrile gave the pure product as an off white solid (53%); ¹H NMR(DMSO-d6, 300 MHz) δH13.45 (s, 1H), 10.12 (s, 1H), 9.21 (dd, J = 4.2,1.1 Hz, 1H), 8.59 (dd, J = 8.4,1.8 Hz, 1H), 8.41 (dd, J = 7.3, 1.5 Hz, 1H), 8.33 (dd, J = 7.7, 1.5 Hz, 1H), 7.82-7.73 (m, 2H), 7.45-7.00 (m, 8H), 5.16 (s, 2H), 3.93 (s, 2H), 3.86 (d, J = 7.3 Hz, 2H), 1.30-1.10 (m, 1H) and 0.50-0.30 (m, 4H); EI-HRMS m/e calculated for C₃₂H₂₇FN₆O₄S (M+) 610.1798, found 610.1804.

### Example 20

### N-(4-{4-[3-Cyclopropylmethyl-1-(2-fluoro-benzyl)-2,6-dioxo-2,3,6,7-tetrahydro-1H-purin-8-ylmethyl]-phenylsulfamoyl}-phenyl)-acetamide

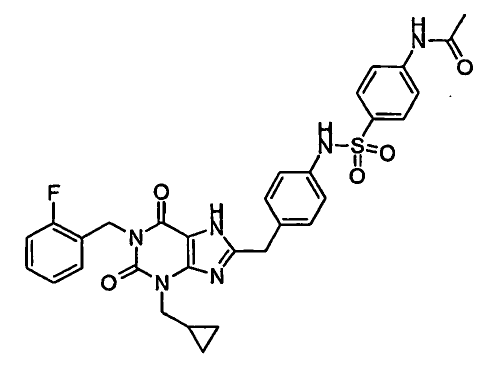

8-(4-Amino-benzyl)-3-cyclopropylmethyl-1-(2-fluoro-benzyl)-3,7-dihydro-purine-2,6-dione was condensed with 4-acetamidobenzenesulfonyl chloride under the conditions previously described to give the stated sulfonamide. Recrystallized from acetonitrile to give the pure product as an off-white solid (68%); ¹H NMR (DMSO-d₆, 300 MHz) δ 13.53 (s, 1H), 10.36 (s, 1H), 10.23 (s, 1H), 7.75 (s, 4H), 7.40-7.05 (m, 8H), 5.19 (s, 2H), 4.02 (s, 2H), 3.91 (d, J=7.0 Hz, 2H), 2.13 (s, 3H), 1.35-1.15 (m, 1H) and 0.55-0.35 (m, 4H); EI-HRMS m/e calculated for C₃₁H₂₉FN₆O₅S (M⁺) 616.1904, found 616.1922.

### Example 21

### N-[4-(1-Benzyl-3-butyl-2,6-dioxo-2,3,6,7-tetrahydro-1H-purin-8-ylmethyl)-phenyl] -4-methyl-benzenesulfonamide

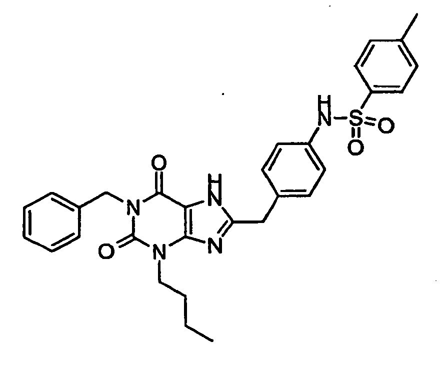

8-(4-Amino-benzyl)-3-cyclopropylmethyl-1-(2-fluoro-benzyl)-3,7-dihydro-purine-2,6-dione was condensed with 4-toluenesulfonyl chloride under the conditions previously described to give the stated sulfonamide as an off white solid (59%); ¹H NMR (DMSO-d₆, 300 MHz) δ 13.49 (s, 1H), 10.28 (s, 1H), 7.70 (d, J = 8.1 Hz, 2H), 7.45-7.25 (m, 7H), 7.21 (d, J = 8.4 Hz, 2H), 7.09 (d, J = 8.4 Hz, 2H), 5.12 (s, 2H), 4.02 (s, 2H), 4.01 (t, J = 7.0 Hz, 2H), 2.39 (s, 3H), 1.75-1.60 (m, 2H), 1.40-1.25 (m, 2H), 0.94 (t, J = 7.5 Hz, 3H); EI-HRMS m/e calculated for C₃₀H₃₁N₅O₄S (M⁺) 557.2097, found 557.2090.

### Example 22

### Pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt

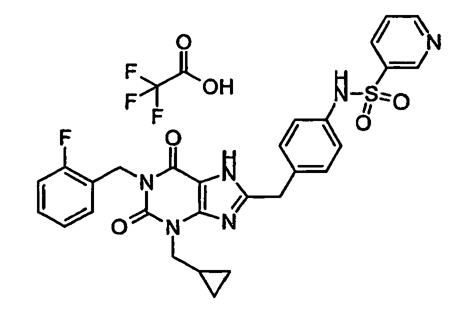

A solution of 5-bromo-6-chloro-pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (prepared as in Example 5, 37.5 mg, 0.056 mmol) in ethanol (50 mL) at 25 °C was treated with 10% Pd/C (4.0 mg). The reaction mixture was submitted to hydrogenation conditions in a Parr bomb at 48 psi for 1 h. At this time, the reaction mixture was filtered through a pad of celite and washed with ethanol followed by a warm solution of 90/10 methylene chloride/ethanol. The filtrated was concentrated in vacuo. The residue was resubmitted to the reaction conditions with triethylamine (0.015 mL, 0.11 mmol) and was hydrogenated for 4 d. At this time, the reaction was filtered through a pad of celite and washed with solvent. The filtrate was concentrated in vacuo. The resulting residue was purified by HPLC (20-70% acetonitrile/water) to afford pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt (5.2 mg, 13.8%); LRMS for C₂₈H₂₅FN₆O₄S (M+H)⁺ at m/z = 561.

### Example 23

### N-{4-[3-Cyclopropylmethyl-1-(2-fluoro-benzyl)-2,6-dioxo-2,3,6,7-tetrahydro-1H-purin-8-ylmethyl] -phenyl}-methanesulfonamide

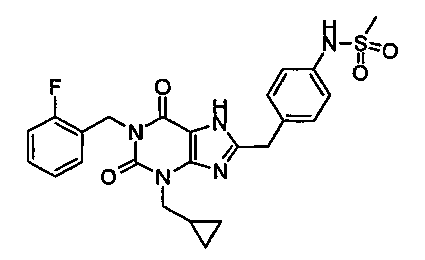

8-(4-Amino-benzyl)-3-cyclopropylmethyl-1-(2-fluoro-benzyl)-3,7-dihydro-purine-2,6-dione was condensed with methanesulfonyl chloride under the conditions previously described to give the stated sulfonamide as an off-white solid (28%); ¹H NMR(DMSO-d₆, 300 MHz) δ 13.42 (s,1H), 9.61 (s,1H), 7.30-6.90 (m, 8H), 5.04 (s, 2H), 3.94 (s, 2H), 3.77 (d, J = 6.6 Hz, 2H), 2.87 (s, 3H), 1.25-1.05 (m, 1H), 0.40-0.25 (m, 4H); EI-HRMS m/e calculated for C₂₄H₂₄FN₅O₄S (M⁺) 497.1533, found 497.1528.

### Example 24

### N-[4-(1-Benzyl-3-butyl-2,6-dioxo-2,3,6,7-tetrahydro-1H-purin-8-ylmethyl)-phenyl]-methanesulfonamide

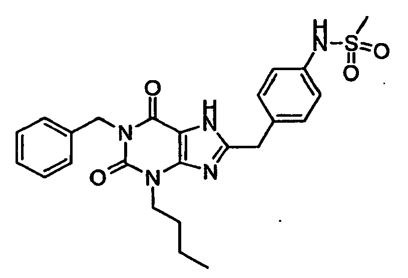

8-(4-Amino-benzyl)-1-benzyl-3-butyl-3,7-dihydro-purine-2,6-dione was condensed with methanesulfonyl chloride under the conditions previously described to give the stated sulfonamide as a pale brown solid (35%); ¹H NMR(DMSO-d₆, 300 MHz) δ 13.54 (s, 1H), 9.76 (s, 1H), 7.45-7.25 (m, 7H), 7.22 (d, J = 8.4 Hz, 2H), 5.14 (s, 2H), 4.10 (s, 2H), 4.04 (t, J = 7.1 Hz, 2H), 3.03 (s, 3H), 1.80-1.65 (m, 2H), 1.40-1.25 (m, 2H), 0.96 (t, J=7.3 Hz, 3H); EI-HRMS m/e calculated for C₂₄H₂₇N₅O₄S (M+) 481.1784, found 481.1781.

### Example 25

### 5-Chloro-1,3-dimethyl-1H-pyrazole-4-sulfonic acid {6-[3-butyl-1-(2-fluoro-benzyl)-2,6-dioxo-2,3,6,7-tetrahydro-1H-purin-8-ylmethyl]-pyridin-3-yl}-amide

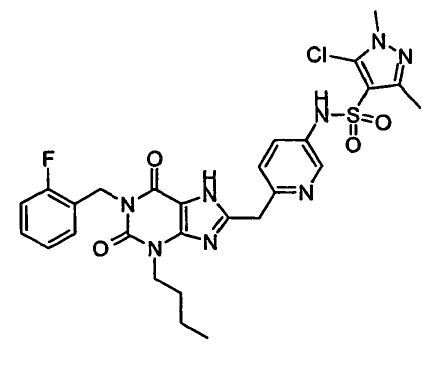

This compound was prepared by the route outlined in schemes 1 and 7.

### Step 1: Preparation of 2-(5-nitro-pyridin-2-yl)-malonic acid dibenzyl ester.

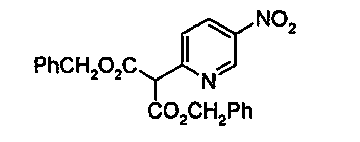

A solution of potassium tert-butoxide in dimethylsulfoxide was treated with dibenzyl malonate. This mixture was stirred at 95 °C for 10 min. At this time, the reaction was treated with a solution of 2-chloro-5-nitropyridine. The resulting mixture was stirred at 95 °C for 1.5 h. At this time, the reaction mixture was poured into ice/water and extracted with ethyl acetate. The organics were washed with a saturated aqueous sodium chloride solution, dried over sodium sulfate, filtered, and concentrated in vacuo. Flash chromatography (Merck Silica gel 60, 230-400 mesh, 20/80 ethyl acetate/hexanes) afforded 2-(5-nitro-pyridin-2-yl)-malonic acid dibenzyl ester (45%) as a bright yellow solid; LRMS for C₂₂H₁₈N₂O₆ (M-H)⁺ at m/z = 405.

### Step 2: Preparation of 2-(5-amino-pyridin-2-yl)-malonic acid dibenzyl ester.

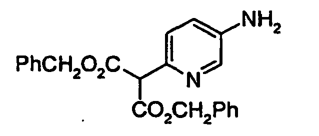

A solution of 2-(5-nitro-pyridin-2-yl)-malonic acid dibenzyl ester in a mixture of methanol, tetrahydrofuran, and water was treated with ammonium chloride (25 eq.) and zinc dust (>10 micron,10 eq.). The mixture was stirred at 25 °C for 40 min. At this time, the reaction was filtered through celite. The filtrate was concentrated in vacuo. The aqueous residue was extracted with ethyl acetate. The organics were washed with a saturated aqueous sodium chloride solution, dried over sodium sulfate, filtered, and concentrated in vacuo to afford 2-(5-amino-pyridin-2-yl)-malonic acid dibenzyl ester as a yellow viscous oil (89%); LRMS for C₂₂H₂₀N₂O₄ (M+H)⁺ at m/z = 377.

### Step 3: Preparation of 2-[5-(5-chloro-1,3-dimethyl-1H-pyrazole-4-sulfonylamino)-pyridin-2-yl]-malonic acid dibenzyl ester

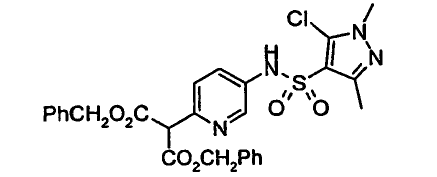

A solution of 2-(5-amino-pyridin-2-yl)-malonic acid dibenzyl ester in pyridine cooled to 0 °C was treated with 5-chloro-1,3-dimethyl-1H-pyrazole-4-sulfonyl chloride. The reaction mixture was allowed to slowly warm to 25 °C and was stirred at 25 °C for 24 h. At this time, the reaction mixture was cooled to 0 °C and acidified to pH = 1 with 3.0 M aqueous hydrochloric acid solution. The resulting mixture was extracted with ethyl acetate. The organics were washed with a 1.0 M aqueous hydrochloric acid solution, water, and a saturated aqueous sodium chloride solution, dried over sodium sulfate, filtered, and concentrated in vacuo. Flash chromatography (Merck Silica gel 60, 230-400 mesh, 65/35 ethyl acetate/hexanes) afforded 2-[5-(5-chloro-1,3-dimethyl-1H-pyrazole-4-sulfonylamino)-pyridin-2-yl]-malonic acid dibenzyl ester (55%) as a colorless solid; LRMS for C₂₇H₂₅ClN₄O₆S (M+H)⁺ at m/z = 569/571.

### Step 4: Preparation of [5-(5-chloro-1,3-dimethyl-1H-pyrazole-4-sulfonylamino)-pyridin-2-yl]-acetic acid

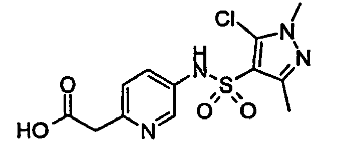

A solution of 2-[5-(5-chloro-1,3-dimethyl-1H-pyrazole-4-sulfonylamino)-pyridin-2-yl]-malonic acid dibenzyl ester in ethyl acetate was treated with 10% palladium on carbon. The mixture was hydrogenolyzed at 25 °C under hydrogen at 1 atmosphere until no starting material remained. At this time, the reaction mixture was filtered through celite. The filtrate was concentrated in vacuo to afford [5-(5-chloro-1,3-dimethyl-1H-pyrazole-4-sulfonylamino)-pyridin-2-yl]-acetic acid (94%) as a colorless solid; LRMS for C₁₂H₁₃ClN₄O₄S (M+H)⁺ at m/z = 345/347.

### Step 5: Preparation of N-[6-amino-1-butyl-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide.

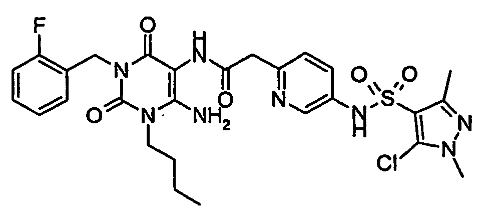

Prepared as for 2-[4-(2-acetylamino-4-methyl-thiazole-5-sulfonylamino)-phenyl]-*N*-[6-amino-1-cyclopropylmethyl-3-(2-fluoro-benzyl)-2,4-dioxo-1,2,3,4-tetrahydro-pyrimidin-5-yl]-acetamide (example 10, step 1) except using [5-(5-chloro-1,3-dimethyl-1H-pyrazole-4-sulfonylamino)-pyridin-2-yl]-acetic acid and 5,6-diamino-1-butyl-3-(2-fluoro-benzyl)-1H-pyrimidine-2,4-dione. *N*-[6-amino-1-butyl-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide was used without purification (91%); LRMS for C₂₇H₃₀ClFN₈O₅S (M+H)⁺ at m/z = 633/635.

### Step 6: Preparation of 5-chloro-1,3-dimethyl-1H-pyrazole-4-sulfonic acid {6-[3-butyl-1- . (2-fluoro-benzyl)-2,6-dioxo-2,3,6,7-tetrahydro-1H-purin-8-ylmethyl] -pyridin-3-yl}-amide.

Prepared as for *N*-(5-{4-[3-cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide (example 10, step 2) except using the 5-chloro-1,3-dimethyl-1H-pyrazole-4-sulfonic acid 6-pyridin-3-yl-amide analog. Flash chromatography (Merck Silica gel 60, 230-400 mesh, 5/95 methanol/methylene chloride) afforded 5-chloro-1,3-dimethyl-1H-pyrazole-4-sulfonic acid {6-[3-butyl-1-(2-fluoro-benzyl)-2,6-dioxo-2,3,6,7-tetrahydro-1H-purin-8-ylmethyl]-pyridin-3-yl}-amide (49%) as an off-white solid; FAB-HRMS m/e calculated for C₂₇H₂₈ClFN₈O₄S (M+H)⁺ 615.1705, found 615.1691.

### Example 26

### 5-Chloro-1,3-dimethyl-1H-pyrazole-4-sulfonic acid {6-[3-cyclobutylmethyl-1-(2-fluoro-benzyl)-2,6-dioxo-2,3,6,7-tetrahydro-1H-purin-8-ylmethyl]-pyridin-3-yl}-amide

This compound was prepared by the route shown in scheme 1.

### Step 1: Preparation of N-[6-amino-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide

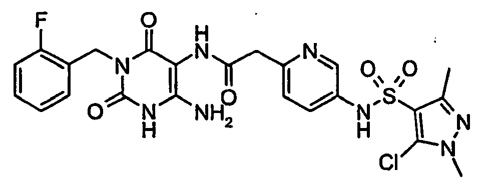

Prepared as for 2-[4-(2-acetylamino-4-methyl-thiazole-5-sulfonylamino)-phenyl]-*N*- [6-amino-1-cyclopropylmethyl-3-(2-fluoro-benzyl)-2,4-dioxo-1,2,3,4-tetrahydro-pyrimidin-5-yl]-acetamide (example 10, step 1) except that the coupling was performed using 1[3-(dimethylamino)propyl]-3-ethylcarbodiimide hydrochloride as the coupling reagent and [5-(5-chloro-1,3-dimethyl-1H-pyrazole-4-sulfonylamino)-pyridin-2-yl]-acetic acid and 5,6-diamino-3-(2-fluoro-benzyl)-1H-pyrimidine-2,4-dione were used as the coupling partners. Flash chromatography (Merck Silica gel 60, 230-400 mesh, 10/90 methanol/chloroform) afforded *N*-[6-amino-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide (42%) as an off white solid; LRMS for C₂₉H₂₉ClFN₇O₄S (M+H)+ at m/z = 577/579.

### Step 2: Preparation of N-[6-amino-1-cyclobutylmethyl-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide.

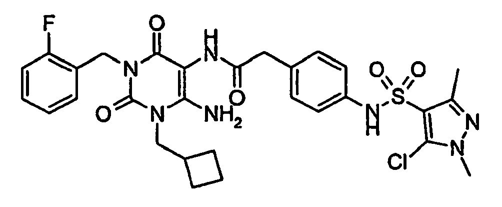

A solution of *N*-[6-amino-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide in N,N,-dimethylformamide was treated with potassium carbonate (4.0 eq.) and (bromomethyl)cyclobutane (2.0 eq.). The reaction mixture was heated to 40 °C for 16 h. At this time, the N,N,-dimethylformamide was removed in vacuo. The residue was triturated with chloroform and then methanol to remove impurities. Flash chromatography (Merck Silica gel 60, 230-400 mesh, 10/90 methanol/chloroform) afforded *N*-[6-amino-1-cyclobutylmethyl-3-(2-fluoro-benzyl)-2,4-dioxo-1,2,3,4-tetrahydro-pyrimidin-5-yl]-2-[5-(5-chloro-1,3-dimethyl-1H-pyrazole-4-sulfonylamino)-pyridin-2-yl]-acetamide (15%) as a light brown solid; LRMS for C₂₉H₃₁ClFN₇O₅S (M+H)⁺ at m/z = 645.

### Step 3: Preparation of 5-chloro-1,3-dimethyl-1H-pyrazole-4-sulfonic acid {6- [3-cyclobutylmethyl-1-(2-fluoro-benzyl)-2,6-dioxo-2,3,6,7-tetrahydro-1H-purin-8-ylmethyl]-pyridin-3-yl}-amide.

Prepared as for *N*-(5-{4-[3-cyclopropylmethyl-1-(2-fluoro-benzyl)-2,6-dioxo-2,3,6,7-tetrahydro-1H-purin-8-ylmethyl] -phenylsulfamoyl}-4-methyl-thiazol-2-yl)-acetamide (example 10, step 2) except using the 5-chloro-1,3-dimethyl-1H-pyrazole-4-sulfonic acid 6-pyridin-3-yl-amide analog. Flash chromatography (Merck Silica gel 60, 230-400 mesh, 10/90 methanol/chloroform) afforded 5-chloro-1,3-dimethyl-1H-pyrazole-4-sulfonic acid {6-[3-cyclobutylmethyl-1-(2-fluoro-benzyl)-2,6-dioxo-2,3,6,7-tetrahydro-1H-purin-8-ylmethyl]-pyridin-3-yl}-amide (62%) as an off-white solid; EI-HRMS m/e calculated for C₂₈H₂₈ClFN₈O₄S (M+H)⁺ 627.1700, found 627.1707.

### Example 27

### 1,3-Dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide

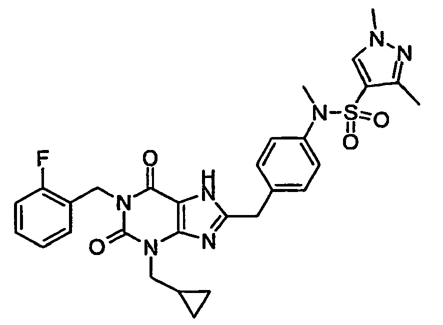

A solution of 5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide (prepared as described in Example 18, 20 mg, 0.032 mmol) in methanol (20 mL) at 25 °C was treated with 10% Pd/C (100 mg). The reaction mixture was submitted to hydrogenation conditions in a Parr bomb at 48 psi for 2 d. At this time, the reaction mixture was filtered through a pad of celite and washed with 90/10 methylene chloride/methanol solution (75 mL). The filtrated was concentrated in vacuo. The resulting residue was purified by HPLC (20-60% acetonitrile/water) to afford 1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide (7.0 mg, 37%) as white solid; (ES)⁺-HRMS m/e calculated for C₂₉H₃₀N₇O₄S (M+Na)⁺ 614.1956, found 614.1955.

### Example 28

### 4-[3-Butyl-1-(2-fluoro-benzyl)-2,6-dioxo-2,3,6,7-tetrahydro-1H-purin-8-ylmethyl]-N-pyridin-2-yl-benzenesulfonamide

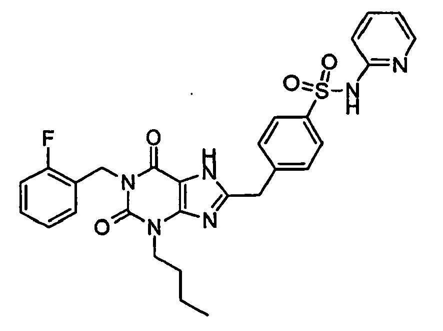

This compound was prepared according to the general procedures outlined in schemes 2 and 6.

### Step 1: Preparation of [4-(pyridin-2-ylsulfamoyl)-phenyl] -acetic acid ethyl ester.

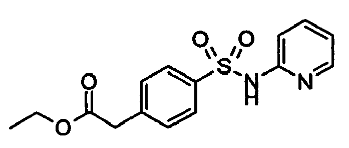

A stirred solution of 2-aminopyridine (282 mg, 3.0 mmol) in dry pyridine (5.0 mL) was treated with (4-chlorosulfonyl-phenyl)-acetic acid ethyl ester (prepared according to the procedure of Kawashima et. al, as described in Chem. Pharm. Bull. 1995, 43(7),1132) dropwise at 0 - 5 °C under argon. Upon completion of addition, the reaction was slowly warmed to 25 °C. The reaction was stirred at 25 °C until no starting materials remained. At this time, the reaction was cooled to 0 °C and was acidified to pH = 5 with a 1.0 M aqueous hydrochloric acid solution. The reaction mixture was poured into water and was extracted with ethyl acetate. The combined organic extracts were washed with water and a saturated aqueous sodium chloride solution, dried over magnesium sulfate, filtered, and concentrated in vacuo. Flash chromatography (Merck Silica gel 60, 230-400 mesh, 3/97 methanol/methylene chloride) afforded [4-(pyridin-2-ylsulfamoyl)-phenyl]-acetic acid ethyl ester (0.26 g, 27%) as a colorless solid; ¹H NMR (CDCl₃, 300 MHz) δ 13.67 (broad s, 1H), 8.30 (dd, J = 5.9,1.1 Hz, 1H), 7.86 (d, J = 8.4 Hz, 2H), 7.65 (tt, J = 8.1,1.7 Hz, 1H), 7.50-7.30 (m, 3H), 6.77 (t, J = 6.3 Hz, 1H), 4.12 (q, J = 7.0 Hz, 2H), 3.63 (s, 2H), 1.22 (t, J = 7.0 Hz, 3H); LRMS for C₁₅H₁₆N₂O₄S (M+H)⁺ at m/z = 321.

### Step 2: Preparation of [4-(pyridin-2-ylsulfamoyl)-phenyl]-acetic acid.

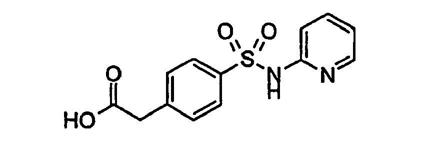

A solution of [4-(pyridin-2-ylsulfamoyl)-phenyl]-acetic acid ethyl ester (0.26 g, 0.813 mmol) in absolute ethanol (10 mL) was treated with a 1.0 M ethanolic potassium hydroxide solution (2.0 mL, 2.0 mmol). The reaction mixture was heated to reflux under argon for 3.5 h. The reaction mixture was then concentrated in vacuo. The residue was dissolved in water and filtered through celite. The filtrate was acidified to pH = 4.5 with a 1.0 M aqueous hydrochloric acid solution. The resulting precipitate was collected by filtration, washed with water, and dried in vacuo to afford [4-(pyridin-2-ylsulfamoyl)-phenyl]-acetic acid (155 mg, 65%) as an off white solid; ¹H NMR (DMSO-d₆, 300 MHz) δ 12.26 (broad s, 1H), 7.96 (d, J = 4.4 Hz, 1H), 7.77 (d, J = 7.7 Hz, 2H), 7.80 (t, J = 7.5 Hz, 1H), 7.37 (d, J = 7.7 Hz, 2H), 7.13 (d, J = 8.8 Hz, 1H), 6.82 (broad s, 1H), 3.62 (s, 2H); LRMS for C₁₃H₁₂N₂O₄S (M+H)⁺ at m/z = 293.

### Step 3: Preparation of N-[1-Butyl-3-(2-fluoro-benzyl)-2,4-dioxo-1,2,3,4-tetrahydro-pyrimidin-5-yl]-2-[4-(pyridin-2-ylsulfamoyl)-phenyl]-acetamide.

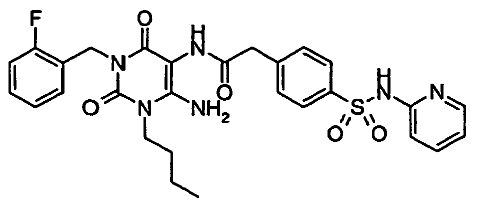

A solution of 5,6-diamino-1-butyl-3-(2-fluoro-benzyl)-1H-pyrimidine-2,4-dione (30.6 mg, 0.1 mmol) and [4-(pyridin-2-ylsulfamoyl)-phenyl]-acetic acid (29.2 mg, 0.1 mmol) in dry *N,N*-dimethylformamide (1 mL) was treated with *N,N*-diisopropylethylamine (61 mL, 0.35 mmol) and *O*-benzotriazol-1-yl-*N,N,N',N*'-tetramethyl-uronium tetrafluoroborate (37.9 mg, 0.1 mmol). The mixture was stirred at 25 °C for 16 h. The reaction mixture was diluted with water and acidified to pH=5.0 with a 0.01M aqueous hydrochloric acid solution. The reaction mixture was extracted with ethyl acetate. The combined organic extracts were washed with water and a saturated aqueous sodium chloride solution, dried over magnesium sulfate, filtered, and concentrated in vacuo to afford *N*-[1-butyl-3-(2-fluoro-benzyl)-2,4-dioxo-1,2,3,4-tetrahydro-pyrimidin-5-yl]-2-[4-(pyridin-2-ylsulfamoyl)-phenyl]-acetamide (86 %); ¹H NMR(CDCl₃, 300 MHz) δ 8.20-6.65 (m, 13H), 5.21 (broad s, 2H), 5.02 (s, 2H), 3.79 (t, J=7 Hz, 2H), 3.60 (s, 2H), 1.65-1.20 (m, 4H), 0.85 (t, J=7 Hz, 3H); LRMS for C₂₈H₂₉FN₆O₅S (M+H)⁺ at m/z = 581.

### Step 4: Preparation of 4-[3-butyl-1-(2-fluoro-benzyl)-2,6-dioxo-2,3,6,7-tetrahydro-1H-purin-8-ylmethyl]-N-pyridin-2-yl-benzenesulfonamide.

A solution of crude *N*-[6-amino-1-butyl-3-(2-fluoro-benzyl)-2,4-dioxo-1,2,3,4-tetrahydro-pyrimidin-5-yl]-2-[4-(pyridin-2-ylsulfamoyl)-phenyl]-acetamide (50 mg, 0.86 mmol) in methanol (5 mL) was treated with a 10% w/v aqueous sodium hydroxide solution (0.1 mL, 0.25 mmol). The resulting solution was heated to reflux for until all of the starting material had been consumed. At this time, the reaction mixture was concentrated in vacuo. Flash chromatography (Merck Silica gel 60, 230-400 mesh, 5/95 methanol/methylene chloride) followed by trituration with cold methanol afforded 4-[3-butyl-1-(2-fluoro-benzyl)-2,6-dioxo-2,3,6,7-tetrahydro-1H-purin-8-ylmethyl]-*N-*pyridin-2-yl-benzenesulfonamide (14.7 mg, 26% from [4-(pyridin-2-ylsulfamoyl)-phenyl]-acetic acid) as a colorless solid; ¹H NMR(DMSO-d₆, 300 MHz) δ 13.50 (broad s, 1H), 7.90 (broad s, 1H), 7.74 (d, J = 8.1 Hz, 2H), 7.63 (t, J = 7.3 Hz, 1H), 7.37 (d, J = 7.7 Hz, 2H), 7.30-6.95 (m, 5H), 6.77 (broad s, 1H), 5.02 (s, 2H), 4.06 (s, 2H), 3.87 (t, J = 7.1 Hz, 2H), 1.54 (t, J = 7.0 Hz, 2H), 1.17 (q, J = 7.5 Hz, 2H), 0.77 (t, J = 7.3 Hz, 3H); FAB-HRMS m/e calculated for C₂₈H₂₇FN₆O₄S (M+H)⁺ 563.1877, found 563.1860.

The compounds cited in examples 29 to 33 were obtained in an anologous manner to that described in example 28.

### Example 29

### 4-[3-Butyl-1-(2-fluoro-benzyl)-2,6-dioxo-2,3,6,7-tetrahydro-1H-purin-8-ylmethyl]-N-pyridin-4-yl-benzenesulfonamide

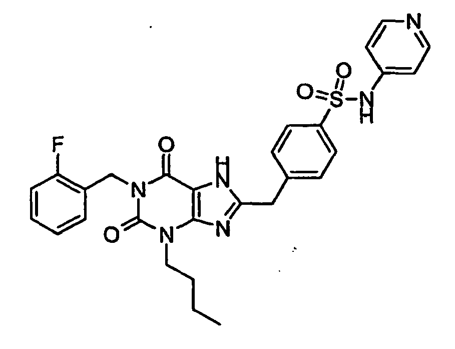

Prepared from 5,6-diamino-1-butyl-3-(2-fluoro-benzyl)-1H-pyrimidine-2,4-dione (30.6 mg, 0.1 mmol) and [4-(pyridin-4-ylsulfamoyl)-phenyl]-acetic acid. 4-[3-Butyl-1-(2-fluoro-benzyl)-2,6-dioxo-2,3,6,7-tetrahydro-1H-purin-8-ylmethyl]-*N*-pyridin-4-yl-benzenesulfonamide was obtained as a colorless solid; ¹H NMR (DMSO-d₆, 300 MHz) δ 13.50 (s, 1H), 12.65 (broad s, 1H), 8.00-7.80 (m, 2H), 7.75-7.60 (m, 2H), 7.55-7.35 (m, 2H), 7.30-6.80 (m, 6H), 5.08 (s, 2H), 4.11 (s, 2H), 3.93 (t, J = 7.3 Hz, 2H), 1.70-1.50 (m, 2H), 1.35-1.15 (m, 2H), 0.83 (t, J=7.1 Hz, 3H); FAB-HRMS m/e calculated for C₂₈H₂₇FN₆O₄S (M+H)⁺ 563.1877, found 563.1858.

### Example 30

### 4-[3-Butyl-1-(2-fluoro-benzyl)-2,6-dioxo-2,3,6,7-tetrahydro-1H-purin-8-ylmethyl]-N-pyridin-3-yl-benzenesulfonamide

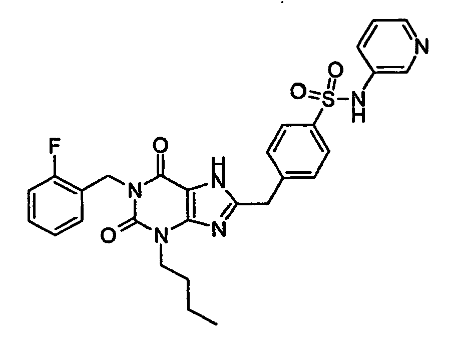

Prepared from 5,6-diamino-1-butyl-3-(2-fluoro-benzyl)-1H-pyrimidine-2,4-dione (30.6 mg, 0.1 mmol) and [4-(pyridin-3-ylsulfamoyl)-phenyl]-acetic acid. 4-[3-Butyl-1-(2-fluoro-benzyl)-2,6-dioxo-2,3,6,7-tetrahydro-1H-purin-8-ylmethyl]-*N*-pyridin-3-yl-benzenesulfonamide was obtained as a colorless solid; ¹H NMR (DMSO-d₆, 300 MHz) δ 13.50 (s, 1H), 10.54 (s, 1H), 8.23 (d, J = 14.3 Hz, 2H), 7.71 (d, J = 8.1 Hz, 2H), 7.55-7.40 (m, 3H), 7.35-6.95 (m, 5H), 5.08 (s, 2H), 4.13 (s, 2H), 3.92 (t, J = 7.3 Hz, 2H), 1.59 (t, J = 7.1 Hz, 2H), 1.23 (q, J = 7.3 Hz, 2H), 0.83 (t, J = 7.5 Hz, 3H); FAB-HRMS m/e calculated for C₂₈H₂₇FN₆O₄S (M+H)⁺ 563.1877, found 563.1860.

### Example 31

### 4-[3-Butyl-1-(2-fluoro-benzyl)-2,6-dioxo-2,3,6,7-tetrahydro-1H-purin-8-ylmethyl]-N-pyrimidin-2-yl-benzenesulfonamide

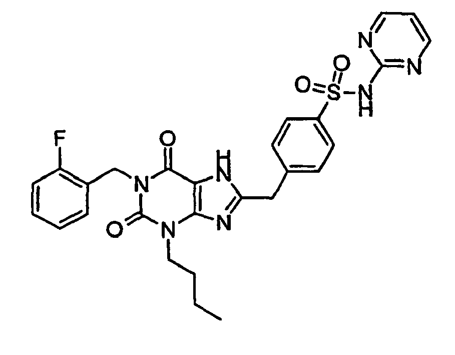

Prepared from 5,6-diamino-1-butyl-3-(2-fluoro-benzyl)-1H-pyrimidine-2,4-dione (30.6 mg, 0.1 mmol) and [4-(pyrimidine-2-sulfonylamino)-phenyl]-acetic acid. 4-[3-Butyl-1-(2-fluoro-benzyl)-2,6-dioxo-2,3,6,7-tetrahydro-1H-purin-8-ylmethyl]--N-pyrimidin-2-yl-benzenesulfonamide was obtained as a colorless solid; ¹H NMR (DMSO-d₆, 300 MHz) δ13.64 (s, 1H), 11.85 (broad s, 1H), 8.57 (d, J = 5.1 Hz, 2H), 8.02 (d, J = 8.4 Hz, 2H), 7.57 (d, J = 8.4 Hz, 2H), 7.40-7.00 (m, 5H), 5.18 (s, 2H), 4.25 (s, 2H), 4.03 (t, J = 7.1 Hz, 2H), 1.75-1.60 (m, 2H), 1.40-1.25 (m, 2H), 0.93 (t, J = 7.3 Hz, 3H); FAB-HRMS m/e calculated for C₂₇H₂₆FN₇O₄S (M+H)+ 564.1829, found 564.1835.

### Example 32

### 4- [3-Butyl-1-(2-fluoro-benzyl)-2,6-dioxo-2,3,6,7-tetrahydro-1H-purin-8-ylmethyl]-N-(1,3,5-trimethyl-1H-pyrazol-4-yl)-benzenesulfonamide

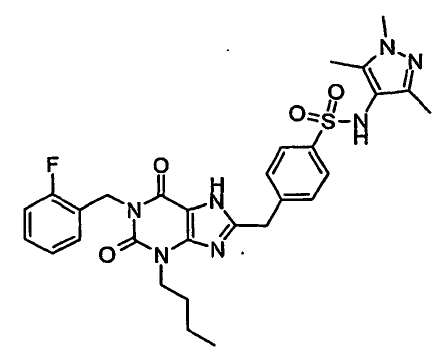

Prepared from 5,6-diamino-1-butyl-3-(2-fluoro-benzyl)-1H-pyrimidine-2,4-dione (30.6 mg, 0.1 mmol) and [4-(1,3,5-trimethyl-1H-pyrazol-4-ylsulfamoyl)-phenyl]-acetic acid. 4-[3-Butyl-1-(2-fluoro-benzyl)-2,6-dioxo-2,3,6,7-tetrahydro-1H-purin-8-ylmethyl]-*N*-(1,3,5-trimethyl-1H-pyrazol-4-yl)-benzenesulfonamide was obtained as a colorless solid; ¹H NMR (DMSO-d₆, 300 MHz) δ 13.48 (s, 1H), 8.94 (s, 1H), 7.49 (d, J = 8.4 Hz, 2H), 7.38 (d, J = 7.7 Hz, 2H), 7.30-6.90 (m, 4H), 5.03 (s, 2H), 4.10 (s, 2H), 3.88 (t, J = 7.1 Hz, 2H), 3.45 (s, 3H), 1.71 (s, 3H), 1.60-1.45 (m, 2H), 1.42 (s, 3H), 1.25-1.10 (m, 2H), 0.79 (t, J=7.5 Hz, 3H); FAB-HRMS m/e calculated for C₂₉H₃₂FN₇O₄S (M+H)⁺ 594.2299, found 594.2281.

### Example 33

### 4-[3-Butyl-1-(2-fluoro-benzyl)-2,6-dioxo-2,3,6,7-tetrahydro-1H-purin-8-ylmethyl]-N-thiazol-2-yl-benzenesulfonamide

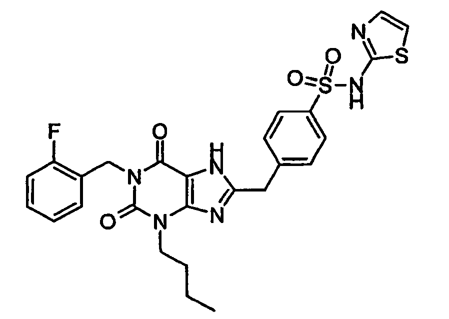

Prepared from 5,6-diamino-1-butyl-3-(2-fluoro-benzyl)-1H-pyrimidine-2,4-dione (30.6 mg, 0.1 mmol) and [4-(thiazol-2-ylsulfamoyl)-phenyl]-acetic acid. 4-[3-Butyl-1-(2-fluoro-benzyl)-2,6-dioxo-2,3,6,7-tetrahydro-1H-purin-8-ylmethyl]-N thiazol-2-yl-benzenesulfonamide was obtained as a colorless solid;1H NMR (DMSO-d6, 300 MHz) δH 13.53 (s, 1H), 12.70 (s, 1H), 7.73 (d, J = 8.1 Hz, 2H), 7.43 (d, J = 8.1 Hz, 2H), 7.35-6.95 (m, 5H), 6.80 (d, J = 4.8 Hz, 1H), 5.08 (s, 2H), 4.13 (s, 2H), 3.93 (t, J = 7.1 Hz, 2H), 1.70-1.50 (m, 2H), 1.30-1.15 (m, 2H), 0.84 (t, J = 7.3 Hz, 3H); FAB-HRMS m/e calculated for C₂₆H₂₅FN₆O₄S₂ (M+H)⁺ 569.1441, found 569.1431.

### Example 34

### 5-Chloro-1,3-dimethyl-1H-pyrazole-4-sulfonic acid {4-[3-butyl-1-(2-fluoro-benzyl)-2,6-dioxo-2,3,6,7-tetrahydro-1H-purin-8-ylmethyl]-phenyl}-amide

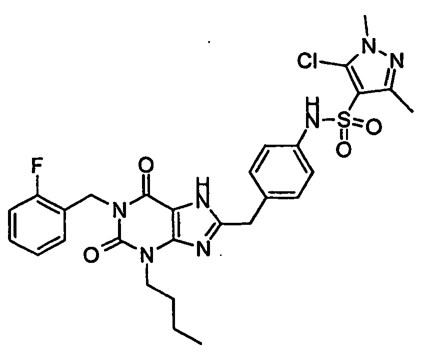

This compound was prepared by the routes outlined in schemes 3 and 4.

### Step 1: Preparation of 1-butyl-6-chloro-1H-pyrimidine-2,4-dione.

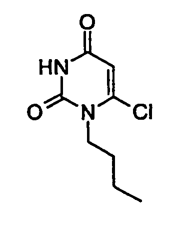

This compound was prepared by the method of Ishikawa et al. as described in Heterocycles 1990, 31(9), 1641.

Chloro-uracil (Lancaster) (23.36 g, 0.16 mol) was dissolved in dimethyl sulfoxide (100 mL) and treated with potassium carbonate (11.2 g, 0.08 mol) and 1-iodobutane (Aldrich) (52.8 mL, 0.48 mol). After stirring at 23 °C for 18 h, the reaction was then mixed with water and extracted with ethyl acetate (3x). The combined ethyl acetate layers were then washed with diluted aqueous sodium chloride solution and brine, dried (sodium sulfate) and concentrated to dryness to afford the crude product as an off-white solid (27.34 g, 85 %). ¹H NMR (CDCl₃, 200 MHz) δ 0.95 (t, 3H),1.40 (m, 2H), 1.58 (m, 2H), 4.02 (t, 2H), 5.80 (s, 1H), 8.93 (br s, 1H).

### Step 2: Preparation of 1-butyl-6-chloro-3-(2-fluorobenzyl)-1H-pyrimidine-2,4-dione.

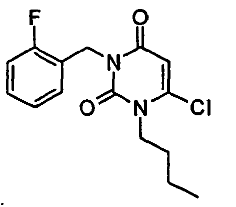

The crude product from step 1 (3.0 g, 14.8 mmol) was dissolved in *N,N-*dimethylformamide (30 mL) and treated with potassium carbonate (4.08 g, 29.6 mmol) and 2-fluorobenzyl bromide (Aldrich) (1.8 ml, 14.8 mmol). The reaction was stirred at 23 °C for 2 hours and then at 48 °C for 3 hours. The reaction was mixed with diluted brine and extracted with ethyl acetate (3X). The combined ethyl acetate extracts were then washed with diluted aqueous sodium chloride solution and brine, dried (sodium sulfate) and concentrated to dryness to afford 1-butyl-6-chloro-3-(2-fluorobenzyl)-1H-pyrimidine-2,4-dione as a yellow oil (4.07 g, 89 %). ¹H NMR (CDCl₃, 200 MHz) δ 0.96 (t, 3H), 1.25-1.48 (m, 2H), 1.58-1.76 (m, 2H), 4.03 (t, 2H), 5.20 (s, 2H), 5.97 (s, 1H), 6.97-7.10 (m, 2H), 7.17-7.30 (m, 2H).

### Step 3: Preparation of 1-butyl-3-(2-fluorobenzyl)-6-[2-(4-nitro-phenyl)-ethylamino]-1H-pyrimidine-2,4-dione.

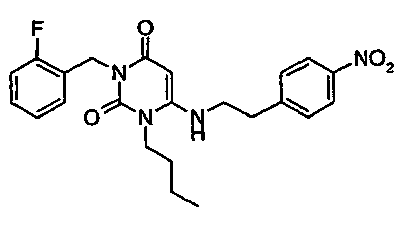

A mixture of 2-(4-nitro-phenyl)-ethylamine hydrochloride salt (Fluka) (7.85g, 38.7 mmol), 1-butyl-6-chloro-3-(2-fluorobenzyl)-1H-pyrimidine-2,4-dione (8.0g, 25.8 mmol) and triethylamine (10.7 mL, 77 mmol) in *N*-methyl-pyrrolidin-2-one (200 mL) was stirred at 75°C for 16 hours. The reaction was then mixed with water and extracted with ethyl acetate (3X). The combined ethyl acetate layers were dried (magnesium sulfate) and concentrated. Column chromatography afforded 1-butyl-3-(2-fluorobenzyl)-6-[2-(4-nitro-phenyl)-ethylamino]-1H-pyrimidine-2,4-dione (4.7 g, 41 %) as a solid. ¹H NMR (CDCl₃, 200 MHz) δ 0.84 (t, 3H), 1.11-1.50 (m, 4H), 3.10 (t, 2H), 3.48 (m, 2H), 3.70 (t, 2H), 4.36 (t, 1H), 4.94 (t, 1H), 5.20 (s, 2H), 6.95-7.10 (m, 2H), 7.17-7.30 (m, 2H), 7.39 (d, 2H), 8.20 (d, 2H). Step 4: Preparation of 3-butyl-1-(2-fluorobenzyl)-8-(4-nitro-benzyl)-3,7-dihydro-purine-2,6-dione.

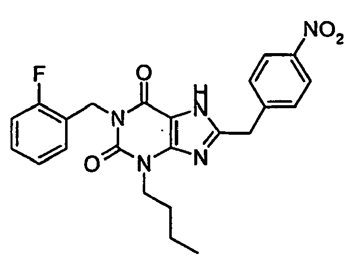

Butyl-3-(2-fluorobenzyl)-6- [2-(4-nitro-phenyl)-ethylamino]-1H-pyrimidine-2,4-dione (2.3 g, 5.2 mmol) was dissolved in ethanol (20 mL) and treated with isoamyl nitrite (Aldrich) (3.6 mL, 26 mmol). Concentrated aqueous hydrochloric acid (1 mL) was added to the reaction mixture. The reaction was stirred at 23 °C for 40 minutes. The ethanol was removed under reduced pressure and the residue washed with diethyl ether. The solid residue was then dissolved in n-butanol (15 mL), and the mixture refluxed for 30 minutes. After cooling to room temperature 1-butyl-3-(2-fluorobenzyl)-6-[2-(4-nitro-phenyl)-ethylamino]-1H-pyrimidine-2,4-dione separated as pale yellow crystals which were collected by filtration (1.91 g, 81 %). LCMS, m/z(M+H) = 452.24.

### Step 5: Preparation of 8-(4-amino-benzyl)-3-butyl-1-(2-fluorobenzyl)-3,7-dihydro-purine-2,6-dione.

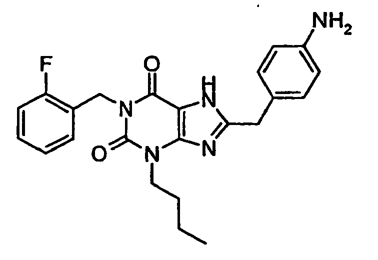

Butyl-3-(2-fluorobenzyl)-6- [2-(4-nitro-phenyl)-ethylamino]-1H-pyrimidine-2,4-dione (2.0 g, 4.46 mmol) was dissolved in methanol (100 mL) and treated with zinc dust (< 10 µm, Aldrich; 2.91 g) followed by the addition of a solution of ammonium chloride (5.96 g,112 mmol) in water (50 mL). The reaction mixture was stirred at room temperature for 3 h. The reaction mixture was then filtered through a pad of celite. The filtrate was concentrated to remove methanol and the residual aquous solution was extracted with ethyl acetate (3x). The combined organic extracts were washed with brine, dried (sodium sulfate) and concentrated in vacuo to afford 8-(4-amino-benzyl)-3-butyl-1-(2-fluorobenzyl)-3,7-dihydro-purine-2,6-dione as a pale yellow solid (1.64 g, 87%). LCMS, m/z(M+H) = 422.18.

### Step 6: Preparation of 5-chloro-1,3-dimethyl-1H-pyrazole-4-sulfonic acid {4-[3-butyl-1-(2-fluoro-benzyl)-2,6-dioxo-2,3,6,7-tetrahydro-1H-purin-8-ylmethyl]-phenyl}-amide.

8-(4-Amino-benzyl)-3-butyl-1-(2-fluoro-benzyl)-3,7-dihydro-purine-2,6-dione (20 mg, 0.047 mmol) was dissolved in pyridine (0.5 ml) and treated with 5-chloro-1,3-dimethyl-1H-pyrazole-4-sulfonyl chloride (Maybridge, 16 mg, 0.07 mmol). The reaction was stirred at 23 °C for 3 h. The solvent was removed and the residue was purified by reverse phase HPLC. LCMS, m/z (M+H) = 615.2.

The compounds cited in examples 35 to 93 were obtained in an anologous manner to that described in example 34.

### Example 35

### Propane-2-sulfonic acid {4-[3-butyl-1-(2-fluoro-benzyl)-2,6-dioxo-2,3,6,7-tetrahydro-1H-purin-8-ylmethyl]-phenyl}-amide

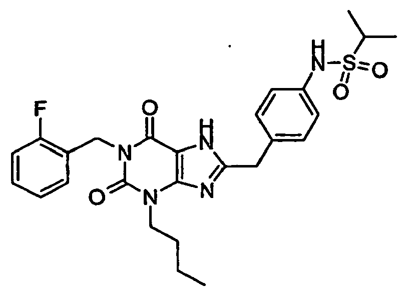

Prepared from 8-(4-amino-benzyl)-3-butyl-1-(2-fluoro-benzyl)-3,7-dihydro-purine-2,6-dione and propane-2-sulfonyl chloride. Purity (ELSD, based on MW = 527.6) = 84%.

### Example 36

### Ethanesulfonic acid {4-[3-butyl-1-(2-fluoro-benzyl)-2,6-dioxo-2,3,6,7-tetrahydro-1H-purin-8-ylmethyl]-phenyl}-amide

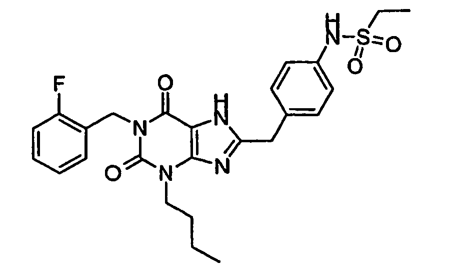

Prepared from 8-(4-amino-benzyl)-3-butyl-1-(2-fluoro-benzyl)-3,7-dihydro-purine-2,6-dione and ethanesulfonyl chloride. Purity (ELSD, based on MW = 513.6) = 91%.

### Example 37

### N-{4-[3-Butyl-1-(2-fluoro-benzyl)-2,6-dioxo-2,3,6,7-tetrahydro-1H-purin-8-ylmethyl]-phenyl}-C-phenyl-methanesulfonamide

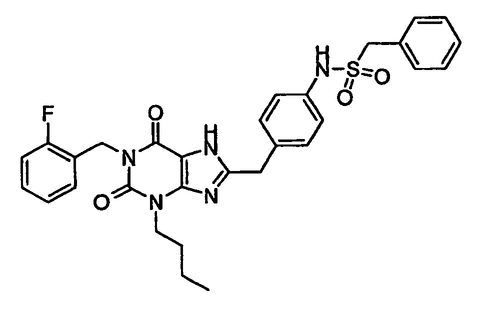

Prepared from 8-(4-amino-benzyl)-3-butyl-1-(2-fluoro-benzyl)-3,7-dihydro-purine-2,6-dione and benzylsulfonyl chloride. Purity (ELSD, based on MW = 575.7) = 80%.

### Example 38

### Phenyl-ethenesulfonic acid {4-[3-butyl-1-(2-fluoro-benzyl)-2,6-dioxo-2,3,6,7-tetrahydro-1H-purin-8-ylmethyl]-phenyl}-amide

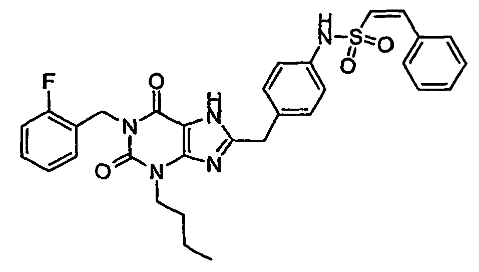

Prepared from 8-(4-amino-benzyl)-3-butyl-1-(2-fluoro-benzyl)-3,7-dihydro-purine-2,6-dione and 2-phenyl-ethenesulfonyl chloride. Purity (ELSD, based on MW = 587.7) = 97%.

### Example 39

### N-{4-[3-Butyl-1-(2-fluoro-benzyl)-2,6-dioxo-2,3,6,7-tetrahydro-1H-purin-8-ylmethyl]-phenyl}-benzenesulfonamide

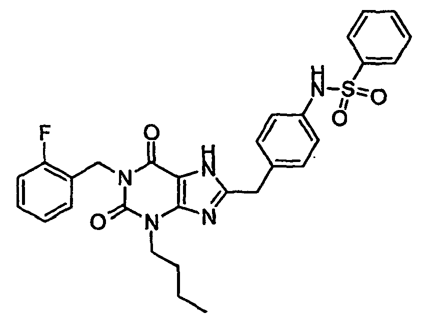

Prepared from 8-(4-anzino-benzyl)-3-butyl-1-(2-fluoro-benzyl)-3,7-dihydro-purine-2,6-dione and -benzenesulfonyl chloride. Purity (ELSD, based on MW = 561.6) = 95%.

### Example 40

### N-{4-[3-Butyl-1-(2-fluoro-benzyl)-2,6-dioxo-2,3,6,7-tetrahydro-1H-purin-8-ylmethyl]-phenyl}-2-fluoro-benzenesulfonamide

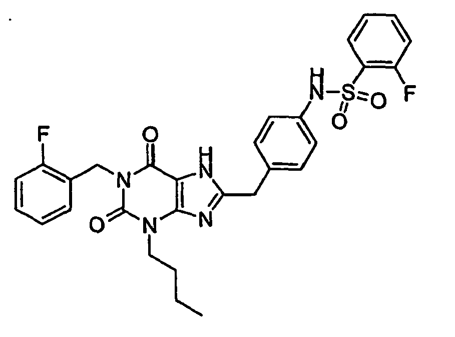

Prepared from 8-(4-amino-benzyl)-3-butyl-1-(2-fluoro-benzyl)-3,7-dihydro-purine-2,6-dione and 2-fluoro-benzenesulfonyl chloride. Purity (ELSD, based on MW = 579.6) = 80%.

### Example 41

### N-{4-[3-Butyl-1-(2-fluoro-benzyl)-2,6-dioxo-2,3,6,7-tetrahydro-1H-purin-8-ylmethyl]-phenyl}-2-trifluoromethyl-benzenesulfonamide

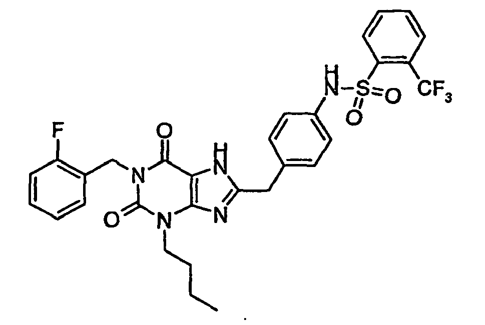

Prepared from 8-(4-amino-benzyl)-3-butyl-1-(2-fluoro-benzyl)-3,7-dihydro-purine-2,6-dione and 2-(trifluoromethyl)-benzenesulfonyl chloride. Purity (ELSD, based on MW = 629.6) = 90%.

### Example 42

### N-{4-[3-Butyl-1-(2-fluoro-benzyl)-2,6-dioxo-2,3,6,7-tetrahydro-1H-purin-8-ylmethyl]-phenyl}-2-chloro-benzenesulfonamide

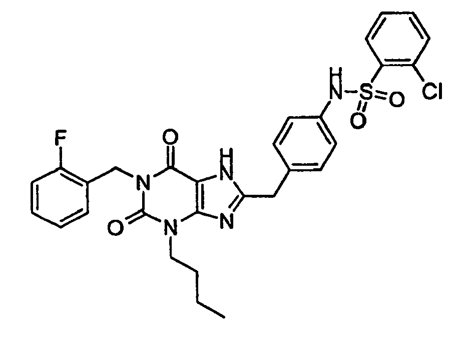

Prepared from 8-(4-amino-benzyl)-3-butyl-1-(2-fluoro-benzyl)-3,7-dihydro-purine-2,6-dione and 2-chloro-benzenesulfonyl chloride. Purity (ELSD, based on MW = 596.1) = 70%.

### Example 43

### N-{4-[3-Butyl-1-(2-fluoro-benzyl)-2,6-dioxo-2,3,6,7-tetrahydro-1H-purin-8-ylmethyl]-phenyl}-2-methyl-benzenesulfonamide

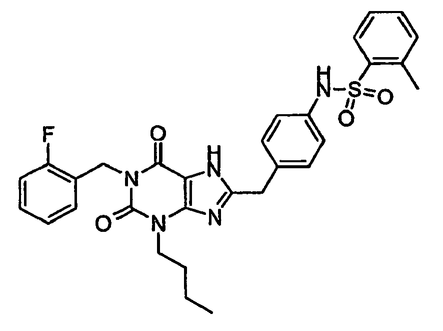

Prepared from 8-(4-amino-benzyl)-3-butyl-1-(2-fluoro-benzyl)-3,7-dihydro-purine-2,6-dione and 2-methyl-benzenesulfonyl chloride. Purity (ELSD, based on MW = 575.7) = 82%.

### Example 44

### N-{4-[3-Butyl-1-(2-fluoro-benzyl)-2,6-dioxo-2,3,6,7-tetrahydro-1H-purin-8-ylmethyl]-phenyl}-3-chloro-benzenesulfonamide

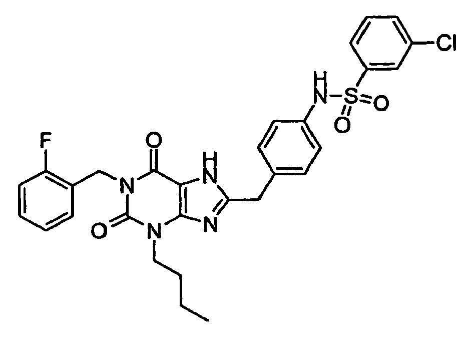

Prepared from 8-(4-amino-benzyl)-3-butyl-1-(2-fluoro-benzyl)-3,7-dihydro-purine-2,6-dione and 3-chloro-benzenesulfonyl chloride. Purity (ELSD, based on MW = 596.1) = 90%.

### Example 45

### N-{4-[3-Butyl-1-(2-fluoro-benzyl)-2,6-dioxo-2,3,6,7-tetrahydro-1H-purin-8-ylmethyl]-phenyl}-3-trifluoromethyl-benzenesulfonamide

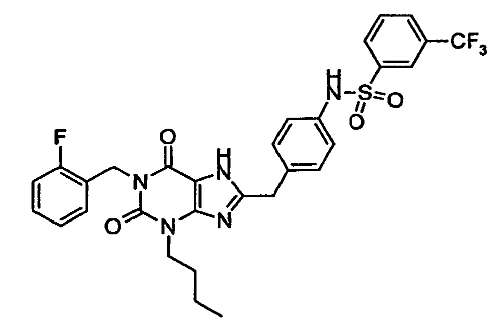

Prepared from 8-(4-amino-benzyl)-3-butyl-1-(2-fluoro-benzyl)-3,7-dihydro-purine-2,6-dione and 3-(trifluoromethyl)-benzenesulfonyl chloride. Purity (ELSD, based on MW = 629.6) = 97%.

### Example 46

### N-{4-[3-Butyl-1-(2-fluoro-benzyl)-2,6-dioxo-2,3,6,7-tetrahydro-1H-purin-8-ylmethyl]-phenyl}-3-nitro-benzenesulfonamide

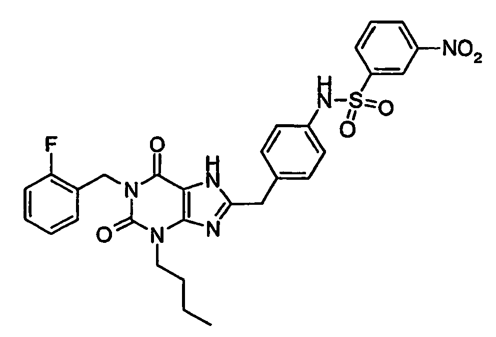

Prepared from 8-(4-amino-benzyl)-3-butyl-1-(2-fluoro-benzyl)-3,7-dihydro-purine-2,6-dione and 3-nitro-benzenesulfonyl chloride. Purity (ELSD, based on MW = 606.6) = 75%.

### Example 47

### N-{4-[3-Butyl-1-(2-fluoro-benzyl)-2,6-dioxo-2,3,6,7-tetrahydro-1H-purin-8-ylmethyl] - phenyl}-3-methyl-benzenesulfonamide

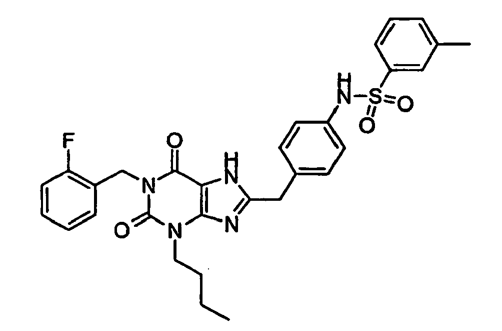

Prepared from 8-(4-amino-benzyl)-3-butyl-1-(2-fluoro-benzyl)-3,7-dihydro-purine-2,6-dione and 3-methyl-benzenesulfonyl chloride. Purity (ELSD, based on MW = 575.7) = 93%.

### Example 48

### N-{4-[3-Butyl-1-(2-fluoro-benzyl)-2,6-dioxo-2,3,6,7-tetrahydro-1H-purin-8-ylmethyl]-phenyl}-4-bromo-benzenesulfonamide

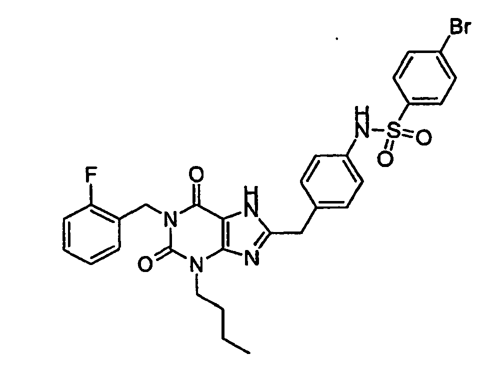

Prepared from 8-(4-amino-benzyl)-3-butyl-1-(2-fluoro-benzyl)-3,7-dihydro-purine-2,6-dione and 4-bromo-benzenesulfonyl chloride. Purity (ELSD, based on MW = 640.5) = 100%.

### Example 49

### N-{4-[3-Butyl-1-(2-fluoro-benzyl)-2,6-dioxo-2,3,6,7-tetrahydro-1H-purin-8-ylmethyl]-phenyl}-4-nitro-benzenesulfonamide

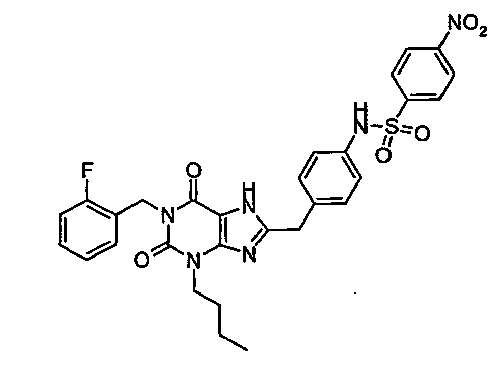

Prepared from 8-(4-amino-benzyl)-3-butyl-1-(2-fluoro-benzyl)-3,7-dihydro-purine-2,6-dione and 4-nitro-benzenesulfonyl chloride. Purity (ELSD, based on MW = 606.6) = 98%.

### Example 50

### N-{4-[3-Butyl-1-(2-fluoro-benzyl)-2,6-dioxo-2,3,6,7-tetrahydro-1H-purm-8-ylmethyl]-phenyl}-4-chloro-benzenesulfonamide

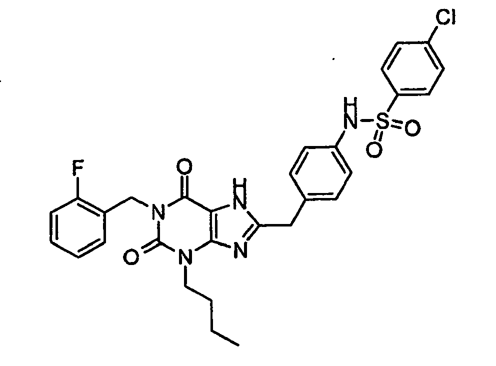

Prepared from 8-(4-amino-benzyl)-3-butyl-1-(2-fluoro-benzyl)-3,7-dihydro-purine-2,6-dione and 4-chloro-benzenesulfonyl chloride. Purity (ELSD, based on MW = 596.1) = 97%.

### Example 51

### N-{4-[3-Butyl-1-(2-fluoro-benzyl)-2,6-dioxo-2,3,6,7-tetrahydro-1H-purin-8-ylmethyl]-phenyl}-4-iodo-benzenesulfonamide

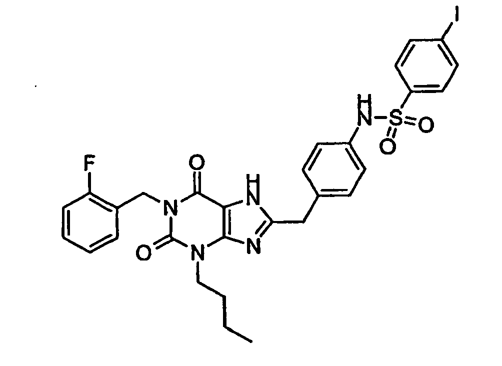

Prepared from 8-(4-amino-benzyl)-3-butyl-1-(2-fluoro-benzyl)-3,7-dihydro-purine-2,6-dione and 4-iodo-benzenesulfonyl chloride. Purity (ELSD, based on MW = 687.5) = 100%.

### Example 52

### N-{4-[3-Butyl-1-(2-fluoro-benzyl)-2,6-dioxo-2,3,6,7-tetrahydro-1H-purin-8-ylmethyl]-phenyl}-4-isopropyl-benzenesulfonamide

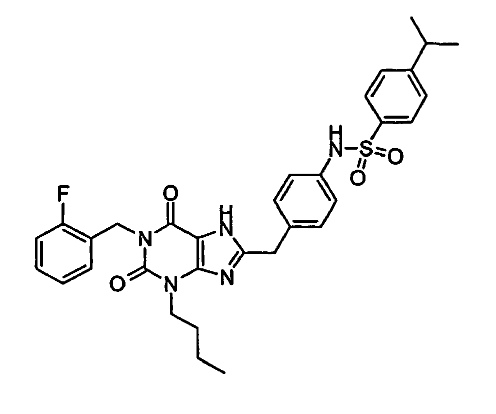

Prepared from 8-(4-amino-benzyl)-3-butyl-1-(2-fluoro-benzyl)-3,7-dihydro-purine-2,6-dione and 4-isopropyl-benzenesulfonyl chloride. Purity (ELSD, based on MW = 603.7) = 97%.

### Example 53

### N-{4-[3-Butyl-1-(2-fluoro-benzyl)-2,6-dioxo-2,3,6,7-tetrahydro-1H-purin-8-ylmethyl]-phenyl} -4-methoxy-benzenesulfonamide

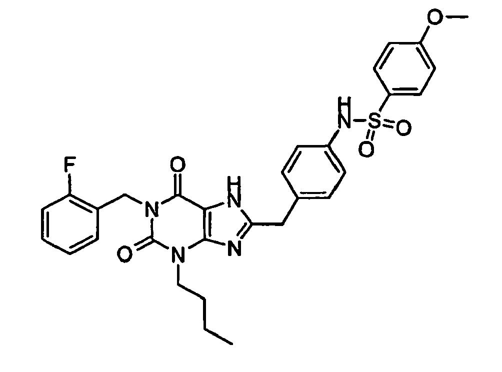

Prepared from 8-(4-amino-benzyl)-3-butyl-1-(2-ffuoro-benzyl)-3,7-dihydro-purine-2,6-dione and 4-methoxy-benzenesulfonyl chloride. Purity (ELSD, based on MW = 591.7) = 94%.

### Example 54

### N-{4-[3-Butyl-1-(2-fluoro-benzyl)-2,6-dioxo-2,3,6,7-tetrahydro-1H-purin-8-ylmethyl]-phenyl}-4-trifluoromethyl-benzenesulfonamide

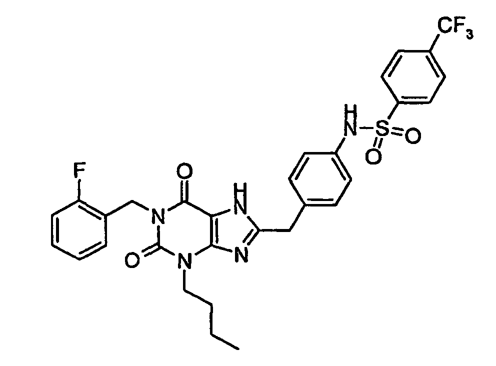

Prepared from 8-(4-amino-benzyl)-3-butyl-1-(2-fluoro-benzyl)-3,7-dihydro-purine-2,6-dione and 4-(trifluoromethyl)-benzenesulfonyl chloride. Purity (ELSD, based on MW = 629.6) = 94%.

### Example 55

### N-{4-[3-Butyl-1-(2-fluoro-benzyl)-2,6-dioxo-2,3,6,7-tetrahydro-1H-purin-8-ylmethyl]-phenyl}-4-ethyl-benzenesulfonamide

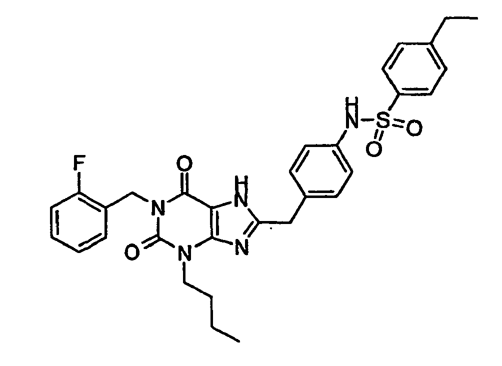

Prepared from 8-(4-amino-benzyl)-3-butyl-1-(2-fluoro-benzyl)-3,7-dihydro-purine-2,6-dione and 4-ethyl-benzenesulfonyl chloride. Purity (ELSD, based on MW = 589.7) = 90%.

### Example 56

### N-{4-[3-Butyl-1-(2-fluoro-benzyl)-2,6-dioxo-2,3,6,7-tetrahydro-1H-purin-8-ylmethyl]-phenyl}-4-trifluoromethoxy-benzenesulfonamide

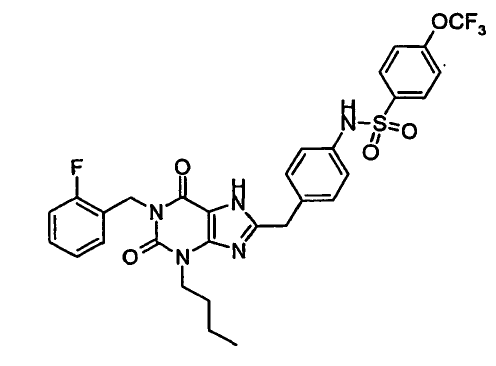

Prepared from 8-(4-amino-benzyl)-3-butyl-1-(2-fluoro-benzyl)-3,7-dihydro-purine-2,6-dione and 4-(trifluoromethoxy)-benzenesulfonyl chloride. Purity (ELSD, based on MW = 645.6) = 87%.

### Example 57

### N-{4-[3-Butyl-1-(2-fluoro-benzyl)-2,6-dioxo-2,3,6,7-tetrahydro-1H-purin-8-ylmethyl]-phenyl}-4-fluoro-benzenesulfonamide

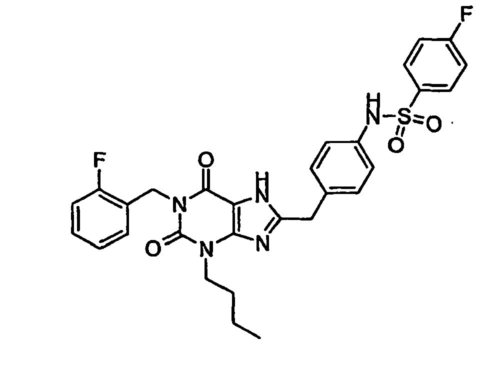

Prepared from 8-(4-amino-benzyl)-3-butyl-1-(2-fluoro-benzyl)-3,7-dihydro-purine-2,6-dione and 4-fluoro-benzenesulfonyl chloride. Purity (ELSD, based on MW = 579.6) = 93%.

### Example 58

### N-{4-[3-Butyl-1-(2-auoro-benzyl)-2,6-dioxo-2,3,6,7-tetrahydro-1H-punn-8-ylmethyl]-phenyl}-2,4-dichloro-benzenesulfonamide

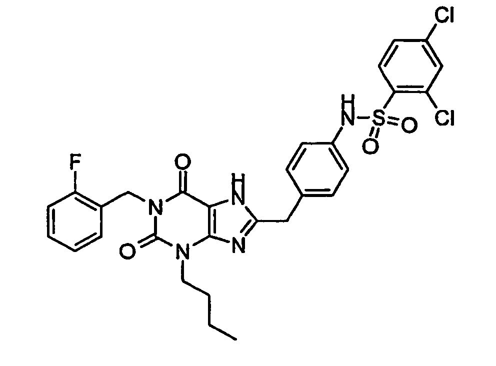

Prepared from 8-(4-amino-benzyl)-3-butyl-1-(2-fluoro-benzyl)-3,7-dihydro-purine-2,6-dione and 2,4-dichloro-benzenesulfonyl chloride. Purity (ELSD, based on MW = 630.5) = 91%.

### Example 59

### N-{4-[3-Butyl-1-(2-fluoro-benzyl)-2,6-dioxo-2,3,6,7-tetrahydro-1H-purin-8-ylmethyl]-phenyl}-2,5-dimethoxy-benzenesulfonamide

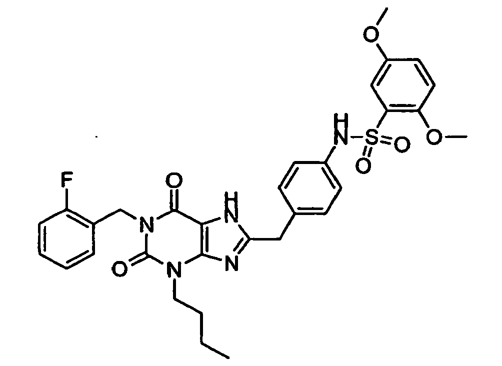

Prepared from 8-(4-amino-benzyl)-3-butyl-1-(2-fluoro-benzyl)-3,7-dihydro-purine-2,6-dione and 2,5-dimethoxy-benzenesulfonyl chloride. Purity (ELSD, based on MW = 621.7) = 90%.

### Example 60

### N-{4-[3-Butyl-1-(2-fluoro-benzyl)-2,6-dioxo-2,3,6,7-tetrahydro-1H-purin-8-ylmethyl]-phenyl}-3-chloro-4-methyl-benzenesulfonamide

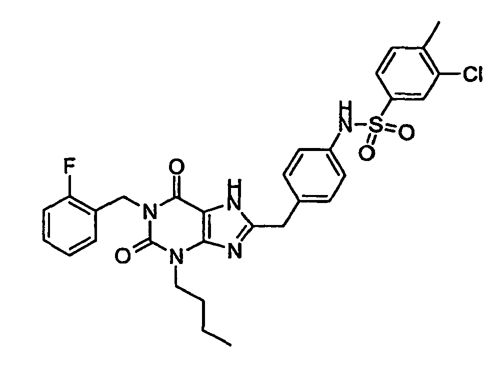

Prepared from 8-(4-amino-benzyl)-3-butyl-1-(2-fluoro-benzyl)-3,7-dihydro-purine-2,6-dione and 3-chloro-4-methyl-benzenesulfonyl chloride. Purity (ELSD, based on MW = 610.1) = 98%.

### Example 61

### N-{4-[3-Butyl-1-(2-fluoro-benzyl)-2,6-dioxo-2,3,6,7-tetrahydro-1H-purin-8-ylmethyl]-phenyl}-2-methoxy-5-methyl-benzenesulfonamide

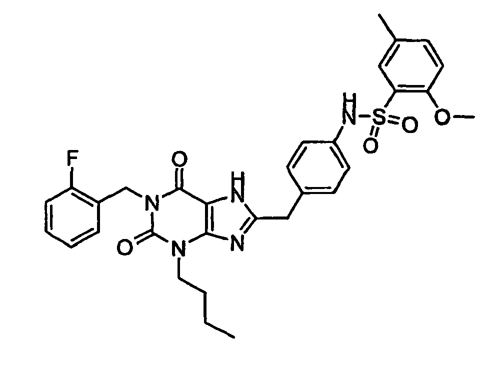

Prepared from 8-(4-amino-benzyl)-3-butyl-1-(2-fluoro-benzyl)-3,7-dihydro-purine-2,6-dione and 2-methoxy-5-methyl-benzenesulfonyl chloride. Purity (ELSD, based on MW = 605.7) = 94%.

### Example 62

### N-{4-[3-Butyl-1-(2-fluoro-benzyl)-2,6-dioxo-2,3,6,7-tetrahydro-1H-purin-8-ylmethyl]-phenyl}-2,5-dichloro-benzenesulfonamide

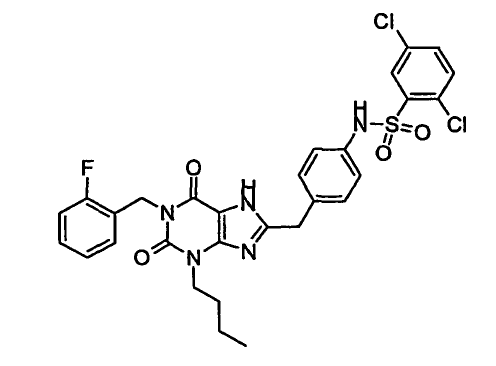

Prepared from 8-(4-amino-benzyl)-3-butyl-1-(2-fluoro-benzyl)-3,7-dihydro-purine-2,6-dione and 2,5-dichloro-benzenesulfonyl chloride. Purity (ELSD, based on MW = 630.5) = 60%.

### Example 63

### N-{4-[3-Butyl-1-(2-fluoro-benzyl)-2,6-dioxo-2,3,6,7-tetrahydro-1H-purin-8-ylmethyl]-phenyl}-3-chloro-2-methyl-benzenesulfonamide

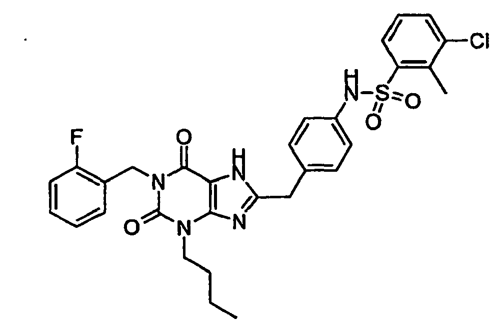

Prepared from 8-(4-amino-benzyl)-3-butyl-1-(2-fluoro-benzyl)-3,7-dihydro-purine-2,6-dione and 3-chloro-2-methyl-benzenesulfonyl chloride. Purity (ELSD, based on MW = 610.1) = 80%.

### Example 64

### N-{4-[3-Butyl-1-(2-fluoro-benzyl)-2,6-dioxo-2,3,6,7-tetrahydro-1H-purin-8-ylmethyl] - phenyl}-2,6-dichloro-benzenesulfonamide

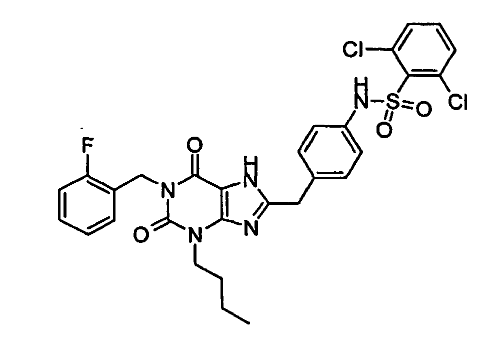

Prepared from 8-(4-amino-benzyl)-3-butyl-1-(2-fluoro-benzyl)-3,7-dihydro-purine-2,6-dione and 2,6-dichloro-benzenesulfonyl chloride. Purity (ELSD, based on MW = 630.5) = 80%.

### Example 65

### N-{4-[3-Butyl-1-(2-fluoro-benzyl)-2,6-dioxo-2,3,6,7-tetrahydro-1H-purin-8-ylmethyl]-phenyl}-2,3-dichloro-benzenesulfonamide

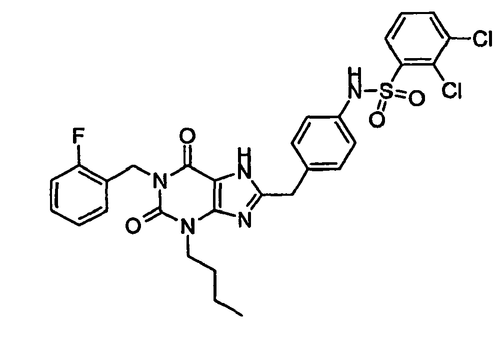

Prepared from 8-(4-amino-benzyl)-3-butyl-1-(2-fluoro-benzyl)-3,7-dihydro-purine-2,6-dione and 2,3-dichloro-benzenesulfonyl chloride. Purity (ELSD, based on MW = 630.5) = 70%.

### Example 66

### N-{4-[3-Butyl-1-(2-fluoro-benzyl)-2,6-dioxo-2,3,6,7-tetrahydro-1H-purin-8-ylmethyl]-phenyl}-3,4-dimethoxy-benzenesulfonamide

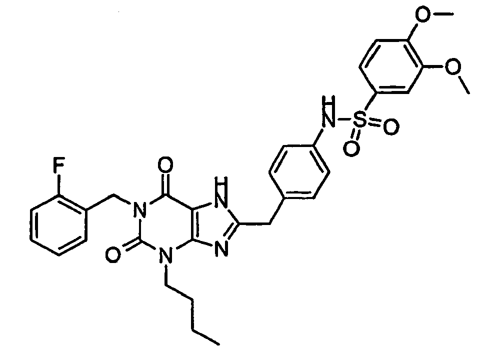

Prepared from 8-(4-amino-benzyl)-3-butyl-1-(2-fluoro-benzyl)-3,7-dihydro-purine-2,6-dione and 3,4-dimethoxy-benzenesulfonyl chloride. Purity (ELSD, based on MW = 621.7) = 94%.

### Example 67

### N- {4-[3-Butyl-1-(2-fluoro-benzyl)-2,6-dioxo-2,3,6,7-tetrahydro-1H-purin-8-ylmethyl]-phenyl}-2-chloro-6-methyl-benzenesulfonamide

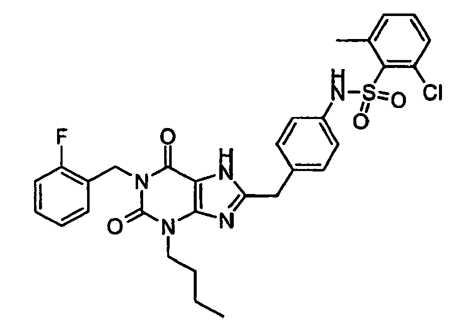

Prepared from 8-(4-amino-benzyl)-3-butyl-1-(2-fluoro-benzyl)-3,7-dihydro-purine-2,6-dione and 2-chloro-6-methyl-benzenesulfonyl chloride. Purity (ELSD, based on MW= 610.1) =70%.

### Example 68

### N-{4-[3-Butyl-1-(2-fluoro-benzyl)-2,6-dioxo-2,3,6,7-tetrahydro-1H-purin-8-ylmethyl]-phenyl}-3,5-dichloro-benzenesulfonamide

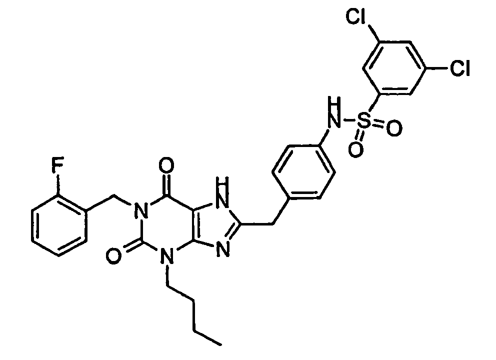

Prepared from 8-(4-amino-benzyl)-3-butyl-1-(2-fluoro-benzyl)-3,7-dihydro-purine-2,6-dione and 3,5-dichloro-benzenesulfonyl chloride. Purity (ELSD, based on MW = 630.5) = 97%.

### Example 69

### N-{4-[3-Butyl-1-(2-fluoro-benzyl)-2,6-dioxo-2,3,6,7-tetrahydro-1H-purin-8-ylmethyl]-phenyl}-3,4-dichloro-benzenesulfonamide

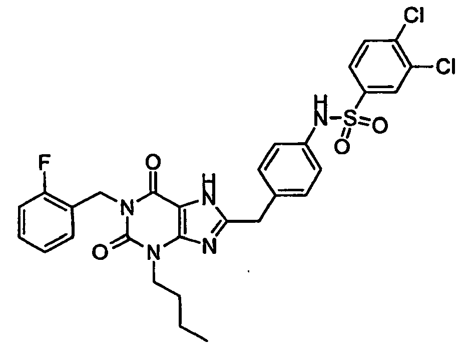

Prepared from 8-(4-amino-benzyl)-3-butyl-1-(2-fluoro-benzyl)-3,7-dihydro-purine-2,6-dione and 3,4-dichloro-benzenesulfonyl chloride. Purity (ELSD, based on MW = 630.5) = 100%.

### Example 70

### N-{4-[3-Butyl-1-(2-fluoro-benzyl)-2,6-dioxo-2,3,6,7-tetrahydro-1H-purin-8-ylmethyl]-phenyl}-2,5-dimethyl-benzenesulfonamide

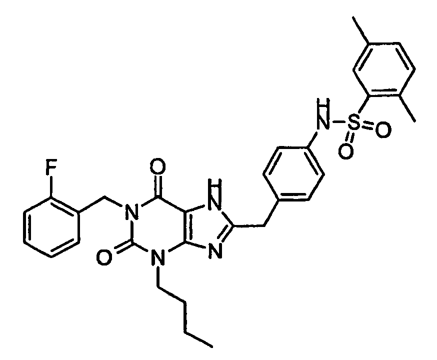

Prepared from 8-(4-amino-benzyl)-3-butyl-1-(2-fluoro-benzyl)-3,7-dihydro-purine-2,6-dione and 2,5-dimethyl-benzenesulfonyl chloride. Purity (ELSD, based on MW = 589.7) = 85%.

### Example 71

### N-{4-[3-Butyl-1-(2-fluoro-benzyl)-2,6-dioxo-2,3,6,7-tetrahydro-1H-purin-8-ylmethyl]-phenyl}-5-fluoro-2-methyl-benzenesulfonamide

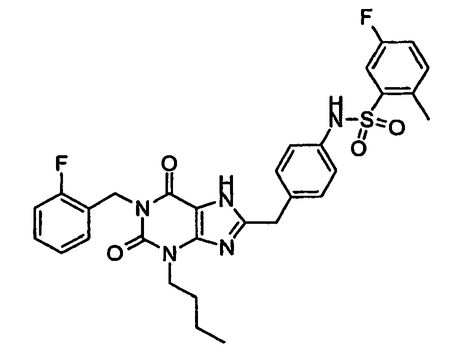

Prepared from 8-(4-amino-benzyl)-3-butyl-1-(2-fluoro-benzyl)-3,7-dihydro-purine-2,6-dione and 5-fluoro-2-methyl-benzenesulfonyl chloride. Purity (ELSD, based on MW = 593.7) = 92%.

### Example 72

### N-{4-[3-Butyl-1-(2-fluoro-benzyl)-2,6-dioxo-2,3,6,7-tetrahydro-1H-purin-8-ylmethyl]-phenyl}-2-chloro-4-fluoro-benzenesulfonamide

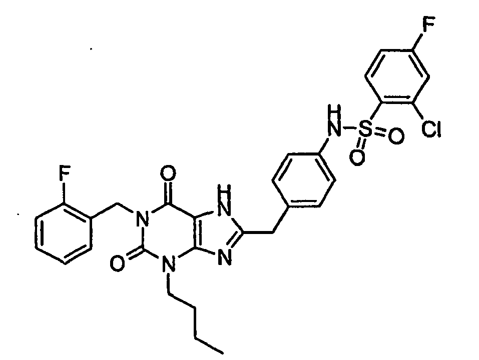

Prepared from 8-(4-amino-benzyl)-3-butyl-1-(2-fluoro-benzyl)-3,7-dihydro-purine-2,6-dione and 2-chloro-4-fluoro-benzenesulfonyl chloride. Purity (ELSD, based on MW = 614.1) = 93%.

### Example 73

### N-{4-[3-Butyl-1-(2-tluoro-benzyl)-2,6-dioxo-2,3,6,7-tetrahydro-1H-purin-8-ylmethyl]-phenyl}-2,4-difluoro-benzenesulfonamide

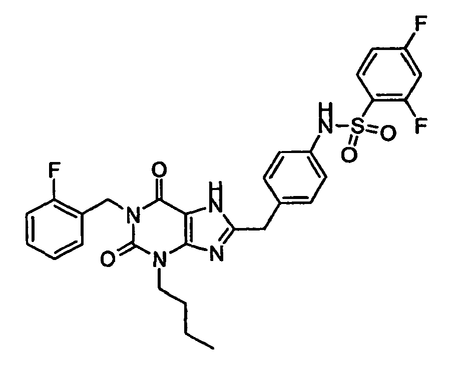

Prepared from 8-(4-amino-benzyl)-3-butyl-1-(2-fluoro-benzyl)-3,7-dihydro-purine-2,6-dione and 2,4-difluoro-benzenesulfonyl chloride. Purity (ELSD, based on MW = 597.6) = 95%.

### Example 74

### N-{4-[3-Butyl-1-(2-fluoro-benzyl)-2,6-dioxo-2,3,6,7-tetrahydro-1H-purin-8-ylmethyl]-phenyl}-2-methyl-5-nitro-benzenesulfonamide

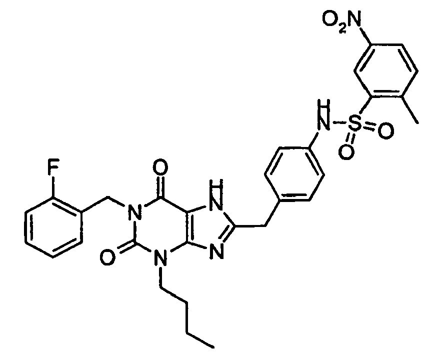

Prepared from 8-(4-amino-benzyl)-3-butyl-1-(2-ffuoro-benzyl)-3,7-dihydro-purine-2,6-dione and 2-methyl-5-nitro-benzenesulfonyl chloride. Purity (ELSD, based on MW = 620.7) = 88%.

### Example 75

### N-{4-[3-Butyl-1-(2-fluoro-benzyl)-2,6-dioxo-2,3,6,7-tetrahydro-1H-purin-8-ylmethyl]-phenyl}-3-chloro-4-fluoro-benzenesulfonamide

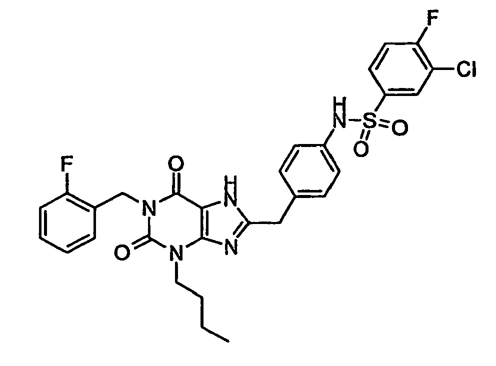

Prepared from 8-(4-amino-benzyl)-3-butyl-1-(2-fluoro-benzyl)-3,7-dihydro-purine-2,6-dione and 3-chloro-4-fluoro-benzenesulfonyl chloride. Purity (ELSD, based on MW = 614.1) = 95%.

### Example 76

### N-{4- [3-Butyl- 1-(2-fluoro-benzyl)-2,6-dioxo-2,3,6,7-tetrahydro-1H-purin-8-ylmethyl]-phenyl}-2,6-difluoro-benzenesulfonamide

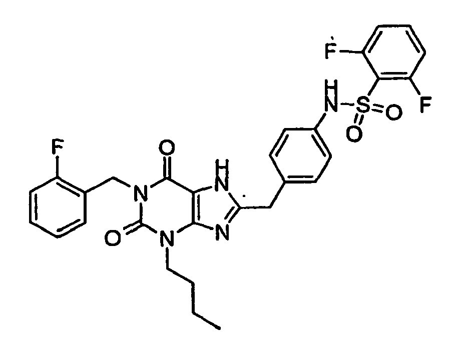

Prepared from 8-(4-amino-benzyl)-3-butyl-1-(2-fluoro-benzyl)-3,7-dihydro-purine-2,6-dione and 2,6-difluoro-benzenesulfonyl chloride. Purity (ELSD, based on MW = 597.6) = 80%.

### Example 77

### N-{4-[3-Butyl-(2-fluoro-benzyl)-2,6-dioxo-2,3,6,7-tetrahydro-1H-purin-8-ylmethyl]-phenyl}-3,4-difluoro-benzenesulfonamide

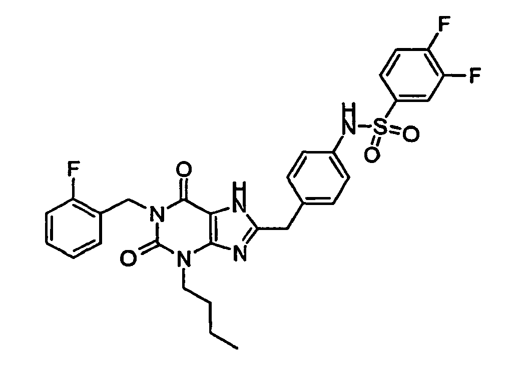

Prepared from 8-(4-amino-benzyl)-3-butyl-1-(2-fluoro-benzyl)-3,7-dihydro-purine-2,6-dione and 3,4-difluoro-benzenesulfonyl chloride. Purity (ELSD, based on MW = 597.6) = 95%.

### Example 78

### N-{4-[3-Butyl-1-(2-fluoro-benzyl)-2,6-dioxo-2,3,6,7-tetrahydro-1H-purin-8-ylmethyl]-phenyl}-2,4,6-trimethyl-benzenesulfonamide

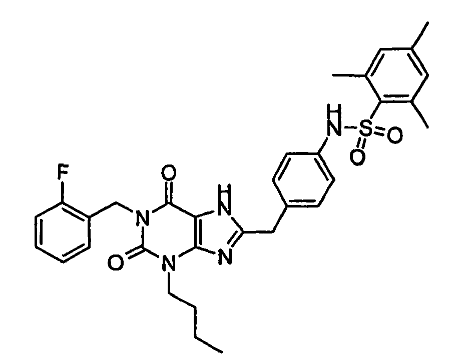

Prepared from 8-(4-amino-benzyl)-3-butyl-1-(2-fluoro-benzyl)-3,7-dihydro-purine-2,6-dione and 2,4,6-trimethyl-benzenesulfonyl chloride. Purity (ELSD, based on MW = 603.7) = 60%.

### Example 79

### N-{4- [3-Butyl-1-(2-fluoro-benzyl)-2,6-dioxo-2,3,6, 7-tetrahydro-1H-purin-8-ylmethyl]-phenyl}-4-chloro-2,5-dimethyl-benzenesulfonamide

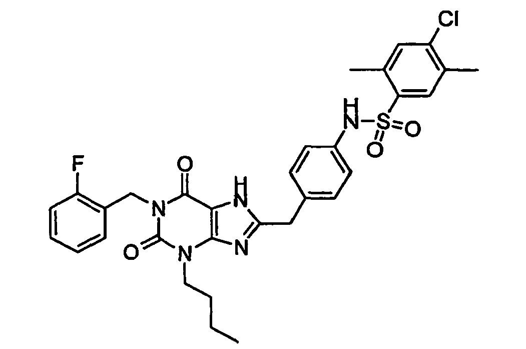

Prepared from 8-(4-amino-benzyl)-3-butyl-1-(2-fluoro-benzyl)-3,7-dihydro-purine-2,6-dione and 4-chloro-2,5-dimethyl-benzenesulfonyl chloride. Purity (ELSD, based on MW = 624.1) = 93%.

### Example 80

### N-{4-[3-Butyl-1-(2-fluoro-benzyl)-2,6-dioxo-2,3,6,7-tetrahydro-1H-purin-8-ylmethyl]-phenyl}-2,4-dichloro-6-methyl-benzenesulfonamide

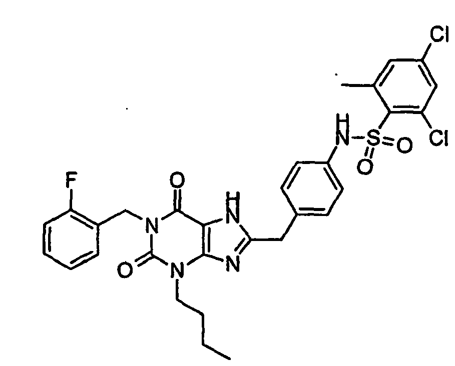

Prepared from 8-(4-amino-benzyl)-3-butyl-1-(2-fluoro-benzyl)-3,7-dihydro-purine-2,6-dione and 2,4-dichloro-6-methyl-benzenesulfonyl chloride. Purity (ELSD, based on MW = 644.6) = 85%.

### Example 81

### N-{4-[3-Butyl-1-(2-fluoro-benzyl)-2,6-dioxo-2,3,6,7-tetrahydro-1H-purin-8-ylmethyl]-phenyl}-2,4-dichloro-5-methyl-benzenesulfonamide

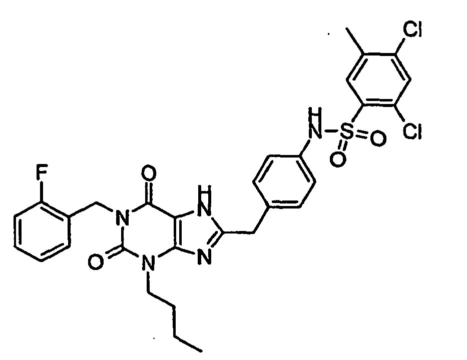

Prepared from 8-(4-amino-benzyl)-3-butyl-1-(2-fluoro-benzyl)-3,7-dihydro-purine-2,6-dione and 2,4-dichloro-5-methyl-benzenesulfonyl chloride. Purity (ELSD, based on MW = 644.6) = 82%.

### Example 82

### N-{4-[3-Butyl-1-(2-fluoro-benzyl)-2,6-dioxo-2,3,6,7-tetrahydro-1H-purin-8-ylmethyl]-phenyl}-2,3,5,6-tetramethyl-benzenesulfonamide

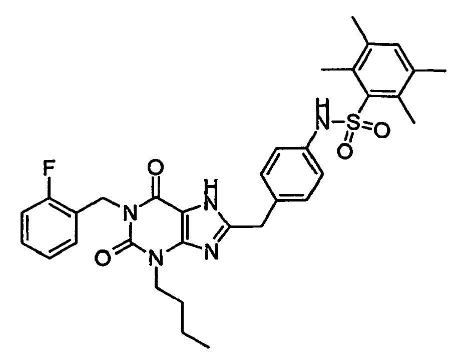

Prepared from 8-(4-amino-benzyl)-3-butyl-1-(2-fluoro-benzyl)-3,7-dihydro-purine-2,6-dione and 2,3,5,6-tetramethyl-benzenesulfonyl chloride. Purity (ELSD, based on MW = 617.7) = 80%.

### Example 83

### N-{4-[3-Butyl-1-(2-fluoro-benzyl)-2,6-dioxo-2,3,6,7-tetrahydro-1H-purin-8-ylmethyl]-phenyl}-4-methoxy 2,3,6-trimethyl-benzenesulfonamide

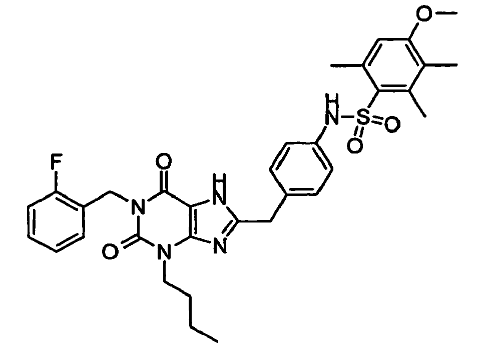

Prepared from 8-(4-amino-benzyl)-3-butyl-1-(2-fluoro-benzyl)-3,7-dihydro-purine-2,6-dione and 4-methoxy-2,3,6-trimethyl-benzenesulfonyl chloride. Purity (ELSD, based on MW = 633.7) = 80%.

### Example 84

### N-{4-[3-Butyl-1-(2-fluoro-benzyl)-2,6-dioxo-2,3,6,7-tetrahydro-1H-purin-8-ylmethyl]-phenyl}-1-naphthalenesulfonamide

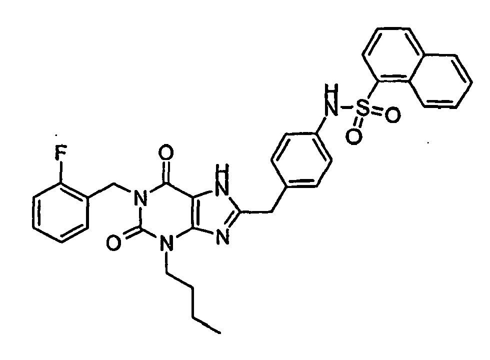

Prepared from 8-(4-amino-benzyl)-3-butyl-1-(2-fluoro-benzyl)-3,7-dihydro-purine-2,6-dione and naphthalene-1-sulfonyl chloride. Purity (ELSD, based on MW = 611.7) = 81%.

### Example 85

### Dimethylamino-naphthalene-1-sulfonic acid {4-[3-butyl-1-(2-fluoro-benzyl)-2,6-dioxo-2,3,6,7-tetrahydro-1H-purin-8-ylmethyl]-phenyl}-amide

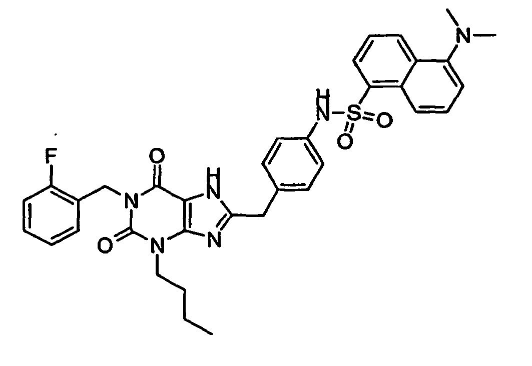

Prepared from 8-(4-amino-benzyl)-3-butyl-1-(2-fluoro-benzyl)-3,7-dihydro-purine-2,6-dione and 5-dimethylamino-1-naphthalenesulfonyl chloride. Purity (ELSD, based on MW = 654.8) = 90%.

### Example 86

### Benzo [1,2,5] oxadiazole-4-sulfonic acid {4-[3-butyl-1-(2-fluoro-benzyl)-2,6-dioxo-2,3,6,7-tetrahydro-1H-purin-8-ylmethyl]-phenyl}-amide

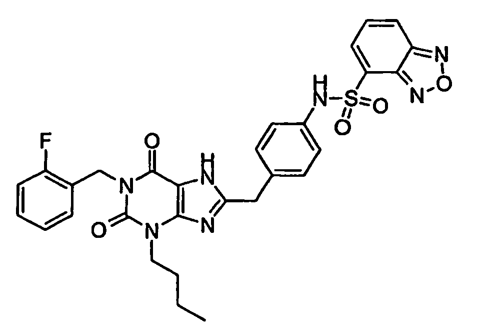

Prepared from 8-(4-amino-benzyl)-3-butyl-1-(2-fluoro-benzyl)-3,7-dihydro-purine-2,6-dione and benzo[1,2,5]oxadiazole-4-sulfonyl chloride. Purity (ELSD, based on MW = 603.6) = 95%.

### Example 87

### Benzo[1,2,5]thiadiazole-4-sulfonic acid {4-[3-butyl-1-(2-fluoro-benzyl)-2,6-dioxo-2,3,6,7-tetrahydro-1H-purin-8-ylmethyl]-phenyl}-amide

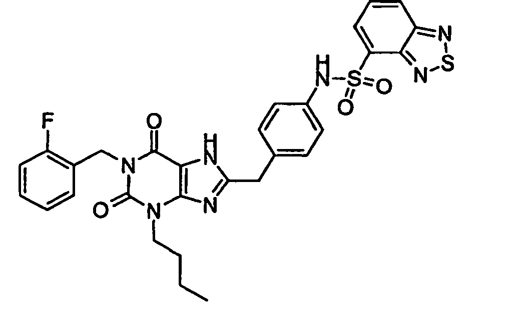

Prepared from 8-(4-amino-benzyl)-3-butyl-1-(2-fluoro-benzyl)-3,7-dihydro-purine-2,6-dione and benzo[1,2,5]thiadiazole-4-sulfonyl chloride. Purity (ELSD, based on MW = 619.7) = 80%.

### Example 88

### Chloro-pyridine-3-sulfonic acid {4-[3-butyl-1-(2-fluoro-benzyl)-2,6-dioxo-2,3,6,7-tetrahydro-1H-purin-8-ylmethyl] -phenyl}-amide

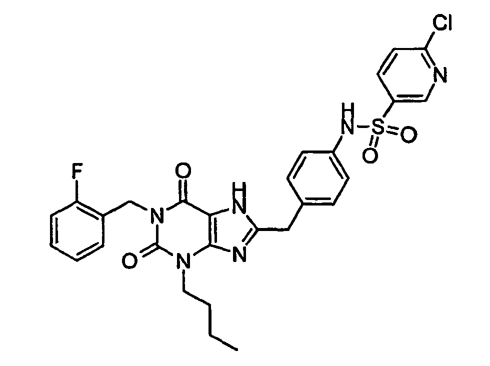

Prepared from 8-(4-amino-benzyl)-3-butyl-1-(2-fluoro-benzyl)-3,7-dihydro-purine-2,6-dione and 6-chloro-pyridine-3-sulfonyl chloride. Purity (ELSD, based on MW = 597.1) = 98%.

### Example 89

### Thiophene-2-sulfonic acid {4-[3-butyl-1-(2-fluoro-benzyl)-2,6-dioxo-2,3,6,7-tetrahydro-1H-purin-8-ylmethyl]-phenyl}-amide

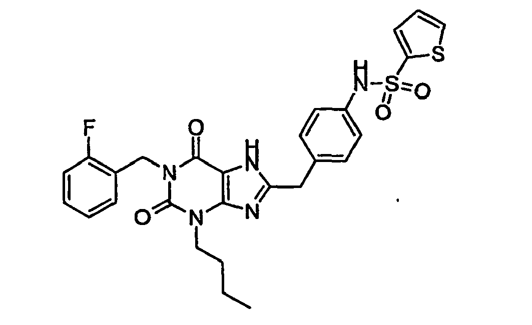

Prepared from 8-(4-amino-benzyl)-3-butyl-1-(2-fluoro-benzyl)-3,7-dihydro-purine-2,6-dione and 2-thio-benzenesulfonyl chloride. Purity (ELSD, based on MW = 567.7) = 67%.

### Example 90

### Chlorothiophene-2-sulfonic acid {4-[3-butyl-1-(2-fluoro-benzyl)-2,6-dioxo-2,3,6,7-tetrahydro-1H-purin-8-ylmethyl]-phenyl}-amide

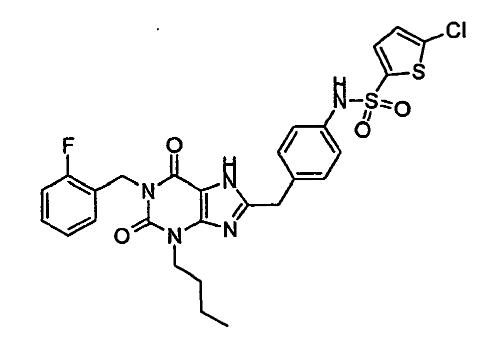

Prepared from 8-(4-amino-benzyl)-3-butyl-1-(2-fluoro-benzyl)-3,7-dihydro-purine-2,6-dione and 5-chloro-2-thio-benzenesulfonyl chloride. Purity (ELSD, based on MW = 602.1) = 96%.

### Example 91

### S-Bromo-thiophene-2-sulfonic acid {4-[3-butyl-1-(2-fluoro-benzyl)-2,6-dioxo-2,3,6,7-tetrahydro-1H-purin-8-ylmethyl]-phenyl}-amide

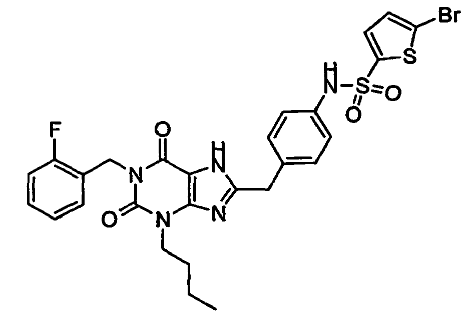

Prepared from 8-(4-amino-benzyl)-3-butyl-1-(2-fluoro-benzyl)-3,7-dihydro-purine-2,6-dione and 5-bromo-2-thio-benzenesulfonyl chloride. Purity (ELSD, based on MW = 646.6) = 92%.

### Example 92

### 4,5-Dibromothiophene-2-sulfonic acid {4-[3-butyl-1-(2-fluoro-benzyl)-2,6-dioxo-2,3,6,7-tetrahydro-1H-purin-8-ylmethyl]-phenyl}-amide

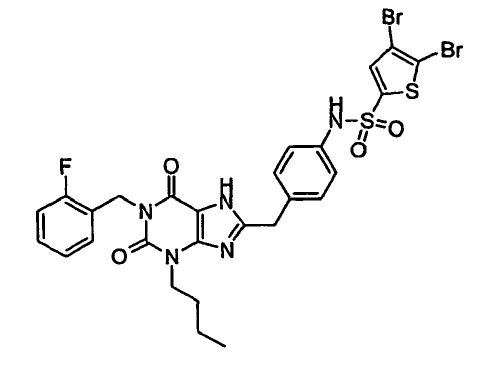

Prepared from 8-(4-amino-benzyl)-3-butyl-1-(2-fluoro-benzyl)-3,7-dihydro-purine-2,6-dione and 4,5-dibromo-2-thio-benzenesulfonyl chloride. Purity (ELSD, based on MW = 725.5) = 97%.

### Example 93

### 3-{4-[3-Butyl-1-(2-fluoro-benzyl)-2,6-dioxo-2,3,6,7-tetrahydro-1H-purin-8-ylmethyl]-phenylsulfamoyl}-thiophene-2-carboxylic acid methyl ester

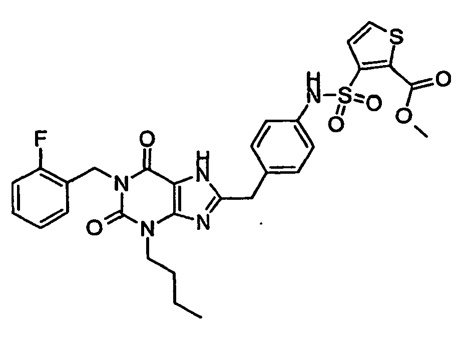

Prepared from 8-(4-amino-benzyl)-3-butyl-1-(2-fluoro-benzyl)-3,7-dihydro-purine-2,6-dione and 3-chlorosulfonyl-thiophene-2-carboxylic acid methyl ester. Purity (ELSD, based on MW = 625.7) = 92%.

### Example 94

### 4-(1-Benzyl-3-butyl-2,6-dioxo-2,3,6,7-tetrahydro-1H-purin-8-ylmethyl)-benzenesulfonamide.

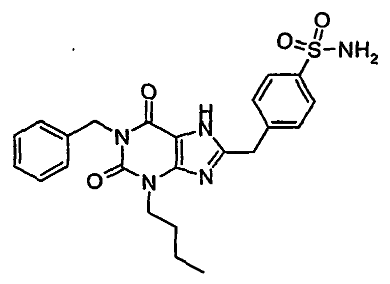

Prepared by the same method as described for 5-chloro-1,3-dimethyl-1H-pyrazole-4-sulfonic acid {4-[3-butyl-1-(2-fluoro-benzyl)-2,6-dioxo-2,3,6,7-tetrahydro-1H-purin-8-ylmethyl]-phenyl}-amide (example 34) except that in step 2 benzyl bromide was used in place of 2-fluorobenzyl bromide and in step 3 4-(2-amino-ethyl)-benzenesulfonamide was used in place of 2-(4-nitro-phenyl)-ethylamine hydrochloride salt. 4-(1-Benzyl-3-butyl-2,6-dioxo-2,3,6,7-tetrahydro-1H-purin-8-ylmethyl)-benzenesulfonamide was obtained as a colorless solid; LRMS for C₂₃H₂₅N₅O₄S (M+H)⁺ at m/z = 468.27.

The in vitro biological activity of several representative preferred compounds of the present invention in the foregoing PEPCK enzymatic assay as represented by in vitro potency is presented in Table 1 below.

**Table 1**

| Compound | IC₅₀ (µM) |
|---|---|
| 1,3-Dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 7) | 0.15 |
| *N*-(5-{4-[3-Cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide (example 10) | 0.22 |
| 2-Amino-4-methyl-thiazole-5-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt (example 11) | 0.23 |
| 5-Chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 1) | 0.29 |
| 1,2-Dimethyl-1H-imidazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 13) | 0.34 |
| 1- Methyl-1H-pyrazole-4-sulfonic acid {4- [3-cyclopropylmethyl-1-(2-fluoro-benzyl)-2,6-dioxo-2,3,6,7-tetrahydro-1H-purin-8-ylmethyl]-phenyl}-amide (example 2) | 0.39 |
| 1-Methyl-1H-imidazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 14) | 0.41 |
| Quinoline-8-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 19) | 0.45 |
| 3,5-Dimethyl-isoxazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 12) | 0.79 |
| 5-Chloro-1,3-dimethyl-1H-pyrazole-4-sulfonic acid {6-[3-butyl-1-(2-fluoro-benzyl)-2,6-dioxo-2,3,6,7-tetrahydro-1H-purin-8-ylmethyl]-pyridin-3-yl}-amide (example 25) | 0.88 |
| *N*-{4-[3-Cyclopropylmethyl-1-(2-fluoro-benzyl)-2,6-dioxo-2,3,6,7-tetrahydro-1H-purin-8-ylmethyl]-phenyl}-*N*,*N*-dimethylsulfamide (example 17) | 0.96 |
| 5-Chloro-1,3-dimethyl-1H-pyrazole-4-sulfonic acid {6-[3-cyclobutylmethyl-1-(2-fluoro-benzyl)-2,6-dioxo-2,3,6,7-tetrahydro-1H-purin-8-ylmethyl]-pyridin-3-yl}-amide (example 26) | 0.98 |
| 5-Chloro-1,3-dimethyl-1H-pyrazole-4-sulfonic acid {3-[3-cyclopropylmethyl-1-(2-fluoro-benzyl)-2,6-dioxo-2,3,6,7-tetrahydro-1H-purin-8-ylmethyl]-phenyl}-amide (example 6) | 1.1 |
| *N*-{4-[3-Cyclopropylmethyl-1-(2-fluoro-benzyl)-2,6-dioxo-2,3,6,7-tetrahydro-1H-purin-8-ylmethyl]-phenyl}-3-methoxybenzenesulfonamide (example 3) | 1.2 |
| *N*-{4-[3-Cyclopropylmethyl-1-(2-fluoro-benzyl)-2,6-dioxo-2,3,6,7-tetrahydro-1H-purin-8-ylmethyl] -phenyl}-methanesulfonamide (example 23) | 1.25 |
| 1,3-Dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide (example 27) | 1.45 |
| Pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt (example 22) | 1.95 |
| 5-Chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide (example 18) | 2.05 |
| 4-[3-Butyl-1-(2-fluoro-benzyl)-2,6-dioxo-2,3,6,7-tetrahydro-1H-purin-8-ylmethyl]-*N*-pyridin-2-yl-benzenesulfonamide (example 28) | 2.4 |
| 4-[3-Butyl-1-(2-fluoro-benzyl)-2,6-dioxo-2,3,6,7-tetrahydro-1H-purin-8-ylmethyl]-N-pyridin-4-yl-benzenesulfonamide (example 29) | 2.4 |
| *N*-{4-[3-Cyclopropylmethyl-1-(2-fluoro-benzyl)-2,6-dioxo-2,3,6,7-tetrahydro-1H-purin-8-ylmethyl]-phenyl}-3-fluoro-benzenesulfonamide (example 4) | 2.55 |
| 4-[3-Butyl-1-(2-fluoro-benzyl)-2,6-dioxo-2,3,6,7-tetrahydro-1H-purin-8-ylmethyl]-*N*-pyridin-3-yl-benzenesulfonamide (example 30) | 4.1 |
| 5-Bromo-6-chloro-pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide (example 5) | 4.58 |
| *N*-(4-{4-[3-Cyclopropylmethyl-1-(2-fluoro-benzyl)-2,6-dioxo-2,3,6,7-tetrahydro-1H-purin-8-ylmethyl] -phenylsulfamoyl}-phenyl)-acetamide (example 20) | 6.25 |
| 4-[3-Butyl-1-(2-fluoro-benzyl)-2,6-dioxo-2,3,6,7-tetrahydro-1H-purin-8-ylmethyl]-*N*-pyrimidin-2-yl-benzenesulfonamide (example 31) | 6.35 |
| 4-[3-Butyl-1-(2-fluoro-benzyl)-2,6-dioxo-2,3,6,7-tetrahydro-1H-purin-8-ylmethyl]-*N*-(1,3,5-trimethyl-1H-pyrazol-4-yl)-benzenesulfonamide (example 32) | 6.45 |
| *N-*[4-(1- Benzyl-3-butyl-2,6-dioxo-2,3,6,7-tetrahydro-1H-purin-8-ylmethyl)-phenyl]-methanesulfonamide (example 24) | 7.55 |
| *N*-[4-(1-Benzyl-3-butyl-2,6-dioxo-2,3,6,7-tetrahydro-1H-purin-8-ylmethyl)-phenyl]-4-methyl-benzenesulfonamide (example 21) | 7.65 |

The therapeutically effective amount of a compound in accordance with this invention can vary within wide limits and may be determined by a manner known in the art. Such dosage will be adjusted to the individual requirements in each particular case including the specific compound or compounds being administered, the route of administration, the condition being treated, as well as the patient being treated. In general, in the case of parenteral administration to adult humans weighing approximately 70 Kg, a daily dosage of about 10 mg to about 10,000 mg, preferably from about 200mg to about 1,000 mg should be appropriate, although the upper limit may be exceeded when indicated.

The daily dosage can be administered as a single dose or in divided doses, or for parental administration, it may be given as continuous infusion. Additionally, administration in the form of an elixir, tablet, capsule or suppository is envisioned and is within the scope of the present invention. The examples below are exemplary, but not limitative of, the invention.

### Example A

### Tablet Formulation

| Item | Ingredients | Mg/Tablet | | | | | |
|---|---|---|---|---|---|---|---|
| 1 | Compound A * | 5 | 25 | 100 | 250 | 500 | 750 |
| 2 | Anhydrous Lactose | 103 | 83 | 35 | 19 | 38 | 57 |
| 3 | Croscarmellose Sodium | 6 | 6 | 8 | 16 | 32 | 48 |
| 4 | Polyvinyl pyrrolidone K30 | 5 | 5 | 6 | 12 | 24 | 36 |
| 5 | Magnesium Stearate | 1 | 1 | 1 | 3 | 6 | 9 |
| | Total Weight | 120 | 120 | 150 | 300 | 600 | 900 |

| | | | | | | | |
|---|---|---|---|---|---|---|---|
| *Compound A represents a compound of the invention. | | | | | | | |

### Manufacturing Procedure:

Mix Items 1, 2 and 3 in a suitable mixer for 15 minutes.

Granulate the powder mix from Step 1 with 20% Polyvinyl pyrrolidone K30 Solution (Item 4).

Dry the granulation from Step 2 at 50°C.

Pass the granulation from Step 3 through a suitable milling equipment.

Add the Item 5 to the milled granulation Step 4 and mix for 3 minutes.

Compress the granulation from Step 5 on a suitable press.

### Example B

### Capsule Formulation

| Item | Ingredients | mg/Capsule | | | | |
|---|---|---|---|---|---|---|
| 1 | Compound A * | 5 | 25 | 100 | 250 | 500 |
| 2 | Anhydrous Lactose | 159 | 123 | 148 | -- | -- |
| 3 | Corn Starch | 25 | 35 | 40 | 35 | 70 |
| 4 | Talc | 10 | 15 | 10 | 12 | 24 |
| 5 | Magnesium Stearate | 1 | 2 | 2 | 3 | 6 |
| | Total Fill Weight | 200 | 200 | 300 | 300 | 600 |

| | | | | | | |
|---|---|---|---|---|---|---|
| *Compound A represents a compound of the invention. | | | | | | |

### Manufacturing Procedure:

Mix Items 1, 2 and 3 in a suitable mixer for 15 minutes.

Add Items 4 & 5 and mix for 3 minutes.

Fill into a suitable capsule.

### Example C

### Injection Solution/Emulsion Preparation

| Item | Ingredient | mg/mL |
|---|---|---|
| 1 | Compound A * | 1 mg |
| 2 | Polyethylene glycol (MW 400) | 10-50 mg |
| 3 | Lecithin | 20-50 mg |
| 4 | Soy Oil | 1-5 mg |
| 5 | Glycerol | 8-12 mg |
| 6 | Water q.s. | 1 mL |

| | | |
|---|---|---|
| *Compound A represents a compound of the invention. | | |

### Manufacturing Procedure:

Dissolve item 1 in item 2.

Add items 3, 4 and 5 to item 6 and mix until dispersed, then homogenize.

Add the solution from step 1 to the mixture from step 2 and homogenize until the dispersion is translucent.

Sterile filter through a 0.2 µm filter and fill into vials.

### Example D

### Injection Solution/Emulsion Preparation

| Item | Ingredient | mg/mL |
|---|---|---|
| 1 | Compound A * | 1 mg |
| 2 | Glycofurol | 10-50 mg |
| 3 | Lecithin | 20-50 mg |
| 4 | Soy Oil | 1-5 mg |
| 5 | Glycerol | 8-12 mg |
| 6 | Water | q.s. 1mL |

| | | |
|---|---|---|
| *Compound A represents a compound of the invention. | | |

## Claims

1. Compounds of formula 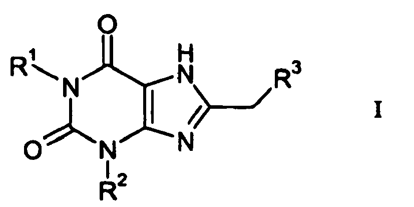 wherein
R¹ is selected from the group consisting of
C₁₋₇- alkyl,
C₁₋₇- alkyl substituted by phenyl and
C₁₋₇- alkyl substituted by halogen substituted phenyl;
R² is selected from the group consisting of
C₁₋₇- alkyl and
C₁₋₇- alkyl substituted by C₃₋₇- cycloalkyl;
R³ is selected from the group consisting of 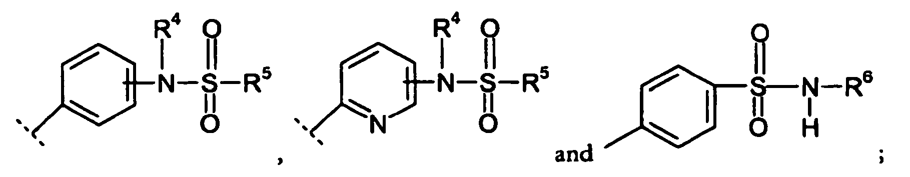
R⁴ is selected from the group consisting of H and C₁₋₇- alkyl;
R⁵ is selected from the group consisting of
C₁₋₇- alkyl,
amino C₁₋₇- alkyl,
C₁₋₇- alkyl substituted by phenyl,
C₂₋₇- alkenyl substituted by phenyl,
phenyl,
phenyl substituted by at least one substituent selected from the group consisting of C₁₋₇-alkyl, C₁₋₇-alkyl substituted by halogen, halogen, C₁₋₇-alkoxy,
C₁₋₇- alkoxy substituted by halogen, nitro and acetamido,
a 5-membered heteroaromatic ring having one heteroatom independently selected from the group consisting of N, O and S, the 5-membered heteroaromatic ring being unsubstituted or substituted by at least one substituent selected from the group consisting of C₁₋₇- alkyl, halogen, carboxy C₁₋₇- alkyl, amino, alkyl amino and acetamido,
a 5-membered heteroaromatic ring having two heteroatoms wherein a first heteroatom is N and a second heteroatom is independently selected from the group consisting of N, O and S, the 5-membered heteroaromatic ring being unsubstituted or substituted by at least one substituent selected from the group consisting of C₁₋₇-alkyl, halogen, carboxy C₁₋₇- alkyl, amino, alkyl amino and acetamido,
a 6-membered heteroaromatic ring having one N, the 6-membered heteroaromatic ring being unsubstituted or substituted by at least one substituent selected from the group consisting of C₁₋₇- alkyl, halogen,carboxy C₁₋₇- alkyl, amino, alkyl amino and acetamido, 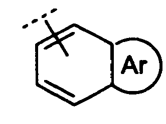 wherein Ar is selected from the group consisting of
a 5-membered heteroaromatic ring fused to the 6-membered ring, having one, two, or three heteroatoms, and wherein a first heteroatom is N, a second heteroatom is N and a third heteroatom is selected from the group consisting of O and S,
a 6-membered aromatic ring fused to the 6-membered ring, having no or one N heteroatoms, the fused 6-membered aromatic ring being unsubstituted or substituted by at least one substituent selected from the group consisting of C₁₋₇-alkyl, halogen, carboxy C₁₋₇- alkyl, amino, alkyl amino and acetamido; and
R⁶ is selected from the group consisting of H,
a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇- alkyl, halogen, carboxy C₁₋₇- alkyl, amino, alkyl amino and acetamido, and
a 6-membered aromatic heterocyclic ring with one or two N heteroatoms, the 6-membered heterocyclic ring being unsubstituted or substituted by at least one substituent selected from the group consisting of C₁₋₇- alkyl, halogen, carboxy C₁₋₇-alkyl, amino, alkyl amino and acetamido,
or a pharmaceutically acceptable salt thereof.

2. The compounds according to claim 1 of formula 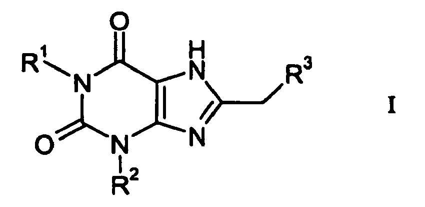 wherein
R¹ is selected from the group consisting of
C₁₋₇- alkyl,
C₁₋₇- alkyl substituted by phenyl and
C₁₋₇- alkyl substituted by halogen substituted phenyl;
R² is selected from the group consisting of
C₁₋₇- alkyl and
C₁₋₇- alkyl substituted by C₃₋₇- cycloalkyl;
R³ is selected from the group consisting of 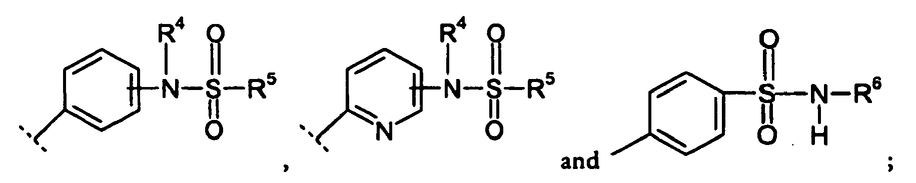
R⁴ is selected from the group consisting of H and C₁₋₇- alkyl;
R⁵ is selected from the group consisting of
C₁₋₇-alkyl,
amino C₁₋₇- alkyl,
C₁₋₇- alkyl substituted by phenyl,
C₂₋₇- alkenyl substituted by phenyl,
phenyl,
phenyl substituted by at least one substituent selected from the group consisting of C₁₋₇- alkyl, C₁₋₇- alkyl substituted by halogen, halogen, C₁₋₇- alkoxy,
C₁₋₇- alkoxy substituted by halogen, nitro and acetamido,
a 5-membered heteroaromatic ring having one heteroatom independently selected from the group consisting of N, O and S, the 5-membered heteroaromatic ring being unsubstituted or substituted by at least one substituent selected from the group consisting of C₁₋₇- alkyl, halogen, carboxy C₁₋₇- alkyl, amino, alkyl amino and acetamido,
a 5-membered heteroaromatic ring having two heteroatoms wherein a first heteroatom is N and a second heteroatom is independently selected from the group consisting of N, O and S, the 5-membered heteroaromatic ring being unsubstituted or substituted by at least one substituent selected from the group consisting of C₁₋₇-alkyl, halogen, carboxy C₁₋₇- alkyl, amino, alkyl amino and acetamido,
a 6-membered heteroaromatic ring having one N, the 6-membered heteroaromatic ring being unsubstituted or substituted by at least one substituent selected from the group consisting of C₁₋₇- alkyl, halogen,carboxy C₁₋₇- alkyl, amino, alkyl amino and acetamido, 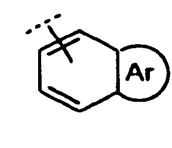 wherein Ar is selected from the group consisting of a 5-membered heteroaromatic ring fused to the 6-membered ring, having one, two, or three heteroatoms, and wherein a first heteroatom is N, a second heteroatom is N and a third heteroatom is selected from the group consisting of O and S,
a 6-membered aromatic ring fused to the 6-membered ring, having no or one N heteroatoms, the fused 6-membered aromatic ring being unsubstituted or substituted by at least one substituent selected from the group consisting of C₁₋₇-alkyl, halogen, carboxy C₁₋₇- alkyl, amino, alkyl amino and acetamido; and
R⁶ is selected from the group consisting of H,
a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇- alkyl, halogen, carboxy C₁₋₇- alkyl, amino, alkyl amino and acetamido, and
a 6-membered aromatic heterocyclic ring with one or two N heteroatoms, the 6-membered heterocyclic ring being unsubstituted or substituted by at least one substituent selected from the group consisting of C₁₋₇- alkyl, halogen, carboxy C₁₋₇-alkyl, amino, alkyl amino and acetamido,
or a pharmaceutically acceptable salts thereof.

3. Compounds of formula I according to claim 1 or 2 wherein R¹ is C₁₋₇- alkyl.

4. Compounds of formula I according to claim 1 or 2 wherein R¹ is C₁₋₇- alkyl substituted by phenyl.

5. Compounds of formula I according to any one of claims 1 to 4 wherein R² is C₁₋₇- alkyl.

6. Compounds of formula I according to claim 1 having the formula

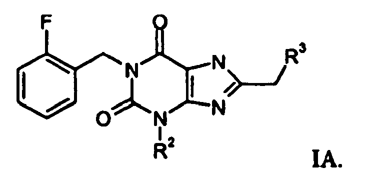

7. Compounds of formula IA according to claim 6 wherein R² is C₁₋₇- alkyl.

8. Compounds of formula IA according to claim 7 wherein R² is n-butyl.

9. Compounds of formula IA according to claim 6 wherein R³ is

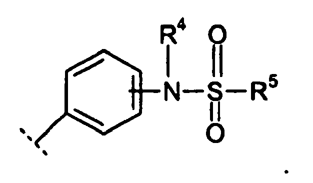

10. Compounds of formula IA according to claim 9 wherein R⁴ is H.

11. Compounds of formula IA according to claim 9 wherein R⁵ is a 5-membered heteroaromatic ring having one heteroatom independently selected from the group consisting of N, O and S, the 5-membered heteroaromatic ring being unsubstituted or substituted by at least one substituent selected from the group consisting of C₁₋₇- alkyl, halogen, carboxy C₁₋₇- alkyl, amino, alkyl amino and acetamido.

12. Compounds of formula IA according to claim 9 wherein R⁵ is a 5-membered heteroaromatic ring having two heteroatoms wherein a first heteroatom is N and a second heteroatom is independently selected from the group consisting of N, O and S, the 5-membered heteroaromatic ring being unsubstituted or substituted by at least one substituent selected from the group consisting of C₁₋₇- alkyl, halogen, carboxy C₁₋₇- alkyl, amino, alkyl amino and acetamido.

13. Compounds of formula IA according to claim 9 wherein R⁵ is a 6-membered heteroaromatic ring having one N, the 6-membered heteroaromatic ring being unsubstituted or substituted by at least one substituent selected from the group consisting of C₁₋₇- alkyl, halogen, carboxy C₁₋₇- alkyl, amino, alkyl amino and acetamido.

14. Compounds of formula IA according to claim 9 wherein R⁵ is 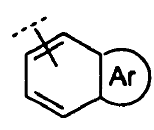 where Ar is selected from the group consisting of
a 5-membered heteroaromatic ring fused to the 6-membered ring, having one, two, or three heteroatoms, and wherein a first heteroatom is N, a second heteroatom is N and a third heteroatom is selected from the group consisting of O and S, and
a 6-membered aromatic ring fused to the 6-membered ring, having no or one N heteroatoms, the fused 6-membered aromatic ring being unsubstituted or substituted by at least one substituent selected from the group consisting of C₁₋₇- alkyl, halogen, carboxy C₁₋₇- alkyl, amino, alkyl amino and acetamido.

15. Compounds of formula IA according to claim 9 wherein R⁵ is C₁₋₇-alkyl or C₁₋₇- alkyl substituted by phenyl.

16. Compounds of formula IA according to claim 9 wherein R⁵ is C₂₋₇- alkenyl substituted by phenyl.

17. Compounds of formula IA according to claim 9 wherein R⁵ is phenyl.

18. Compounds of formula IA according to claim 9 wherein R⁵ is phenyl substituted by at least one substituent selected from the group consisting of C₁₋₇- alkyl, C₁₋₇- alkyl substituted by halogen, halogen, C₁₋₇- alkoxy, C₁₋₇- alkoxy substituted by halogen, nitro and acetamido.

19. Compounds of formula IA according to claim 18 wherein R⁵ is phenyl substituted by halogen.

20. Compounds of formula IA according to claim 18 wherein R⁵ is phenyl substituted by C₁₋₇- alkyl or C₁₋₇-alkyl substituted by halogen.

21. Compounds of formula IA according to claim 18 wherein the phenyl group has two or three substituents selected from the group consisting of C₁₋₇- alkyl, C₁₋₇- alkyl substituted by halogen, halogen, C₁₋₇- alkoxy, C₁₋₇- alkoxy substituted by halogen, nitro and acetamido.

22. Compounds of formula IA according to claim 6 wherein R³ is

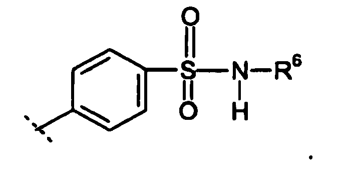

23. Compounds of formula IA according to claim 22 wherein R⁶ is a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇- alkyl, halogen, carboxy C₁₋₇- alkyl, amino, alkyl amino and acetamido.

24. Compounds of formula IA according to claim 22 wherein R⁶ is a 6-membered aromatic heterocyclic ring with one or two N heteroatoms, the 6-membered heterocyclic ring being unsubstituted or substituted by at least one substituent selected from the group consisting of C₁₋₇- alkyl, halogen, carboxy C₁₋₇- alkyl, amino, alkyl amino and acetamido.

25. Compounds of formula IA according to claim 6 wherein R³ is

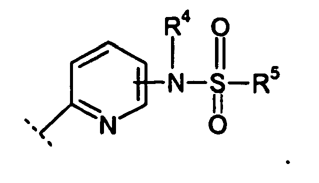

26. Compounds of formula IA according to claim 25 wherein R⁴ is H, and R⁵ is a 5-membered aromatic heterocyclic ring with one or two heteroatoms wherein a first heteroatom is N and a second heteroatom is selected from the group consisting of N and S, the 5-membered heterocyclic ring being unsubstituted or substituted by at least one substituent selected from the group consisting of C₁₋₇- alkyl, halogen, carboxy C₁₋₇- alkyl, amino, alkyl amino and acetamido.

27. Compounds of formula IA according to claim 6 wherein R² is C₁₋₇- alkyl substituted by cycloalkyl.

28. Compounds of formula I according to claim 8 with the formula

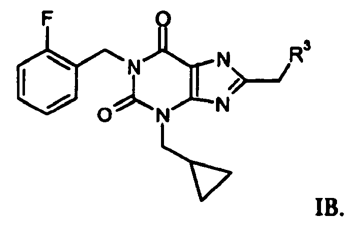

29. Compounds of formula IB according to claim 28 wherein R³ is

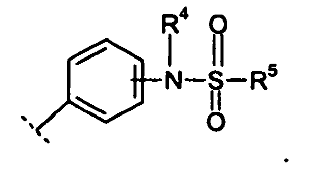

30. Compounds of formula IB according to claim 29 wherein R⁴ is H.

31. Compounds of formula IB according to claim 29 wherein R⁵ is a 5-membered heteroaromatic ring having two heteroatoms wherein a first heteroatom is N and a second heteroatom is independently selected from the group consisting of N, O and S, the 5-membered heteroaromatic ring being unsubstituted or substituted by at least one substituent selected from the group consisting of C₁₋₇- alkyl, halogen, carboxy C₁₋₇-alkyl, amino, alkyl amino and acetamido.

32. Compounds of formula IB according to claim 29 wherein R⁵ is phenyl substituted by at least one substituent selected from the group consisting of C₁₋₇- alkyl, C₁₋₇- alkyl substituted by halogen, halogen, C₁₋₇- alkoxy, C₁₋₇- alkoxy substituted by halogen, nitro and acetamido.

33. Compounds of formula IB according to claim 29 wherein R⁵ is a 6-membered heteroaromatic ring having one N, the 6-membered heteroaromatic ring being unsubstituted or substituted by at least one substituent selected from the group consisting of C₁₋₇- alkyl, halogen, carboxy C₁₋₇- alkyl, amino, alkyl amino and acetamido.

34. Compounds of formula IB according to claim 29 wherein R⁵ is C₁₋₇- alkyl.

35. Compounds of formula IB according to claim 29 wherein R⁴ is C₁₋₇- alkyl.

36. Compounds of formula I according to claim 1 or 2 wherein the compound is selected from the group consisting of
5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
1-methyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide
*N*-{4-[3-cyclopropylmethyl-1-(2-fluoro-benzyl)-2,6-dioxo-2,3,6,7-tetrahydro-1H-purin-8-ylmethyl] -phenyl} -3-methoxy-benzenesulfonamide,
*N*-{4-[3-cyclopropylmethyl-1-(2-fluoro-benzyl)-2,6-dioxo-2,3,6,7-tetrahydro-1H-purin-8-ylmethyl]-phenyl}-3-fluoro-benzenesulfonamide,
5-bromo-6-chloro-pyridine-3-sulfonic acid {4-[3-cyclopropylmethyl-1-(2-fluorobenzyl)-2,6-dioxo-2,3,6,7-tetrahydro-1H-purin-8-ylmethyl]-phenyl}-amide,
5-chloro-1,3-dimethyl-1H-pyrazole-4-sulfonic acid {3-[3-cyclopropylmethyl-1-(2-fluoro-benzyl)-2,6-dioxo-2,3,6,7-tetrahydro-1H-purin-8-ylmethyl]-phenyl}-amide,
1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
*N*-(5-{4-[3-cyclopropylmethyl-1-(2-fluoro-benzyl)-2,6-dioxo-2,3,6,7-tetrahydro-1H-purin-8-ylmethyl]-phenylsulfamoyl}-4-methyl-thiazol-2-yl)-acetamide,
2-amino-4-methyl-thiazole-5-sulfonic acid {4-[3-cyclopropylmethyl-1-(2-fluorobenzyl)-2,6-dioxo-2,3,6,7-tetrahydro-1H-purin-8-ylmethyl] -phenyl}-amide trifluoro-acetic acid salt,
3,5-dimethyl-isoxazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
1,2-dimethyl-1H-imidazole-4-sulfonic acid {4-[3-cyclopropylmethyl-1-(2-fluorobenzyl)-2,6-dioxo-2,3,6,7-tetrahydro-1H-purin-8-ylmethyl]-phenyl}-amide,
1-methyl-1H-imidazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
*N*-{4-[3-cyclopropylmethyl-1-(2-fluoro-benzyl)-2,6-dioxo-2,3,6,7-tetrahydro-1H-purin-8-ylmethyl]-phenyl}-*N,N*-dimethylsulfamide,
5-chloro-1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methylamide,
quinoline-8-sulfonic acid {4-[3-cyclopropylmethyl-1-(2-fluoro-benzyl)-2,6-dioxo-2,3,6,7-tetrahydro-1H-purin-8-ylmethyl]-phenyl}-amide,
*N*-(4-{4-[3-cyclopropylmethyl-1-(2-fluoro-benzyl)-2,6-dioxo-2,3,6,7-tetrahydro-1H-purin-8-ylmethyl]-phenylsulfamoyl}-phenyl)-acetamide,
*N*-[4-(1-benzyl-3-butyl-2,6-dioxo-2,3,6,7-tetrahydro-1H-purin-8-ylmethyl)-phenyl]-4-methyl-benzenesulfonamide,
pyridine-3-sulfonic acid {4-[3-cyclopropylmethyl-1-(2-fluoro-benzyl)-2,6-dioxo-2,3,6,7-tetrahydro-1H-purin-8-ylmethyl]-phenyl}-amide trifluoro-acetic acid salt,
*N*-{4-[3-cydopropylmethyl-1-(2-fluoro-benzyl)-2,6-dioxo-2,3,6,7-tetrahydro-1H-purin-8-ylmethyl]-phenyl}-methanesulfonamide,
*N*-[4-(1-benzyl-3-butyl-2,6-dioxo-2,3,6,7-tetrahydro-1H-purin-8-ylmethyl)-phenyl]-methanesulfonamide,
5-chloro-1,3-dimethyl-1H-pyrazole-4-sulfonic acid {6-[3-butyl-1-(2-fluoro-benzyl)-2,6-dioxo-2,3,6,7-tetrahydro-1H-purin-8-ylmethyl]-pyridin-3-yl}-amide,
5-chloro-1,3-dimethyl-1H-pyrazole-4-sulfonic acid {6-[3-cyclobutylmethyl-1-(2-fluorobenzyl)-2,6-dioxo-2,3,6,7-tetrahydro- 1H-purin-8-ylmethyl] -pyridin-3-yl}-amide,
1,3-dimethyl-1H-pyrazole-4-sulfonic acid {4-[3-cyclopropylmethyl-1-(2-fluoro-benzyl)-2,6-dioxo-2,3,6,7-tetrahydro-1H-purin-8-ylmethyl]-phenyl}-methyl-amide,
4-[3-butyl-1-(2-fluoro-benzyl)-2,6-dioxo-2,3,6,7-tetrahydro-1H-purin-8-ylmethyl]-*N-*pyridin-2-yl-benzenesulfonamide,
4-[3-butyl-1-(2-fluoro-benzyl)-2,6-dioxo-2,3,6,7-tetrahydro-1H-purin-8-ylmethyl]*-N-*pyridin-4-yl-benzenesulfonamide,
4- [3-butyl-1-(2-fluoro-benzyl)-2,6-dioxo-2,3,6,7-tetrahydro-1H-purin-8-ylmethyl*-N-*pyridin-3-yl-benzenesulfonamide,
4-[3-butyl-1-(2-fluoro-benzyl)-2,6-dioxo-2,3,6,7-tetrahydro-1H-purin-8-ylmethyl}-*N-*pyrimidin-2-yl-benzenesulfonamide, and
4-[3-butyl-1-(2-fluoro-benzyl)-2,6-dioxo-2,3,6,7-tetrahydro-1H-purin-8-ylmethyl]-*N-*(1,3,5-trimethyl-1H-pyrazol-4-yl)-benzenesulfonamide.

37. A process for the preparation of a compound according to any one of claims 1 to 36, which process comprises cyclisation of a compound of formula II 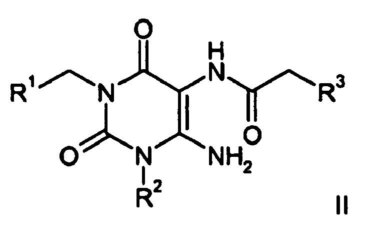 wherein R¹, R² and R³ are as defined in any one of claims 1 to 36, to yield the compound of formula I.

38. Compounds according to any one of claims 1 to 36, when manufactured by a process according to claim 37.

39. Pharmaceutical compositions comprising a compound according to any one of claims 1 to 36 or a pharmaceutically acceptable salt thereof, and a pharmaceutically acceptable excipient.

40. Compounds according to any one of claims 1 to 36 for use as therapeutic active substances.

41. The use of compounds according to any one of claims 1 to 36 for the preparation of medicaments for the treatment and/or prophylaxis of diseases which are associated with phosphoenolpyruvate carboxykinase.

42. The use according to claim 41 for the treatment of type 2 diabetes.

## Patentansprüche

1. Verbindungen der Formel 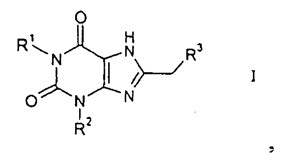 worin
R¹ aus der Gruppe ausgewählt ist, bestehend aus
C₁₋₇-Alkyl,
C₁₋₇-Alkyl, substituiert durch Phenyl, und
C₁₋₇-Alkyl, substituiert durch Halogen-substituiertes Phenyl;
R² aus der Gruppe ausgewählt ist, bestehend aus
C₁₋₇-Alkyl und
C₁₋₇-Alkyl, substituiert durch C₃₋₇-Cycloalkyl;
R³ aus der Gruppe ausgewählt ist, bestehend aus 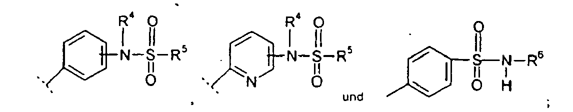
R⁴ aus der Gruppe ausgewählt ist, bestehend aus H und C₁₋₇-Alkyl;
R⁵ aus der Gruppe ausgewählt ist, bestehend aus
C₁₋₇-Alkyl,
Amino-C₁₋₇-alkyl,
C₁₋₇-Alkyl, substituiert durch Phenyl,
C₂₋₇-Alkenyl, substituiert durch Phenyl,
Phenyl,
Phenyl, substituiert durch mindestens einen Substituenten, ausgewählt aus der Gruppe, bestehend aus C₁₋₇-Alkyl, C₁₋₇-Alkyl, substituiert durch Halogen, Halogen, C₁₋₇-Alkoxy, C₁₋₇-Alkoxy, substituiert durch Halogen, Nitro und Acetamido,
einem 5gliedrigen heteroaromatischen Ring, der ein Heteroatom, unabhängig ausgewählt aus der Gruppe, bestehend aus N, O und S, aufweist, wobei der 5gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist,
einem 5gliedrigen heteroaromatischen Ring, der zwei Heteroatome ausweist, wobei ein erstes Heteroatom N ist und ein zweites Heteroatom unabhängig aus der Gruppe ausgewählt ist, bestehend aus N, O und S, wobei der 5gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist,
einem 6gliedrigen heteroaromatischen Ring, der ein N aufweist, wobei der 6gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist, 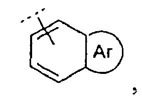 wobei Ar aus der Gruppe ausgewählt ist, bestehend aus einem 5gliedrigen heteroaromatischen Ring, anelliert an den 6gliedrigen Ring, der ein, zwei oder drei Heteroatome aufweist, und wobei ein erstes Heteroatom N ist, ein zweites Heteroatom N ist und ein drittes Heteroatom aus der Gruppe ausgewählt ist, bestehend aus O und S,
einem 6gliedrigen aromatischen Ring, anelliert an den 6gliedrigen Ring, der kein oder ein N-Heteroatom aufweist, wobei der anellierte 6gliedrige aromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist; und
R⁶ aus der Gruppe ausgewählt ist, bestehend aus H,
einem 5gliedrigen aromatischen heterocyclischen Ring mit einem oder zwei Heteroatomen, wobei ein erstes Heteroatom N ist und ein zweites Heteroatom aus der Gruppe ausgewählt ist, bestehend aus N und S, wobei der 5gliedrige heterocyclische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist, und
einem 6gliedrigen aromatischen heterocyclischen Ring mit einem oder zwei N-Heteroatomen, wobei der 6gliedrige heterocyclische Ring unsubstituiert oder durch mindestens einen Substituenten, ausgewählt aus der Gruppe, bestehend aus C₁₋₇-Alkyl, Halogen,
Carboxy-C₁₋₇-alkyl, Amino, Alkylamino und Acetamido, substituiert ist,
oder ein pharmazeutisch akzeptables Salz davon.

2. Verbindungen nach Anspruch 1 der Formel 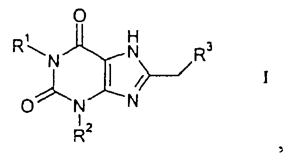 worin
R¹ aus der Gruppe ausgewählt ist, bestehend aus
C₁₋₇-Alkyl,
C₁₋₇-Alkyl, substituiert durch Phenyl, und
C₁₋₇-Alkyl, substituiert durch Halogen-substituiertes Phenyl;
R² aus der Gruppe ausgewählt ist, bestehend aus
C₁₋₇-Alkyl und
C₁₋₇-Alkyl, substituiert durch C₃₋₇-Cycloalkyl;
R³ aus der Gruppe ausgewählt ist, bestehend aus 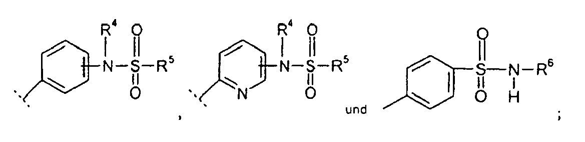
R⁴ aus der Gruppe ausgewählt ist, bestehend aus H und C₁₋₇-Alkyl-,
R⁵ aus der Gruppe ausgewählt ist, bestehend aus
C₁₋₇-Alkyl,
Amino-C₁₋₇-alkyl,
C₁₋₇-Alkyl, substituiert durch Phenyl,
C₁₋₇-Alkenyl, substituiert durch Phenyl,
Phenyl,
Phenyl, substituiert durch mindestens einen Substituenten, ausgewählt aus der Gruppe, bestehend aus C₁₋₇-Alkyl, C₁₋₇-Alkyl, substituiert durch Halogen, Halogen, C₁₋₇-Alkoxy, C₁₋₇-Alkoxy, substituiert durch Halogen, Nitro und Acetamido,
einem 5gliedrigen heteroaromatischen Ring, der ein Heteroatom, unabhängig ausgewählt aus der Gruppe, bestehend aus N, O und S, aufweist, wobei der 5gliedrige heteroaromatische Ring unsubstituiert oder durch mindestens einen Substituenten, ausgewählt aus der Gruppe, bestehend aus C₁₋₇-Alkyl, Halogen, Carbox-C₁₋₇-alkyl, Amino, Alkylamino und Acetamido, substituiert ist,
einem 5gliedrigen heteroaromatischen Ring, der zwei Heteroatome aufweist, wobei ein erstes Heteroatom N ist und ein zweites Heteroatom unabhängig aus der Gruppe ausgewählt ist, bestehend aus N, O und S, wobei der 5gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist,
einem 6gliedrigen heteroaromatischen Ring, der ein N aufweist, wobei der 6gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist, 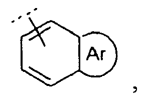 wobei Ar aus der Gruppe ausgewählt ist, bestehend aus einem 5gliedrigen heteroaromatischen Ring, anelliert an den 6gliedrigen Ring, der ein, zwei oder drei Heteroatome aufweist, und wobei ein erstes Heteroatom N ist, ein zweites Heteroatom N ist und ein drittes Heteroatom aus der Gruppe ausgewählt ist, bestehend aus O und S,
einem 6gliedrigen aromatischen Ring, anelliert an den 6gliedrigen Ring, der kein oder ein N-Heteroatom aufweist, wobei der anellierte 6gliedrige aromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist; und
R⁶ aus der Gruppe ausgewählt ist, bestehend aus H,
einem 5gliedrigen aromatischen heterocyclischen Ring mit einem oder zwei Heteroatomen, wobei ein erstes Heteroatom N ist und ein zweites Heteroatom aus der Gruppe ausgewählt ist, bestehend aus N und S, wobei der 5gliedrige heterocyclische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist, und
einem 6gliedrigen aromatischen heterocyclischen Ring mit einem oder zwei N-Heteroatomen, wobei der 6gliedrige heterocyclische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist,
oder pharmazeutisch akzeptable Salze davon.

3. Verbindungen der Formel I nach Anspruch 1 oder 2, wobei R¹ C₁₋₇-Alkyl ist.

4. Verbindungen der Formel I nach Anspruch 1 oder 2, wobei R¹ C₁₋₇-Alkyl, substituiert durch Phenyl, ist.

5. Verbindungen der Formel I nach einem der Ansprüche 1 bis 4, wobei R² C₁₋₇-Alkyl ist.

6. Verbindungen der Formel I nach Anspruch 1 mit der Formel

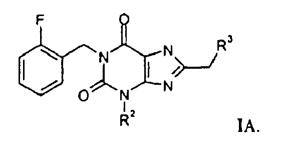

7. Verbindungen der Formel IA nach Anspruch 6, wobei R² C₁₋₇-Alkyl ist.

8. Verbindungen der Formel IA nach Anspruch 7, wobei R² n-Butyl ist.

9. Verbindungen der Formel IA nach Anspruch 6, wobei R³ 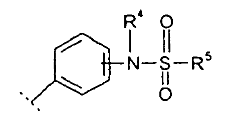 ist.

10. Verbindungen der Formel IA nach Anspruch 9, wobei R⁴ H ist.

11. Verbindungen der Formel IA nach Anspruch 9, wobei R⁵ ein 5gliedriger heteroaromatischer Ring ist, der ein Heteroatom, unabhängig ausgewählt aus der Gruppe, bestehend aus N, O und S, aufweist, wobei der 5gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

12. Verbindungen der Formel IA nach Anspruch 9, wobei R⁵ ein 5gliedriger heteroaromatischer Ring ist, der zwei Heteroatome aufweist, wobei ein erstes Heteroatom N ist und ein zweites Heteroatom unabhängig aus der Gruppe ausgewählt ist, bestehend aus N, O und S, wobei der 5gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

13. Verbindungen der Formel IA nach Anspruch 9, wobei R⁵ ein 6gliedriger heteroaromatischer Ring ist, der ein N aufweist, wobei der 6gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

14. Verbindungen der Formel IA nach Anspruch 9, wobei R⁵ 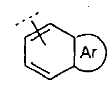 ist, wobei Ar aus der Gruppe ausgewählt ist, bestehend aus
einem 5gliedrigen heteroaromatischen Ring, anelliert an den 6gliedrigen Ring, der ein, zwei oder drei Heteroatome aufweist, und wobei ein erstes Heteroatom N ist, ein zweites Heteroatom N ist und ein drittes Heteroatom aus der Gruppe ausgewählt ist, bestehend aus O und S, und
einem 6gliedrigen aromatischen Ring, anelliert an den 6gliedrigen Ring, der kein oder ein N-Heteroatom aufweist, wobei der anellierte 6gliedrige aromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

15. Verbindungen der Formel IA nach Anspruch 9, wobei R⁵ C₁₋₇-Alkyl oder C₁₋₇-Alkyl, substituiert durch Phenyl, ist.

16. Verbindungen der Formel IA nach Anspruch 9, wobei R⁵ C₂₋₇-Alkenyl, substituiert durch Phenyl, ist.

17. Verbindungen der Formel IA nach Anspruch 9, wobei R⁵ Phenyl ist.

18. Verbindungen der Formel IA nach Anspruch 9, wobei R⁵ Phenyl, substituiert durch mindestens einen Substituenten, ausgewählt aus der Gruppe, bestehend aus C₁₋₇-Alkyl, C₁₋₇-Alkyl, substituiert durch Halogen, Halogen, C₁₋₇-Alkoxy, C₁₋₇-Alkoxy, substituiert durch Halogen, Nitro und Acetamido, ist.

19. Verbindungen der Formel IA nach Anspruch 18, wobei R⁵ Phenyl, substituiert durch Halogen, ist.

20. Verbindungen der Formel IA nach Anspruch 18, wobei R⁵ Phenyl, substituiert durch C₁₋₇-Alkyl, oder C₁₋₇-Alkyl, substituiert durch Halogen, ist.

21. Verbindungen der Formel IA nach Anspruch 18, wobei die Phenylgruppe zwei oder drei Substituenten aufweist, ausgewählt aus der Gruppe, bestehend aus C₁₋₇-Alkyl, C₁₋₇-Alkyl, substituiert durch Halogen, Halogen, C₁₋₇-Alkoxy, C₁₋₇-Alkoxy, substituiert durch Halogen, Nitro und Acetamido.

22. Verbindungen der Formel IA nach Anspruch 6, wobei R³ 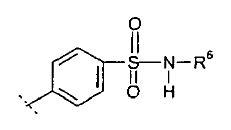 ist.

23. Verbindungen der Formel IA nach Anspruch 22, wobei R⁶ ein 5gliedriger aromatischer heterocyclischer Ring mit einem oder zwei Heteroatomen ist, wobei ein erstes Heteroatom N ist und ein zweites Heteroatom aus der Gruppe ausgewählt ist, bestehend aus N und S, wobei der 5 gliedrige heterocyclische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

24. Verbindungen der Formel IA nach Anspruch 22, wobei R⁶ ein 6gliedriger aromatischer heterocyclischer Ring mit einem oder zwei N-Heteroatomen ist, wobei der 6gliedrige heterocyclische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

25. Verbindungen der Formel IA nach Anspruch 6, wobei R³ 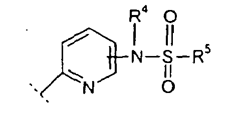 ist.

26. Verbindungen der Formel IA nach Anspruch 25, wobei R⁴ H ist und R⁵ ein 5gliedriger aromatischer heterocyclischer Ring mit einem oder zwei Heteroatomen ist, wobei ein erstes Heteroatom N ist und ein zweites Heteroatom aus der Gruppe ausgewählt ist, bestehend aus N und S, wobei der 5gliedrige heterocyclische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

27. Verbindungen der Formel IA nach Anspruch 6, wobei R² C₁₋₇-Alkyl, substituiert durch Cycloalkyl, ist.

28. Verbindungen der Formel I nach Anspruch 8 mit der Formel

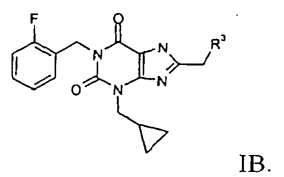

29. Verbindungen der Formel IB nach Anspruch 28, wobei R³ 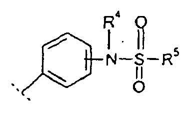 ist.

30. Verbindungen der Formel IB nach Anspruch 29, wobei R⁴ H ist.

31. Verbindungen der Formel IB nach Anspruch 29, wobei R⁵ ein 5gliedriger heteroaromatischer Ring ist, der zwei Heteroatome aufweist, wobei ein erstes Heteroatom N ist und ein zweites Heteroatom unabhängig aus der Gruppe ausgewählt ist, bestehend aus N, O und S, wobei der 5gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

32. Verbindungen der Formel IB nach Anspruch 29, wobei R⁵ Phenyl, substituiert durch mindestens einen Substituenten, ausgewählt aus der Gruppe, bestehend aus C₁₋₇-Alkyl, C₁₋₇-Alkyl, substituiert durch Halogen, Halogen, C₁₋₇-Alkoxy, C₁₋₇-Alkoxy, substituiert durch Halogen, Nitro und Acetamido, ist.

33. Verbindungen der Formel IB nach Anspruch 29, wobei R⁵ ein 6gliedriger heteroaromatischer Ring ist, der ein N aufweist, wobei der 6gliedrige heteroaromatische Ring unsubstituiert oder durch mindestens einen Substituenten, ausgewählt aus der Gruppe, bestehend aus C₁₋₇-Alkyl, Halogen, Carboxy-C₁₋₇-alkyl, Amino, Alkylamino und Acetamido, substituiert ist.

34. Verbindungen der Formel IB nach Anspruch 29, wobei R⁵ C₁₋₇-Alkyl ist.

35. Verbindungen der Formel IB nach Anspruch 29, wobei R⁴ C₁₋₇-Alkyl ist.

36. Verbindungen der Formel I nach Anspruch 1 oder 2, wobei die Verbindung aus der Gruppe ausgewählt ist, bestehend aus
5-Chlor-1,3-dimethyl-1H-pyrazol-4-sulfonsäure-{4-[3-cyclopropylmethyl-1-(2-fluor-benzyl)-2,6-dioxo-2,3,6,7-tetrahydro-1H-purin-8-ylmethyl]-phenyl}-amid,
1-Methyl-1H-pyrazol-4-sulfonsäure-{4-[3-cyclopropylmethyl-1-(2-fluor-benzyl)-2,6-dioxo-2,3,6,7-tetrahydro-1H-purin-8-ylmethyl]-phenyl}-amid,
N-{4-[3-Cyclopropylmethyl-1-(2-fluor-benzyl)-2,6-dioxo-2,3,6,7-tetrahydro-1H-purin-8-ylmethyl]-phenyl}-3-methoxy-benzolsulfonamid,
N-{4-[3-Cyclopropylmethyl-1-(2-fluor-benzyl)-2,6-dioxo-2,3,6,7-tetrahydro-1H-purin-8-ylmethyl]-phenyl}-3-fluor-benzolsulfonamid,
5-Brom-6-chlor-pyridin-3-sulfonsäure-{4-[3-cyclopropylmethyl-1-(2-fluor-benzyl)-2,6-dioxo-2,3,6,7-tetrahydro- 1H-purin-8-ylmethyl]-phenyl }-amid,
5-Chlor-1,3-dimethyl-1H-pyrazol-4-sulfonsäure- {3-[3-cyclopropylmethyl-1-(2-fluor-benzyl)-2,6-dioxo-2,3,6,7-tetrahydro-1H-purin-8-ylmethyl]-phenyl} -amid,
1,3-Dimethyl-1H-pyrazol-4-sulfonsäure- {4-[3-cyclopropylmethyl-1-(2-fluor-benzyl)-2,6-dioxo-2,3,6,7-tetrahydro-1H-purin-8-ylmethyl]-phenyl }-amid,
N-(5- {4-[3-Cyclopropylmethyl-1-(2-fluor-benzyl)-2,6-dioxo-2,3,6,7-tetrahydro-1H-purin-8-ylmethyl]-phenylsulfamoyl} -4-methyl-thiazol-2-yl)-acetamid;
2-Amino-4-methyl-thiazol-5-sulfonsäure- {4-[3-cyclopropylmethyl-1-(2-fluorobenzyl)-2,6-dioxo-2,3,6,7-tetrahydro-1H-purin-8-ylmethyl]-phenyl}-amid-trifluor-essigsäuresalz,
3,5-Dimethyl-isoxazol-4-sulfonsäure- {4-[3-cyclopropylmethyl-1-(2-fluor-benzyl)-2,6-dioxo-2, 3, 6, 7-tetrahydro-1H-purin-8-ylmethyl]-phenyl}-amid;
1,2-Dimethyl-1H-imidazol-4-sulfonsäure- {4- [3-cyclopropylmethyl-1-(2-fluor-benzyl)-2 ,6-dioxo-2,3,6,7-tetrahydro-1H-purin-8-ylmethyl]-phenyl}-amid,
1-Methyl-1H-imidazol-4-sulfonsäure- {4-[3-cyclopropylmethyl-1-(2-fluor-benzyl)-2,6-dioxo-2,3,6,7-tetrahydro-1H-purin-8-ylmethyl]-phenyl}-amid,
N-{4- [3-Cyclopropylmethyl-1-(2-fluor-benzyl)-2,6-dioxo-2,3,6,7-tetrahydro-1H-purin-8-yl-methyl]-phenyl}-N,N-dimethylsulfamid,
5-Chlor-1,3-dimethyl-1H-pyrazol-4-sulfonsäure-{4-[3-cyclopropylmethyl-1-(2-fluor-benzyl)-2,6-dioxo-2,3,6,7-tetrahydro-1H-purin-8-ylmethyl]-phenyl}-methyl-amid,
Chinolin-8-sulfonsäure- {4-[3-cyclopropylmethyl-1-(2-fluor-benzyl)-2,6-dioxo-2,3,6,7-tetrahydro-1H-purin-8-ylmethyl]-phenyl}-amid,
N-(4- {4-[3-Cyclopropylmethyl-1-(2-fluor-benzyl)-2,6-dioxo-2,3,6,7-tetrahydro-1H-purin-8-ylmethyl]-phenylsulfamoyl}-phenyl)-acetamid,
N-[4-(1-Benzyl-3-butyl-2,6-dioxo-2,3,6,7-tetrahydro-1H-purin-8-ylmethyl)-phenyl]- . 4-methyl-benzolsulfonamid,
Pyridin-3-sulfonsäure-{4-[3-cyclopropylmethyl-1-(2-fluor-benzyl)-2,6-dioxo-2,3,6,7-tetrahydro-1H-purin-8-ylmethyl]-phenyl}-amid-trifluor-essigsäuresalz,
N-{4-[3-Cyclopropylmethyl-1-(2-fluor-benzyl)-2,6-dioxo-2,3,6,7-tetrahydro-1H-purin-8-ylmethyl]-phenyl}-methansulfonamid,
N-[4-(1-Benzyl-3-butyl-2,6-dioxo-2,3,6,7-tetrahydro-1H-purin-8-ylmethyl)-phenyl]-methan-sulfonamid,
5-Chlor-1,3-dimethyl-1H-pyrazol-4-sulfonsäure-{6-[3-butyl-1-(2-fluor-benzyl)-2,6-dioxo-2,3,6,7-tetrahydro-1H-purin-8-ylmethyl]-pyridin-3-yl}-amid,
5-Chlor-1,3-dimethyl-1 H-pyrazol-4-sulfonsäure- {6-[3-cyclobutylmethyl-1-(2-fluor-benzyl)-2,6-dioxo-2,3 ,6,7 -tetrahydro-1H -purin-8-ylmethyl]-pyridin-3-yl}-amid,
1,3-Dimethyl-1H-pyrazol-4-sulfonsäure- {4- [3-cyclopropylmethyl-1 -(2-fluor-benzyl)-2,6-dioxo-2, 3,6,7-tetrahydro-1H-purin-8-ylmethyl]-phenyl}-methyl-amid,
4- [3- Butyl-1-(2-fluor-benzyl)-2,6-dioxo-2,3,6,7 -tetrahydro-1H-purin-8-ylmethyl]-N-pyridin-2-yl-benzolsulfonamid,
4-[3-Butyl-1-(2-fluor-benzy1)-2,6-dioxo-2,3,6,7-tetrahydro-1H-purin-8-ylmethyl]-N-pyridin-4-yl-benzolsulfonamid,
4-[3-Butyl-1-(2-fluor-benzyl)-2,6-dioxo-2,3,6,7-tetrahydro-1H-purin-8-ylmethyl]-N-pyridin-3-yl-benzolsulfonamid,
4-[3-Butyl-1-(2-fluor-benzyl)-2,6-dioxo-2,3,6,7-tetrahydro-1H-purin-8-ylmethyl]-N-pyrimidin-2-yl-benzolsulfonamid und
4-[3-Butyl-1-(2-fluor-benzyl)-2,6-dioxo-2,3,6,7-tetrahydro-1H-purin-8-ylmethyl]-N-(1,3,5-trimethyl-1H-pyrazol-4-yl)-benzolsulfonamid.

37. Verfahren zur Herstellung einer Verbindung nach einem der Ansprüche 1 bis 36, wobei das Verfahren die Cyclisierung einer Verbindung der Formel II 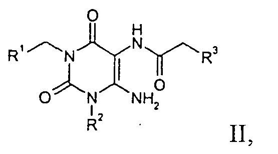 worin R¹, R² und R³ wie in einem der Ansprüche 1 bis 36 definiert sind, unter Erhalt der Verbindung der Formel I umfaßt.

38. Verbindungen nach einem der Ansprüche 1 bis 36, hergestellt durch ein Verfahren nach Anspruch 37.

39. Pharmazeutische Zusammensetzungen, umfassend eine Verbindung nach einem der Ansprüche 1 bis 36 oder ein pharmazeutisch akzeptables Salz davon und einen pharmazeutisch akzeptablen Hilfsstoff.

40. Verbindungen nach einem der Ansprüche 1 bis 36 zur Verwendung als therapeutisch aktive Substanzen.

41. Verwendung von Verbindungen nach einem der Ansprüche 1 bis 36 zur Herstellung von Medikamenten zur Behandlung und/oder Prophylaxe von Krankheiten, die mit Phosphoenolpyruvatcarboxykinase in Verbindung stehen.

42. Verwendung nach Anspruch 41 zur Behandlung von Diabetes vom Typ 2.

## Revendications

1. Composés de formule 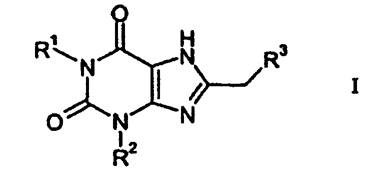 dans laquelle
R¹ est choisi dans la classe formée par les groupes alkyle en C₁ à C₇,
alkyle en C₁ à C₇ substitué par phényle et
alkyle en C₁ à C₇ substitué par phényle substitué par halogène ;
R² est choisi dans la classe formée par les groupes alkyle en C₁ à C₇ et
alkyle en C₁ à C₇ substitué par cycloalkyle en C₃ à C₇ ;
R³ est choisi dans la classe formée par 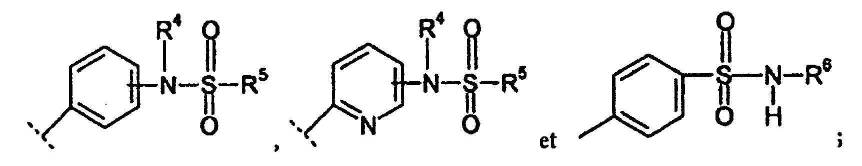
R⁴ est choisi dans la classe formée par H et les groupes alkyle en C₁ à C₇ ;
R⁵ est choisi dans la classe formée par les groupes alkyle en C₁ à C₇,
amino (alkyle en C₁ à C₇),
alkyle en C₁ à C₇ substitué par phényle,
alcényle en C₂ à C₇ substitué par phényle,
phényle,
phényle substitué par au moins un substituant choisi dans la classe formée par les groupes alkyle en C₁ à C₇, alkyle en C₁ à C₇ substitué par halogène, halogène, alkoxy en C₁ à C₇,
alkoxy en C₁ à C₇ substitué par halogène, nitro ou acétamido,
un cycle hétéroaromatique pentagonal ayant un seul hétéroatome choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy-(alkyle en C₁ à C₇), amino, alkylamino et acétamido,
un cycle hétéroaromatique pentagonal ayant deux hétéroatomes dans lequel un premier hétéroatome est N et un second hétéroatome est choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy(alkyle en C₁ à C₇), amino, alkylamino et acétamido,
un cycle hétéroaromatique hexagonal ayant un seul N, le cycle hétéroaromatique hexagonal étant non substitué ou substitué par au moins un substituant choisi dans la classe formée par les groupes alkyle en C₁ à C₇, halogène, carboxy(alkyle en C₁ à C₇), amino, alkylamino et acétamido, 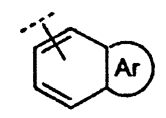 où Ar est choisi dans la classe formée par
un cycle hétéroaromatique pentagonal condensé au cycle hexagonal, ayant un, deux ou trois hétéroatomes, et dans lequel un premier hétéroatome est N, un deuxième hétéroatome est N et un troisième hétéroatome est choisi dans la classe formée par O et S,
un cycle aromatique hexagonal condensé au cycle hexagonal, ayant zéro ou un seul hétéroatome N, le cycle aromatique hexagonal condensé étant non substitué ou substitué par au moins un substituant choisi dans la classe formée par les groupes alkyle en C₁ à C₇, halogène, carboxy (alkyle en C₁ à C₇), amino, alkylamino et acétamido ; et
R⁶ est choisi dans la classe formée par H,
un cycle hétérocyclique aromatique pentagonal ayant un ou deux hétéroatomes dans lequel un premier hétéroatome est N et un second hétéroatome est choisi dans la classe formée par N et S, le cycle hétérocyclique pentagonal étant non substitué ou substitué par au moins un substituant choisi dans la classe formée par les groupes alkyle en C₁ à C₇, halogène, carboxy-(alkyle en C₁ à C₇), amino, alkylamino et acétamido, et
un cycle hétérocyclique aromatique hexagonal ayant un ou deux hétéroatomes N, le cycle hétérocyclique hexagonal étant non substitué ou substitué par au moins un substituant choisi dans la classe formée par les groupes alkyle en C₁ à C₇, halogène, carboxy(alkyle en C₁ à C₇), amino, alkylamino et acétamido,
ou un sel pharmaceutiquement acceptable de ceux-ci.

2. Composés selon la revendication 1, de formule 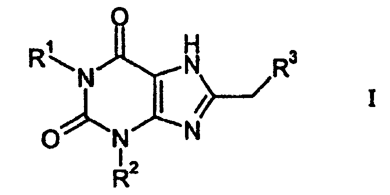 dans laquelle
R¹ est choisi dans la classe formée par les groupes alkyle en C₁ à C₇,
alkyle en C₁ à C₇ substitué par phényle et
alkyle en C₁ à C₇ substitué par phényle substitué par halogène ;
R² est choisi dans la classe formée par les groupes alkyle en C₁ à C₇ et
alkyle en C₁ à C₇ substitué par cycloalkyle en C₃ à C₇ ;
R³ est choisi dans la classe formée par 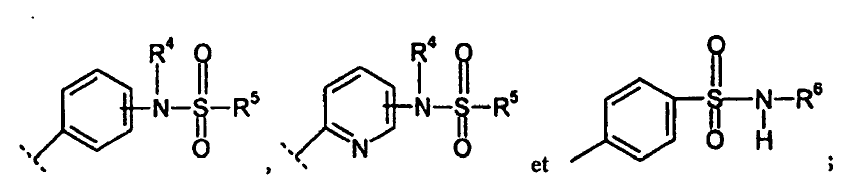
R⁴ est choisi dans la classe formée par H et les groupes alkyle en C₁ à C₇ ;
R⁵ est choisi dans la classe formée par les groupes alkyle en C₁ à C₇,
amino (alkyle en C₁ à C₇),
alkyle en C₁ à C₇ substitué par phényle,
alcényle en C₂ à C₇ substitué par phényle,
phényle,
phényle substitué par au moins un substituant choisi dans la classe formée par les groupes alkyle en C₁ à C₇, alkyle en C₁ à C₇ substitué par halogène, halogène, alkoxy en C₁ à C₇,
alkoxy en C₁ à C₇ substitué par halogène, nitro et acétamido,
un cycle hétéroaromatique pentagonal ayant un seul hétéroatome choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy-(alkyle en C₁ à C₇), amino, alkylamino et acétamido,
un cycle hétéroaromatique pentagonal ayant deux hétéroatomes dans lequel un premier hétéroatome est N et un second hétéroatome est choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy(alkyle en C₁ à C₇), amino, alkylamino et acétamido,
un cycle hétéroaromatique hexagonal ayant un seul N, le cycle hétéroaromatique hexagonal étant non substitué ou substitué par au moins un substituant choisi dans la classe formée par les groupes alkyle en C₁ à C₇, halogène, carboxy(alkyle en C₁ à C₇), amino, alkylamino et acétamido, 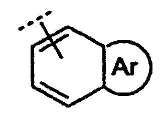 où Ar est choisi dans la classe formée par
un cycle hétéroaromatique pentagonal condensé au cycle hexagonal, ayant un, deux ou trois hétéroatomes, et dans lequel un premier hétéroatome est N, un deuxième hétéroatome est N et un troisième hétéroatome est choisi dans la classe formée par O et S,
un cycle aromatique hexagonal condensé au cycle hexagonal, ayant zéro ou un seul hétéroatome N, le cycle aromatique hexagonal condensé étant non substitué ou substitué par au moins un substituant choisi dans la classe formée par les groupes alkyle en C₁ à C₇, halogène, carboxy(alkyle en C₁ à C₇), amino, alkylamino et acétamido ; et
R⁶ est choisi dans la classe formée par H,
un cycle hétérocyclique aromatique pentagonal ayant un ou deux hétéroatomes dans lequel un premier hétéroatome est N et un second hétéroatome est choisi dans la classe formée par N et S, le cycle hétérocyclique pentagonal étant non substitué ou substitué par au moins un substituant choisi dans la classe formée par les groupes alkyle en C₁ à C₇, halogène, carboxy-(alkyle en C₁ à C₇), amino, alkylamino et acétamido, et
un cycle hétérocyclique aromatique hexagonal ayant un ou deux hétéroatomes N, le cycle hétérocyclique hexagonal étant non substitué ou substitué par au moins un substituant choisi dans la classe formée par les groupes alkyle en C₁ à C₇, halogène, carboxy-(alkyle en C₁ à C₇), amino, alkylamino et acétamido,
ou un sel pharmaceutiquement acceptable de ceux-ci.

3. Composés de formule I selon la revendication 1 ou 2, dans lesquels R¹ est un groupe alkyle en C₁ à C₇.

4. Composés de formule I selon la revendication 1 ou 2, dans lesquels R¹ est un groupe alkyle en C₁ à C₇ substitué par phényle.

5. Composés de formule I selon l'une quelconque des revendications 1 à 4, dans lesquels R² est un groupe alkyle en C₁ à C₇.

6. Composés de formule I selon la revendication 1, ayant la formule

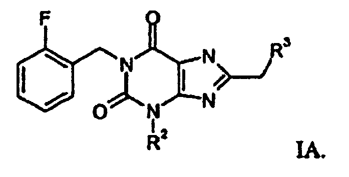

7. Composés de formule IA selon la revendication 6, dans lesquels R² est un groupe alkyle en C₁ à C₇.

8. Composés de formule IA selon la revendication 7, dans lesquels R² est un groupe *n*-butyle.

9. Composés de formule IA selon la revendication 6, dans lesquels R³ est

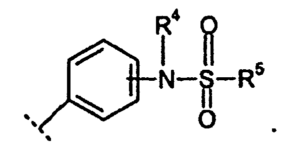

10. Composés de formule IA selon la revendication 9, dans lesquels R⁴ est H.

11. Composés de formule IA selon la revendication 9, dans lesquels R⁵ est un cycle hétéroaromatique pentagonal ayant un seul hétéroatome choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy (alkyle en C₁ à C₇), amino, alkylamino et acétamido.

12. Composés de formule IA selon la revendication 9, dans lesquels R⁵ est un cycle hétéroaromatique pentagonal ayant deux hétéroatomes, dans lequel un premier hétéroatome est N et un second hétéroatome est choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy(alkyle en C₁ à C₇), amino, alkylamino et acétamido.

13. Composés de formule IA selon la revendication 9, dans lesquels R⁵ est un cycle hétéroaromatique hexagonal ayant un seul N, le cycle hétéroaromatique hexagonal étant non substitué ou substitué par au moins un substituant choisi dans la classe formée par les groupes alkyle en C₁ à C₇, halogène, carboxy(alkyle en C₁ à C₇), amino, alkylamino et acétamido.

14. Composés de formule IA selon la revendication 9, dans lesquels R⁵ est 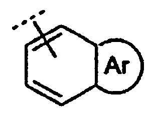 où Ar est choisi dans la classe formée par
un cycle hétéroaromatique pentagonal condensé au cycle hexagonal, ayant un, deux ou trois hétéroatomes, et dans lequel un premier hétéroatome est N, un deuxième hétéroatome est N et un troisième hétéroatome est choisi dans la classe formée par O et S, et
un cycle aromatique hexagonal condensé au cycle hexagonal, ayant zéro ou un seul hétéroatome N, le cycle aromatique hexagonal condensé étant non substitué ou substitué par au moins un substituant choisi dans la classe formée par les groupes alkyle en C₁ à C₇, halogène, carboxy(alkyle en C₁ à C₇), amino, alkylamino et acétamido.

15. Composés de formule IA selon la revendication 9, dans lesquels R⁵ est un groupe alkyle en C₁ à C₇ ou alkyle en C₁ à C₇ substitué par phényle.

16. Composés de formule IA selon la revendication 9, dans lesquels R⁵ est un groupe alcényle en C₂ à C₇ substitué par phényle.

17. Composés de formule IA selon la revendication 9, dans lesquels R⁵ est un groupe phényle.

18. Composés de formule IA selon la revendication 9, dans lesquels R⁵ est un groupe phényle substitué par au moins un substituant choisi dans la classe formée par les groupes alkyle en C₁ à C₇, alkyle en C₁ à C₇ substitué par halogène, halogène, alkoxy en C₁ à C₇, alkoxy en C₁ à C₇ substitué par halogène, nitro et acétamido.

19. Composés de formule IA selon la revendication 18, dans lesquels R⁵ est un groupe phényle substitué par halogène.

20. Composés de formule IA selon la revendication 18, dans lesquels R⁵ est un groupe phényle substitué par alkyle en C₁ à C₇ ou alkyle en C₁ à C₇ substitué par halogène.

21. Composés de formule IA selon la revendication 18, dans lesquels le groupe phényle porte deux ou trois substituants choisis dans la classe formée par les groupes alkyle en C₁ à C₇, alkyle en C₁ à C₇ substitué par halogène, halogène, alkoxy en C₁ à C₇, alkoxy en C₁ à C₇ substitué par halogène, nitro et acétamido.

22. Composés de formule IA selon la revendication 6, dans lesquels R³ est

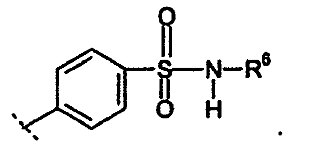

23. Composés de formule IA selon la revendication 22, dans lesquels R⁶ est un cycle hétérocyclique aromatique pentagonal ayant un ou deux hétéroatomes, dans lequel un premier hétéroatome est N et un second hétéroatome est choisi dans la classe formée par N et S, le cycle hétérocyclique pentagonal étant non substitué ou substitué par au moins un substituant choisi dans la classe formée par les groupes alkyle en C₁ à C₇, halogène, carboxy(alkyle en C₁ à C₇), amino, alkylamino et acétamido.

24. Composés de formule IA selon la revendication 22, dans lesquels R⁶ est un cycle hétérocyclique aromatique hexagonal avec un ou deux hétéroatomes N, le cycle hétérocyclique hexagonal étant non substitué ou substitué par au moins un substituant choisi dans la classe formée par les groupes alkyle en C₁ à C₇, halogène, carboxy (alkyle en C₁ à C₇), amino, alkylamino et acétamido.

25. Composés de formule IA selon la revendication 6, dans lesquels R³ est

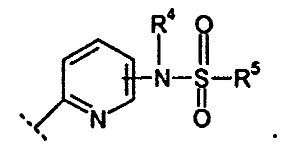

26. Composés de formule IA selon la revendication 25, dans lesquels R⁴ est H, et R⁵ est un cycle hétérocyclique aromatique pentagonal ayant un ou deux hétéroatomes, dans lequel un premier hétéroatome est N et un second hétéroatome est choisi dans la classe formée par N et S, le cycle hétérocyclique pentagonal étant non substitué ou substitué par au moins un substituant choisi dans la classe formée par les groupes alkyle en C₁ à C₇, halogène, carboxy(alkyle en C₁ à C₇), amino, alkylamino et acétamido.

27. Composés de formule IA selon la revendication 6, dans lesquels R² est un groupe alkyle en C₁ à C₇ substitué par cycloalkyle.

28. Composés de formule I selon la revendication 8, ayant la formule

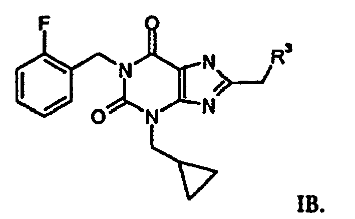

29. Composés de formule IB selon la revendication 28, dans lesquels R³ est

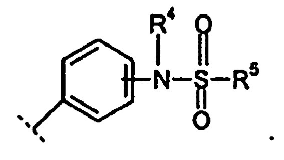

30. Composés de formule IB selon la revendication 29, dans lesquels R⁴ est H.

31. Composés de formule IB selon la revendication 29, dans lesquels R⁵ est un cycle hétéroaromatique pentagonal ayant deux hétéroatomes, dans lequel un premier hétéroatome est N et un second hétéroatome est choisi indépendamment dans la classe formée par N, O et S, le cycle hétéroaromatique pentagonal étant non substitué ou substitué par au moins un substituant choisi dans la classe formée par les groupes alkyle en C₁ à C₇, halogène, carboxy(alkyle en C₁ à C₇), amino, alkylamino et acétamido.

32. Composés de formule IB selon la revendication 29, dans lesquels R⁵ est un groupe phényle substitué par au moins un substituant choisi dans la classe formée par les groupes alkyle en C₁ à C₇, alkyle en C₁ à C₇ substitué par halogène, halogène, alkoxy en C₁ à C₇, alkoxy en C₁ à C₇ substitué par halogène, nitro et acétamido.

33. Composés de formule IB selon la revendication 29, dans lesquels R⁵ est un cycle hétéroaromatique hexagonal ayant un seul N, le cycle hétéroaromatique hexagonal étant non substitué ou substitué par au moins un substituant choisi dans la classe formée par les groupes alkyle en C₁ à C₇, halogène, carboxy (alkyle en C₁ à C₇), amino, alkylamino et acétamido.

34. Composés de formule IB selon la revendication 29, dans lesquels R⁵ est un groupe alkyle en C₁ à C₇.

35. Composés de formule IB selon la revendication 29, dans lesquels R⁴ est un groupe alkyle en C₁ à C₇.

36. Composés de formule I selon la revendication 1 ou 2, dans lesquels le composé est choisi dans la classe formée par les suivants :
{4-[3-cyclopropylméthyl-1-(2-fluorobenzyl)-2,6-dioxo-2,3,6,7-tétrahydro-1H-purine-8-ylméthyl]phényl}amide d'acide 5-chloro-1,3-diméthyl-1H-pyrazole-4-sulfonique,
{4-[3-cyclopropylméthyl-1-(2-fluorobenzyl)-2,6-dioxo-2,3,6,7-tétrahydro-1H-purine-8-ylméthyl]phényl}amide d'acide 1-méthyl-1H-pyrazole-4-sulfonique,
*N*-{4-[3-cyclopropylméthyl-1-(2-fluorobenzyl)-2,6-dioxo-2,3,6,7-tétrahydro-1H-purine-8-ylméthyl]phényl}-3-méthoxy-benzènesulfonamide,
*N*-{4-[3-cyclopropylméthyl-1-(2-fluorobenzyl)-2,6-dioxo-2,3,6,7-tétrahydro-1H-purine-8-ylméthyl]phényl}-3-fluoro-benzènesulfonamide,
{4-[3-cyclopropylméthyl-1-(2-fluorobenzyl)-2,6-dioxo-2,3,6,7-tétrahydro-1H-purine-8-ylméthyl]phényl}amide d'acide 5-bromo-6-chloropyridine-3-sulfonique,
{3-[3-cyclopropylméthyl-1-(2-fluorobenzyl)-2,6-dioxo-2,3,6,7-tétrahydro-1H-purine-8-ylméthyl]phényl}amide d'acide 5-chloro-1,3-diméthyl-1H-pyrazole-4-sulfonique,
{4-[3-cyclopropylméthyl-1-(2-fluorobenzyl)-2,6-dioxo 2,3,6,7-tétrahydro-1H-purine-8-ylméthyl]phényl}amide d'acide 1,3-diméthyl-1H-pyrazole-4-sulfonique,
*N*-(5-{4-[3-cyclopropylméthyl-1-(2-fluorobenzyl)-2,6-dioxo-2,3,6,7-tétrahydro-1H-purine-8-ylméthyl]phénylsulfamoyl}-4-méthyl-thiazole-2-yl)acétamide,
sel d'acide trifluoroacétique de {4-[3-cyclopropylméthyl-1-(2-fluorobenzyl)-2,6-dioxo-2,3,6,7-tétrahydro-1H-purine-8-ylméthyl]phényl}amide d'acide 2-amino-4-méthyl-thiazole-5-sulfonique,
{4-[3-cyclopropylméthyl-1-(2-fluorobenzyl)-2,6-dioxo-2,3,6,7-tétrahydro-1H-purine-8-ylméthyl]phényl}amide d'acide 3,5-diméthyl-isoxazole-4-sulfonique,
{4-[3-cyclopropylméthyl-1-(2-fluorobenzyl)-2,6-dioxo-2,3,6,7-tétrahydro-1H-purine-8-ylméthyl]phényl}amide d'acide 1,2-diméthyl-1H-imidazole-4-sulfonique,
{4-[3-cyclopropylméthyl-1-(2-fluorobenzyl)-2,6-dioxo-2,3,6,7-tétrahydro-1H-purine-8-ylméthyl]phényl}amide d'acide 1-méthyl-1H-imidazole-4-sulfonique,
*N*-{4-[3-cyclopropylméthyl-1-(2-fluorobenzyl)-2,6-dioxo-2,3,6,7-tétrahydro-1H-purine-8-ylméthyl]phényl}-N,N-diméthylsulfamide,
{4-[3-cyclopropylméthyl-1-(2-fluorobenzyl)-2,6-dioxo-2,3,6,7-tétrahydro-1H-purine-8-ylméthyl]phényl}méthylamide d'acide 5-chloro-1,3-diméthyl-1H-pyrazole-4-sulfonique,
{4-[3-cyclopropylméthyl-1-(2-fluorobenzyl)-2,6-dioxo-2,3,6,7-tétrahydro-1H-purine-8-ylméthyl]phényl}amide d'acide quinoléine-8-sulfonique,
*N*-(4-{4-[3-cyclopropylméthyl-1-(2-fluorobenzyl)-2,6-dioxo-2,3,6,7-tétrahydro-1H-purine-8-ylméthyl]phénylsulfamoyl}-phényl)acétamide,
*N*-[4-(1-benzyl-3-butyl-2,6-dioxo-2,3,6,7-tétrahydro-1H-purine-8-ylméthyl)phényl]-4-méthyl-benzènesulfonamide,
sel d'acide trifluoracétique de {4-[3-cyclopropylméthyl-1-(2-fluorobenzyl)-2,6-dioxo-2,3,6,7-tétrahydro-1H-purine-8-ylméthyl]phényl}amide d'acide pyridine-3-sulfonique,
*N*-{4-[3-cyclopropylméthyl-1-(2-fluorobenzyl)-2,6-dioxo-2,3,6,7-tétrahydro-1H-purine-8-ylméthyl]phényl}méthane-sulfonamide,
*N*-[4-(1-benzyl-3-butyl-2,6-dioxo-2,3,6,7-tétrahydro-1H-purine-8-ylméthyl)phényl]méthanesulfonamide,
{6-[3-butyl-1-(2-fluorobenzyl)-2,6-dioxo-2,3,6,7-tétrahydro-1H-purine-8-ylméthyl]pyridine-3-yl}amide d'acide 5-chloro-1,3-diméthyl-1H-pyrazole-4-sulfonique,
{6-[3-cyclobutylméthyl-1-(2-fluorobenzyl)-2,6-dioxo-2,3,6,7-tétrahydro-1H-purine-8-ylméthyl]pyridine-3-yl}amide d'acide 5-chloro-1,3-diméthyl-1H-pyrazole-4-sulfonique,
{4-[3-cyclopropylméthyl-1-(2-fluorobenzyl)-2,6-dioxo-2,3,6,7-tétrahydro-1H-purine-8-ylméthyl]phényl}-méthyl-amide d'acide 1,3-diméthyl-1H-pyrazole-4-sulfonique,
4-[3-butyl-1-(2-fluorobenzyl)-2,6-dioxo-2,3,6,7-tétrahydro-1H-purine-8-ylméthyl]-*N*-pyridine-2-yl-benzènesulfonamide,
4-[3-butyl-1-(2-fluorobenzyl)-2,6-dioxo-2,3,6,7-tétrahydro-1H-purine-8-ylméthyl]-*N*-pyridine-4-yl-benzènesulfonamide,
4-[3-butyl-1-(2-fluorobenzyl)-2,6-dioxo-2,3,6,7-tétrahydro-1H-purine-8-ylméthyl]-*N*-pyridine-3-yl-benzènesulfonamide,
4-[3-butyl-1-(2-fluorobenzyl)-2,6-dioxo-2,3,6,7-tétrahydro-1H-purine-8-ylméthyl]-*N*-pyrimidine-2-yl-benzènesulfonamide, et
4-[3-butyl-1-(2-fluorobenzyl)-2,6-dioxo-2,3,6,7-tétrahydro-1H-purine-8-ylméthyl]-*N*-(1,3,5-triméthyl-1H-pyrazole-4-yl)benzènesulfonamide.

37. Procédé pour la préparation d'un composé selon l'une quelconque des revendications 1 à 36, lequel procédé comprend la cyclisation d'un composé de formule II 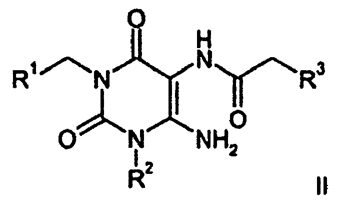 dans laquelle R¹, R² et R³ sont tels que définis dans l'une quelconque des revendications 1 à 36, pour donner le composé de formule I.

38. Composés selon l'une quelconque des revendications 1 à 36, lorsqu'ils sont fabriqués par un procédé selon la revendication 37.

39. Compositions pharmaceutiques comprenant un composé selon l'une quelconque des revendications 1 à 36, ou un sel pharmaceutiquement acceptables de celui-ci, et un excipient pharmaceutiquement acceptable.

40. Composés selon l'une quelconque des revendications 1 à 36, pour leur utilisation comme substances thérapeutiques actives.

41. Utilisation de composés selon l'une quelconque des revendications 1 à 36, pour la préparation de médicaments destinés au traitement et/ou à la prophylaxie de maladies associées à la phosphoénolpyruvate-carboxykinase.

42. Utilisation selon la revendication 41, pour le traitement du diabète type 2.
